# EUROPEAN PATENT APPLICATION

(11) **EP 4 082 557 A2**
(43) Date of publication of application: **02.11.2022**
(21) Application number: 22164880.1
(22) Date of filing: 17.07.2018
(51) Int. Cl.: A61K 38/10, C07K 14/72, C07K 14/47, A61K 9/00, A61K 31/573, A61K 38/02, A61K 38/17, A61K 38/21, A61K 45/06, A61K 47/02

(54) **PEPTIDES AND METHODS OF TRANSPLANTATION AND RESTORATIVE ORGAN FUNCTION**

(30) Priority: 17.07.2017 US 201762533636 P
(62) Divisional of application: 18835689.3
(71) Applicant: Bioincept LLC, Cherry Hill, NJ 08003 (US)
(72) Inventor: BARNEA, Eytan, R., New York, NY 10016 (US)
(74) Representative: Heath, Abigail

(57) **Abstract**

This application describes compounds that are preimplantation factor (PIF) peptides, mimetics thereof, pharmaceutically acceptable salts thereof, or combinations thereof. This application also describes the use of those compounds for improving transplant tolerance, for restoring endocrine function, and for the treatment of transplant recipients of partial endocrine tissue grafts.

## Description

### CROSS-REFERENCE TO RELATED APPLICATIONS

This application claims priority to and the benefit of U.S. Provisional Application No. 62/533,636, filed July 17, 2017, which is hereby incorporated by reference in its entirety.

### FIELD OF INVENTION

The disclosure relates to methods of promoting, enhancing and/or restoring endocrine function in a transplant recipient. The disclosure also relates to the simultaneous restoration of local and systemic function of endocrine tissue for more than about 20 days, including ovarian tissue.

### BACKGROUND

Adrenal insufficiency is inability of adrenal gland to release adequate amount of hormones. Its genetically inherited form, Congenital adrenal hyperplasia (CAH), due to deficiency of 21-hydroxylase, is the most common endocrine genetic disorder in humans, presenting with clinical symptoms of virilization, neuroendocrine perturbations and metabolic disease. Current treatment algorithm with glucocorticoid substitution can reverse these symptoms only partially and exhibit the unpleasant side effects. Restoration of normal adrenal function by adrenal cell transplantation would be eminently suited to treat this common and sometimes disturbing disease. Transplanted adrenocortical cells would respond on physiological demands and reconstitute endocrine feed-back including the circadian rhythm of hormone secretion. However, this strategy is extremely limited due to requirement of life-long use of immune suppressive drugs [1, 2]. These drugs can lead to opportunistic infections, organ dysfunction and diabetes among others [3]. Importantly, such side effects may lower compliance, causing rejection of the organ [3]. Intense efforts are ongoing to overcome those limitations, for example, by using organ encapsulation as recently reported [4]. Further improvements are required for the common use of such transplants.

Mammalian pregnancy, whether through natural conception, donor embryo acceptance or cross-species embryo transfer, involves the successful transplant of a semi-allogeneic or allogeneic graft. Paradoxically, the maternal immune system remains competent and active during pregnancy and does not reject the fetus, as it would any other transplant [1]. Embryo rejection, in fact, indicates a pregnancy complication. It is also noteworthy that autoimmune conditions, unless severe, improve during pregnancy only to recur post-partum,
indicating the existence of a unique temporary immunological milieu specific to pregnancy [2-4]. No other condition quite replicates these observed phenomena. Despite extensive investigations, an inclusive explanation as to why the fetus is offered special immunologic privilege has not been forthcoming [5,6]. It is assumed, however, that pregnancy-specific compounds play a key role in such singular immune modulation [7].

Trophoblast cells, which form the epithelial part of the placenta, play a key role in maintaining tolerance to the fetus [8]. Extravillous trophoblasts (EVTs), which invade the decidual stroma (interstitial invasion) and open the uterine spiral arteries [9-11], selectively express HLA class I molecules throughout gestation [10]. EVT cells express the non-classical class Ib antigens (Ag) HLA-G, HLA-E and HLA-F, and HLA-C, a non-classical class Ia Ag. However, they lack HLA-A and -B, both T-cell related HLA ligands [12], probably to prevent attack by maternal cytotoxic CD8+ T lymphocytes. Progesterone (P4) promotes trophoblast invasion [13], and it increases HLA-G expression in primary trophoblasts and JEG-3 cells [14-16]. In JEG-3 cells, P4 is able to induce heterotopic associations of HLA-G/HLA-E and cell-surface expression of HLA-C, -E and -G [15, 17]. However, early in gestation, P4 is of corpus luteum origin, and the level of circulating P4 is low [18]. Effective local steroid production is only taken over by the placenta by week 12 of gestation [19]. Thus, the role of pregnancy specific endogenous compound(s) in regulating trophoblast class I HLA molecules remains currently incomplete. Our premise is that immune modulation and embryo/fetus acceptance are specifically embryo-derived and embryo-driven, in coordination with the maternal immune response.

Preimplantation factor (PIF), a small peptide secreted by viable embryos, is likely to play an important role in maternal recognition that leads to fetal-tolerance [20-22]. PIF is detectable as early as the two-cell stage and its levels in culture are associated with embryo development [23, 24]. Circulating PIF levels in early pregnancy also correlate with a favorable pregnancy outcome [25]. PIF has an essentially autotrophic effect on embryo development, which is blocked by anti-PIF antibody [23]. In the embryo, PIF targets protein-disulfide isomerase/thioredoxin and heat shock proteins (HSPs), promoting embryo development and protecting against serum toxicity [21, 23, 24]. Additionally, PIF lowers natural killer (NK) cell toxicity [26]. PIF promotes endometrial receptivity to support embryo implantation [28-30]. It regulates both systemic and local maternal immunity, creating Th2 bias while preserving an effective anti-pathogenic Th1 response [27, 32, 33]. These findings have been translated to treatment of diverse immune disorders, transplantation, and brain injury models outside pregnancy, and have led to a Fast-Track FDA clinical trial for autoimmune disease recently completed satisfactorily (NCT02239562) [34-41].

PIF expression in the placenta is highest shortly post-implantation and declines at term [20, 25, 42]. A premature decline in PIF has been associated with preeclampsia and intrauterine growth retardation, thus evidencing the peptide's important role in maintaining effective placental function [41, 43]. PIF promotes invasion by EVTs, without affecting proliferation [42, 44]. Its effect on EVTs is dependent on increased metalloproteinase and reduction of its inhibitor. Pathway analysis demonstrated that PIF action is dependent on the MAPK, PI3K, and JAK-STAT pathways [42]. As recently reported, PIF acts on, and its effect is dependent on, critical apoptosis regulating the p53 pathway [46]. Presently, there is limited information on local compounds that regulate HLAs expression, especially during the earliest stages of pregnancy when it is most critical. PIF is secreted by viable embryos; similarly, soluble HLA-G is secreted by several viable embryos [23, 48]. Thus, both have an intimate interaction from the earliest stages of embryos development. This interaction between PIF and HLA-G continues as both are found in the maternal circulation of viable pregnancy. Considering that both ligands are expressed by the trophoblast, this supports the premise that PIF (due its immune regulatory properties) may have a local regulatory role on trophoblast HLA class I function. Such information would further substantiate PIF's important role in early pregnancy events.

### SUMMARY OF EMBODIMENTS

One of the promising therapeutic agents, which might improve the outcome of the transplantation without systemic immune suppression, could be Preimplantation Factor (PIF). Organ transplantation is of common use for bone marrow and solid organs. These procedures generally are necessary and frequently are life-saving, addressing restoration of vital organs function. Despite intense efforts to find a matching donor, life-long use of immune suppressive drugs post-transplant is generally is required [1, 2]. Graft maintenance drugs which are immune suppressive agents, can lead to serious opportunistic infections, organ dysfunction and diabetes development among others [3]. Importantly, such side effects may lower compliance, causing rejection of the organ [3]. Relevant for solid organs, such as heart liver, kidney once organ is transplanted and if not rejected, will start to function without a minute by minute requirement of a feed-back loop. Therefore the adaptation of the transplant organ to the organism can be gradual and progressive.

Ovary, pancreatic islets and adrenal gland transplantation is more limited since hormones can replace those deficiencies [4, 5]. In addition, for maintaining these organs, immune suppressive agents are required which are associated with side effects. In contrast with solid organ transplants, endocrine organs require almost a real time feed-back for addressing their required function. Therefore, following their transplantation the transplant not only has to survive rejection but the organ must release the relevant hormone and uniquely integrate and function through a very tight coordination. The hormone released will have to respond to the organism's needs and get the feed-back to the transplant from trophic hormones or glucose in case of pancreatic islets. Currently, hormonal replacement therapies have severe limitations. In the case of the adrenals, the need for using continuous steroids leads to several complications such as weight gain, diabetes, hypertension, and immune suppression.

In the case of the ovaries, hormonal replacement is effective, but does not restore effective cyclic function that could lead to reproduction. In the case of pancreatic islets, insulin injections are required sometime even several times a day to maintain function. Endocrine organ transplants have a rigorous feedback mechanism which is not replicated with current replacement therapy and poses the greatest challenge.

For example, in the adrenals the cortisol secretion follows a circadian rhythm which when perturbed, leads to serious dysfunction. This feed-back loop is composed of the hypothalamus originated cortico- releasing hormone, the pituitary ACTH and the adrenal gland where through steroidogenesis cortisol is released to the circulation which feeds back to the pituitary completing the loop. Replacement therapy sometimes fails to prevent an acute adrenal crisis and most often does not lead to restoration of well-being. Bornstein, S.R., Predisposing factors for adrenal insufficiency. N Engl J Med, 2009. 360(22): p. 2328-39.

In case of the ovaries, menstrual function is a highly coordinated process. Following menstruation, the cycle continues through the follicular, ovulation, and secretory phases which again lead up to the next menstruation. The cycle continues for four weeks on a monthly basis. Therefore, for a transplanted ovary to effectively function, the prime driver is GNRH- gonadotropin releasing hormone, which controls the pituitary FSH and LH, which regulates ovarian steroidogenesis. This sequence of events; FSH followed by LH dominance, assures proper cyclic function - any perturbation of this sequence of events will lead to lack of ovulation and even arrest of menstrual function.

In the case of the islet cells of the pancreas, they act as circulating glucose sensors leading to increased secretion. When glucose levels rise, the response has to be exact since too little insulin will result in very high glucose levels or excess insulin secretion lowering glucose levels which could be life-threatening.

Islet cell transplants are limited due to cells exhaustion, inflammation and immune rejection ovary (except auto-transplant) and adrenal gland transplants are rarely, if ever, performed. In the case of islet cell transplants, various encapsulation methods are being developed for isolating the cells to segregate them against attack by the host's immune system. Alternatively, when multi-visceral transplantation is carried out, the pancreas can be transplanted as well, but this demands for lifelong immune suppression [4]. Further improvements are required for the common use of such transplants.

In pregnancy, where semi and allogeneic fetus (donor embryo leads to successful outcomes, indicates that understating this process and mechanisms involved may provide a suitable answer to enable more effective endocrine organ transplants while mitigating the need for immune suppressive agents. Immunosuppressive agents at present are less practical, since replacement therapy is available.

We discovered that PIF is secreted by viable embryos from the earliest stages of pregnancy due to its immune regulatory properties leading to our discovery of successful monotherapy ovarian allotransplant in a primate model. Total ovarian function was restored providing evidence of non-rejection long term post treatment and total restoration of ovarian cyclic function- evidence that the hypothalamic pituitary ovarian axis has been restored. The onsite acceptance of the ovary without signs of rejection was also coupled with accelerated and complete surgical wound healing including hair regrowth. This dual improvement makes PIF an attractive agent for ovarian allotransplantation. With the goal also to lead to adrenal transplantation, the data generated with PIF documented that short term exposure to bovine adrenal cells in culture prevent cell exhaustion, maintained cortisol secretion and led to long term adrenal cells function once encapsulated. This opens the opportunity for such xenotransplantation.

Pregnancy is unique since it enables a semi and allogeneic embryo transfer success [7]. Moreover, cross species embryo transfer can be successful as well [5]. Thus genetics does not appear to play an important role in reproductive success. Paradoxically, during pregnancy instead of immune suppression, anti-pathogen activity is largely preserved as well as autoimmune disorders can improve unless severe. [6, 7] This protection against autoimmunity is pregnancy specific since post-partum or even earlier, after miscarriage, flare up may occur [2, 3, 20]. The above supports the view that from earliest stages of pregnancy such protective mechanisms are operative.

The embryo surrounded by the zona pellucida secretes PIF, which exerts a direct autotrophic and protective effect negating adverse maternal environment [16, 8]. Therefore semi and allogeneic (donor) embryos can be self-sustained in the maternal organism prior to implantation. In this context, PIF was shown to target the embryo to reduce oxidative stress and protein misfolding critical for survival by targeting PDI/thioredoxin and HSPs [17]. Similar protein targets also have been identified in human immune cells

The disclosure provides a method of modulating or maintaining endocrine function in a subject. In one aspect, the method comprises administering to the subject a therapeutically effective amount of a Preimplantation Factor (PIF) peptide, compositions thereof, mimetics thereof, pharmaceutically acceptable salts thereof, or combinations thereof of any of the aforementioned.

The disclosure also provides a method of enhancing endocrine function in a subject. In one aspect, the method comprises administering to the subject a therapeutically effective amount of a PIF peptide, compositions thereof, mimetics thereof, pharmaceutically acceptable salts thereof, or combinations thereof.

The disclosure also provides a method of restoring endocrine function in a subject. In one aspect, the method comprises administering to the subject a therapeutically effective amount of a PIF peptide, compositions thereof, mimetics thereof, pharmaceutically acceptable salts thereof, or combinations thereof.

The disclosure also provides a method of xenotransplantation to a subject. In one aspect, the method comprises: culturing a cell from a non-human animal in a medium comprising a PIF peptide, and transplanting the cell into the subject.

The disclosure also provides a method of increasing the likelihood of success of a xenotransplantation in a subject. In one aspect, the method comprises: (i) administering to a nonhuman animal a therapeutically effective amount of a PIF peptide, compositions thereof, mimetics thereof, pharmaceutically acceptable salts thereof, or combinations thereof; and (ii) transplanting a cell, tissue, or organ from the nonhuman animal into the subject.

The disclosure also provides a method of restoring endocrine tissue function after transplantation of the endocrine tissue in a subject. In one aspect, the method comprises administering to the subject a therapeutically effective amount of a PIF peptide, compositions thereof, mimetics thereof, pharmaceutically acceptable salts thereof, or combinations thereof.

The disclosure also provides a method of modulating expression of CYP17A1 or IL-10 in one or a plurality of adrenocortical cells. In one aspect, the method comprises contacting the adrenocortical cell with an effective amount of a PIF peptide, compositions thereof, mimetics thereof, pharmaceutically acceptable salts thereof, or combinations thereof.

The disclosure also provides a method of inducing transplant tolerance of a semi-allogeneic and/or xeno-embryo in a subject by increasing expression of HLA-Class I molecules in the subject or on the embryo to an amount sufficient to increase the likelihood of transplant acceptance of the embryo as compared to the levels of HLA Class I molecules on an embryo or in a subject not treated with a PIF peptide, compositions thereof, mimetics thereof, pharmaceutically acceptable salts thereof, or combinations thereof. In one aspect, the method comprises contacting the embryo and/or the subject with a therapeutically effective amount of a PIF peptide, compositions thereof, mimetics thereof, pharmaceutically acceptable salts thereof, or combinations thereof.

The disclosure also provides a method of inducing transplant tolerance of one or a plurality of donor semi-allogeneic cell and/or cells derived from a species other than the transplant recipient in a recipient subject by increasing expression of HLA-Class I molecules in the subject or on the donor cells to an amount sufficient to increase the likelihood of transplant acceptance of the cells as compared to the levels of HLA Class I molecules on donor cells or in the subject untreated with a PIF peptide, compositions thereof, mimetics thereof, pharmaceutically acceptable salts thereof, or combinations thereof. In one aspect, the method comprises contacting the one or plurality of donor cells and/or the subject with a therapeutically effective amount of a PIF peptide, compositions thereof, mimetics thereof, pharmaceutically acceptable salts thereof, or combinations thereof.

The disclosure also provides a method of inducing expression of an HLA-Class I molecule in a subject and/or in a donor tissue. In one aspect, the method comprises contacting the donor tissue and/or administering to the subject a therapeutically effective amount of a PIF peptide, compositions thereof, mimetics thereof, pharmaceutically acceptable salts thereof, or combinations thereof.

The disclosure also provides a method of restoring menstruation in a mammal in need of restoration. In one aspect, the method comprises administering to the mammal a therapeutically effective amount of a PIF peptide, compositions thereof, mimetics thereof, pharmaceutically acceptable salts thereof, or combinations thereof.

The disclosure also provides a pharmaceutical composition. In one aspect, the composition comprises comprising: (i) a therapeutically effective amount of a PIF peptide, compositions thereof, mimetics thereof, pharmaceutically acceptable salts thereof, or a combinations thereof; (ii) a therapeutically effective amount of a steroid; and (iii) a pharmaceutically acceptable carrier.

The disclosure also provides a method of promoting or enhancing wound healing in a subject. In one aspect, the method comprises: administering to the subject a pharmaceutical composition comprising a therapeutically effective amount of PIF peptide, compositions thereof, mimetics thereof, pharmaceutically acceptable salts thereof, or a combination thereof.

In some embodiments, the methods provided herein may be free of a step of administering to the subject and/or free of contacting the embryo with an active agent other than PIF peptide, compositions thereof, mimetics thereof, pharmaceutically acceptable salts thereof, or a combinations thereof.

In some embodiments, the methods provided herein may further comprise administering to the subject a therapeutically effective amount of a steroid.

In some embodiments, the methods provided herein may further comprise a step of culturing donor endocrine tissue prior to transplanting the endocrine tissue.

### BRIEF DESCRIPTION OF DRAWINGS

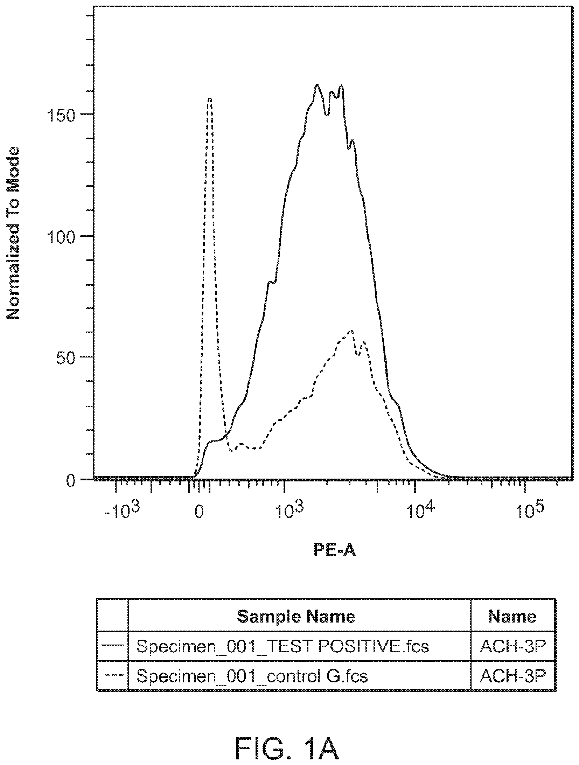

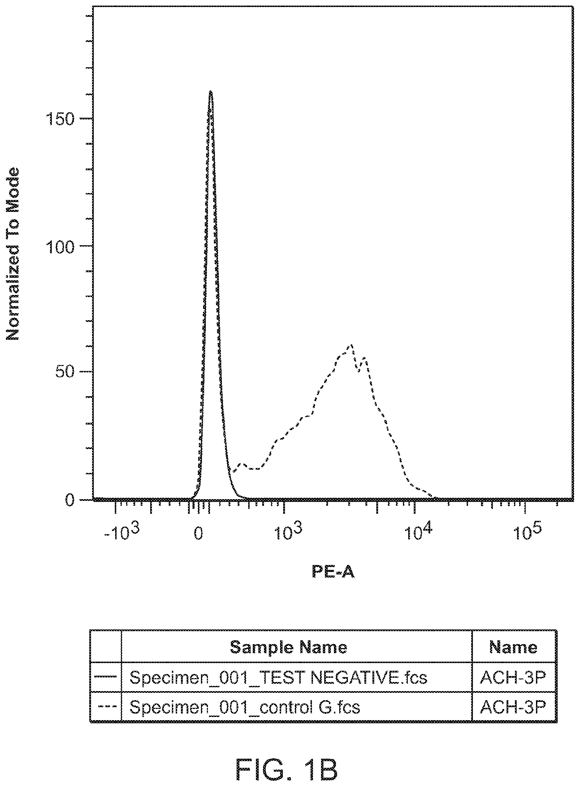

FIG. 1A illustrates HLA-G expression in JEG-3 cells compared with ACH-3P cells. Data shows low expression of HLA-G in ACH-3P cells (FIG. 1B). There the study was conducted using JEG-3 cells.

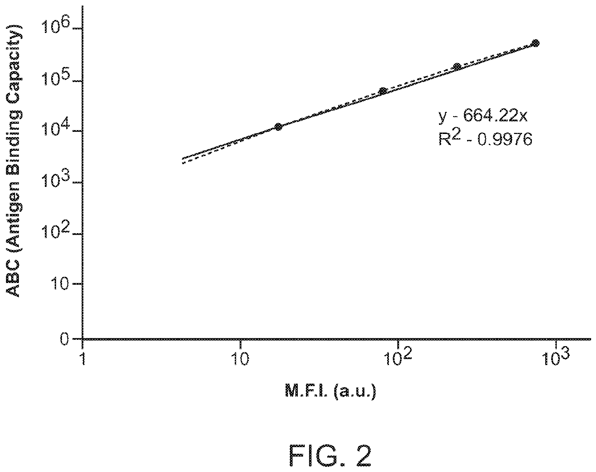

FIG. 2 is a graph showing bead population MFI calibration versus antigen binding capacity. In order to determine the level of expression of each antigen, as tested by using MFI, a calibration curve was set up. The vertical axis reflects the antigen-binding capacity, which is plotted against the horizontal axis MFI. The calculation is based on the best fit where R²= 0.99.

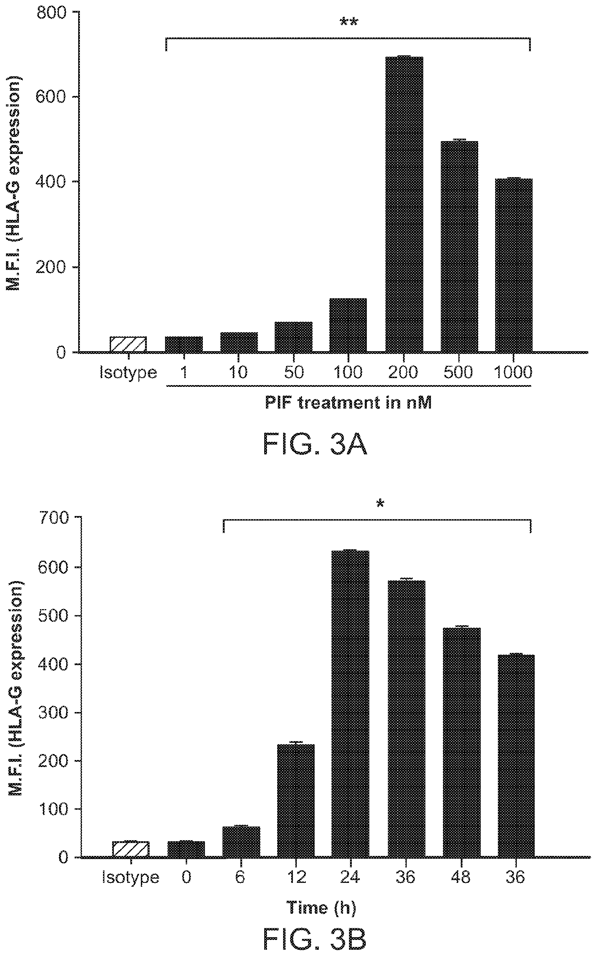

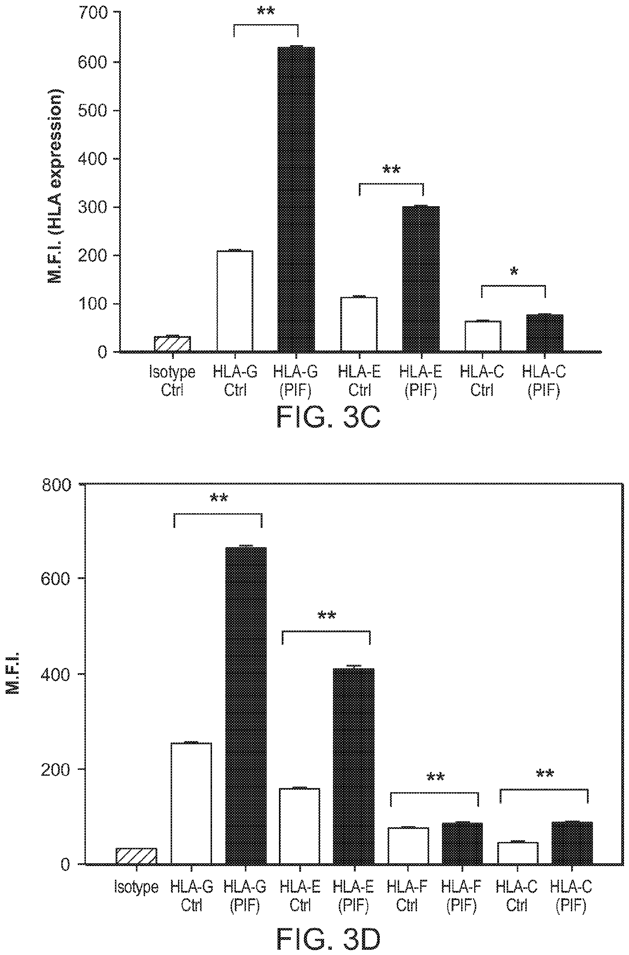

FIG. 3A-FIG. 3D illustrate the effect of PIF (1-1000 nM) on HLA class I molecule expression by JEG-3 cells. After 0-72 hours of culture, the effect was determined by Western blotting and flow cytometry using isotype as control. FIG. 3A: Results showed that PIF increased HLA-G expression in JEG-3 cells, attaining maximal effect at a concentration of 200 nM. FIG. 3B: The maximal effect of PIF was noted at 24 hours of incubation. FIG. 3C: PIF at 200 nM and 24 hours incubation promoted the expression of HLA-G, HLA-E, and slightly increased HLA-C expression cell surface. FIG. 3D: PIF at 200 nM and 24 hours incubation promoted the expression of HLA-G, HLA-E, moderately increasing HLA-C and HLA-F expression intracellularly. The data are mean ±SE of triplicates repeated three times. * p<0.05, ** p<0.01.

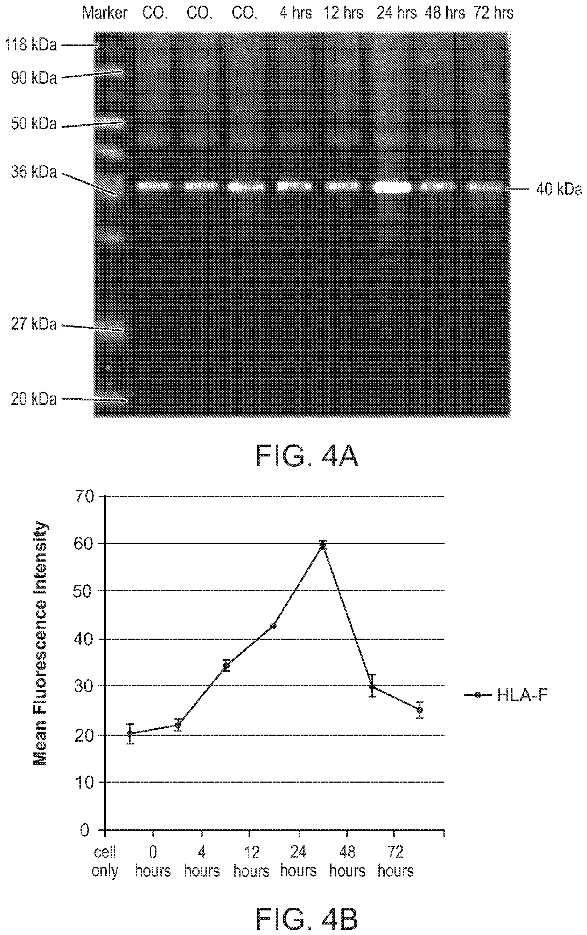

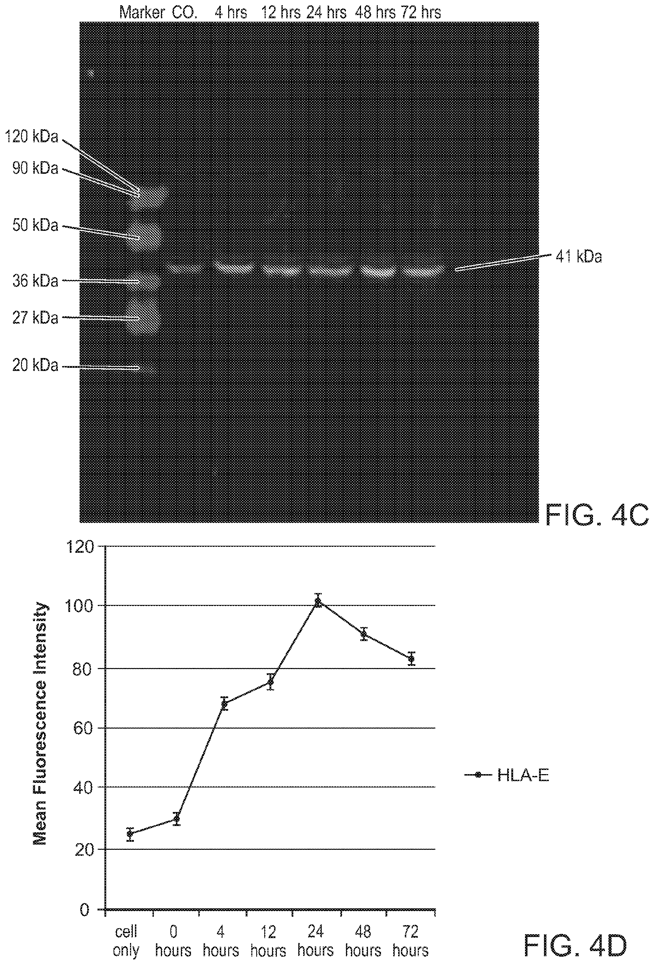

FIG. 4A-FIG. 4D illustrate cell surface expression of HLA-F and HLA-E induced by PIF at 200 nM PIF, with stimulation at 4, 12, 24, 48 and 72 hours. FIG. 4A shows a Western blot of the total protein expression of HLA-F, as induced by PIF stimulation. FIG. 4B is a graph showing cell surface expression of HLA-F, as induced by PIF. FIG. 4C shows a Western blot of the total protein expression of HLA-E, as induced by PIF stimulation. FIG. 4D quantifies cell surface expression of HLA-E, as induced by PIF. For each experiment, controls used were only cell samples.

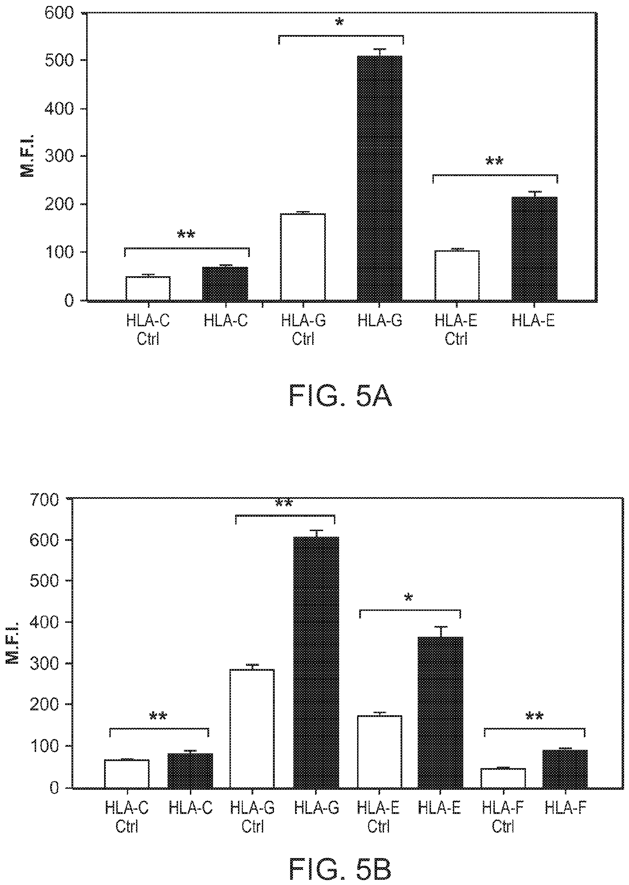

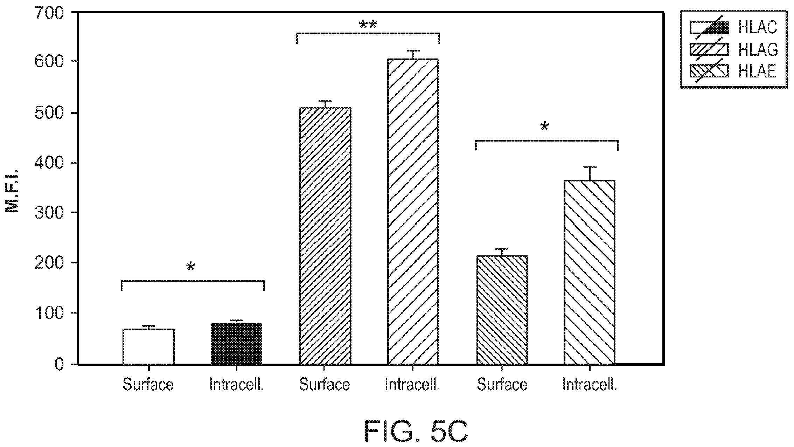

FIG. 5A-FIG. 5C illustrate how progesterone promotes HLA class I molecule expression by JEG-3 cells. The effect of P4 on HLA I subtypes was examined in JEG-3 cells using the maximally effective P4 concentration 1 µg/ml, with cells cultured for 24 hours. Data were analyzed by Western blotting and flow cytometry. FIG. 5A is a graph of data showing that P4 increased production of all tested HLA antigens intracellularly, HLA-G being the most increased ligand followed by HLA-E. FIG. 5B is a graph showing P4 induced significant HLA-C, -E, -F and -G expression on the cell surface. FIG. 5C is a graph of data demonstrating that HLA expression was higher in the intracellular domain as compared to an extracellular location. *P<0.01, **P<0.05, mean+/-SEM.

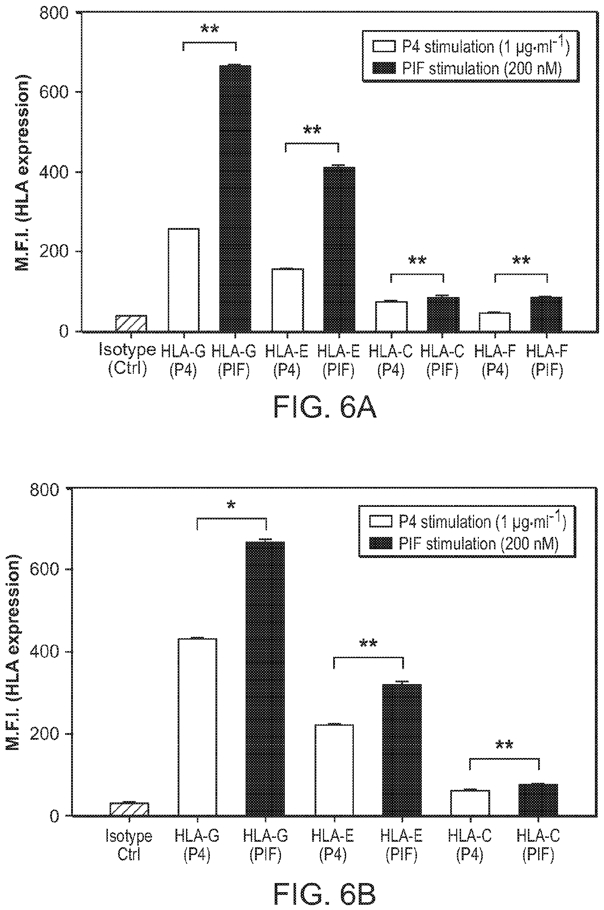

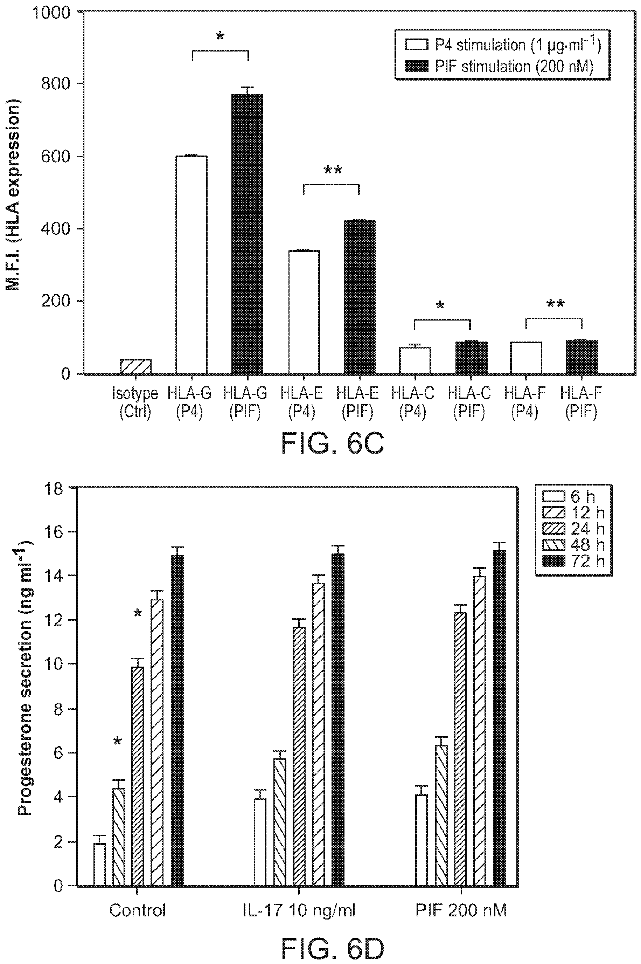

FIG. 6A-FIG. 6D illustrate the effect of PIF and P4 on HLA class I antigen expression, and the effect of IL-17 on P4 secretion by JEG-3 cells. The effect of 200 nM PIF on HLA class I expression by JEG-3 cells was compared with that of 1 µg/ml P4, using Western blotting and flow cytometry. The effect of 200 nM PIF and 10 ng/ml IL-17 on P4 secretion by JEG-3 cells was also tested, using ELISA, after cells were incubated for 6-72 hours. FIG. 6A: PIF induced a significant increase in HLA-G and HLA-E as compared to P4. The effect of PIF on HLA-C and F expression was mild. FIG. 6B: PIF had a greater effect than P4 in promoting HLA class I intracellular expression. FIG. 6C: PIF increased the expression of HLA class I molecules on the cell surface, particularly HLA-G and HLA-E expression. FIG. 6D: Both IL-17 and PIF increased P4 secretion by JEG-3 cells in a time dependent manner. * P<0.05, ** P<0.01.

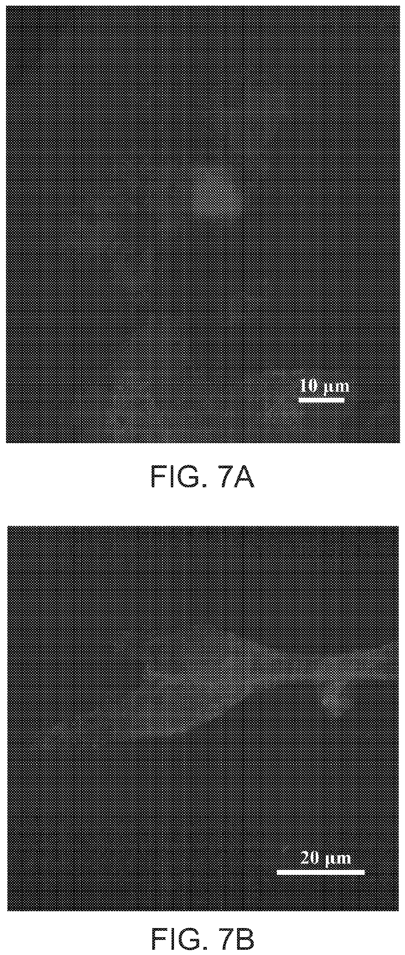

FIG. 7A-FIG. 7B show image analysis demonstrating PIF-induced up-regulation of cell surface expression of HLA-G in JEG-3 cells, as determined by confocal microscopy analysis. Acquisition was carried out in stacks, resulting in 3-D images. FIG. 7A shows a cell only sample. FIG. 7B shows a PIF-treated sample. Light grey, anti-HLA-G antibody; dark grey-black, DAPI stain. In original colored renditions of the photograph, cells have surface expression of HLA-G, whereas the blue staining of DAPI is only visible in the nucleus of the cells. This is to show how HLA-G is present on the surface of the cells.

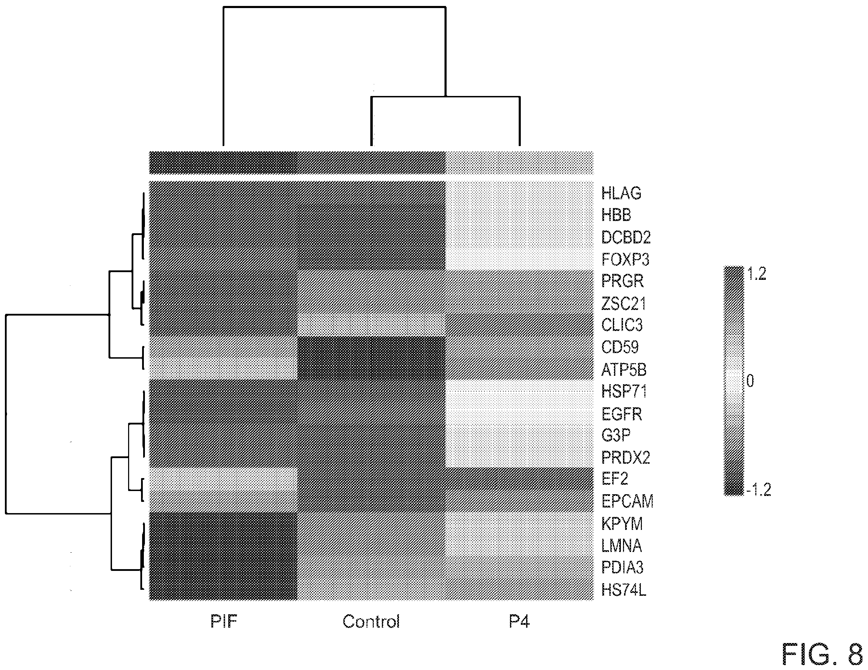

FIG. 8 shows a heatmap of protein expression and hierarchical clustering of the proteins and treatment. Heatmap of median centered protein expression data with horizontal hierarchical clustering of different proteins using the median linkage agglomeration method and vertical hierarchical clustering of treatment conditions using complete linkage agglomeration. Correlation distance metric was used for clustering data.

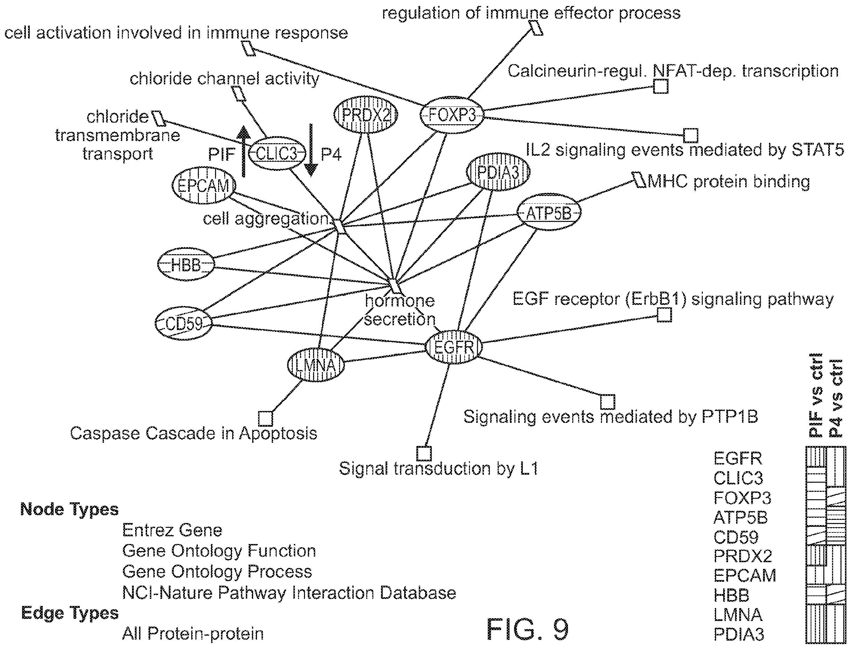

FIG. 9 illustrates exploratory Gene Association Networks analysis of PIF and P4: treated vs. non-treated control cells. Protein-protein interaction and protein annotation are depicted in network linkage fashion, where up-regulated proteins are indicated with a dark grey border and down-regulated proteins with a light grey border. Border width is proportional to protein differential expression probability. GO and NCI-Nature Pathway Annotations are depicted and color coded. The heatmap represents fold expression compared to control. CLIC3 is the only quality difference, being up-regulated by PIF and down-regulated by P4, when compared to control.

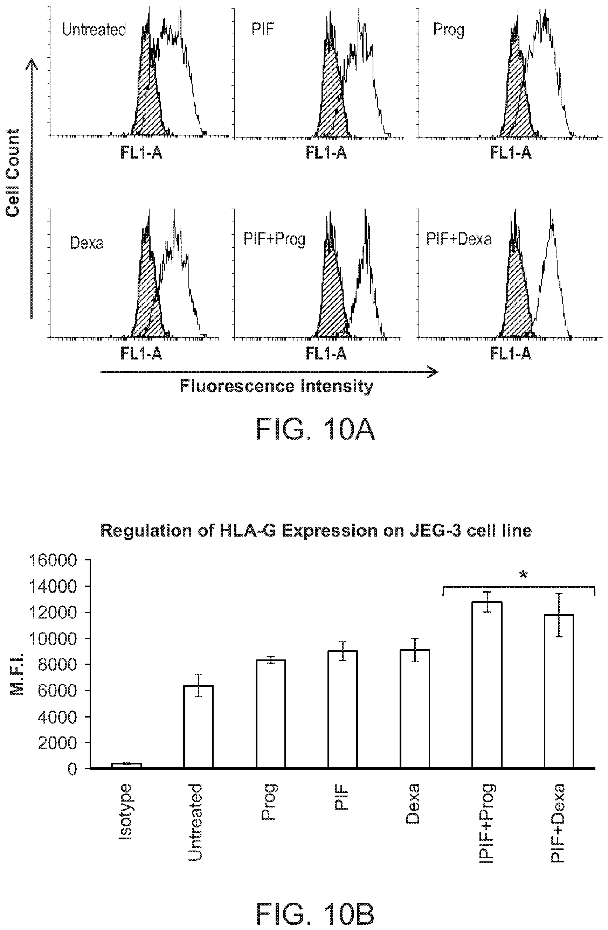

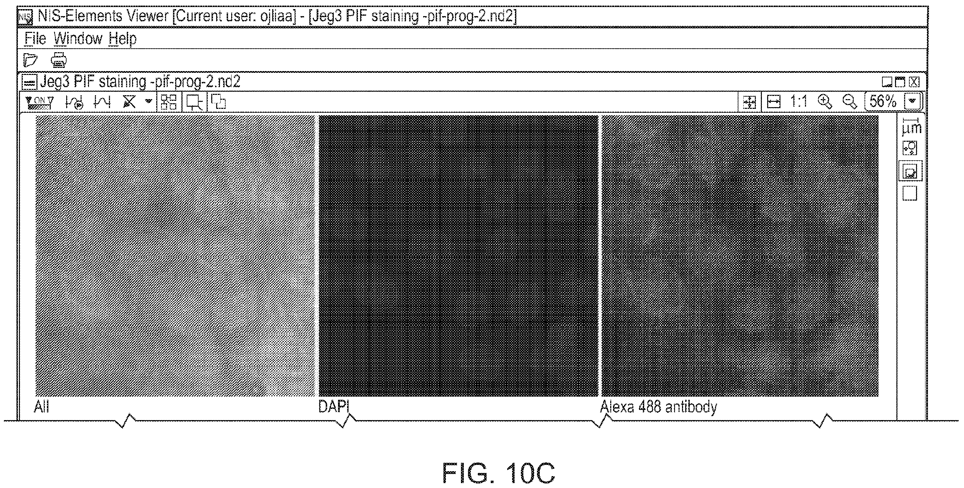

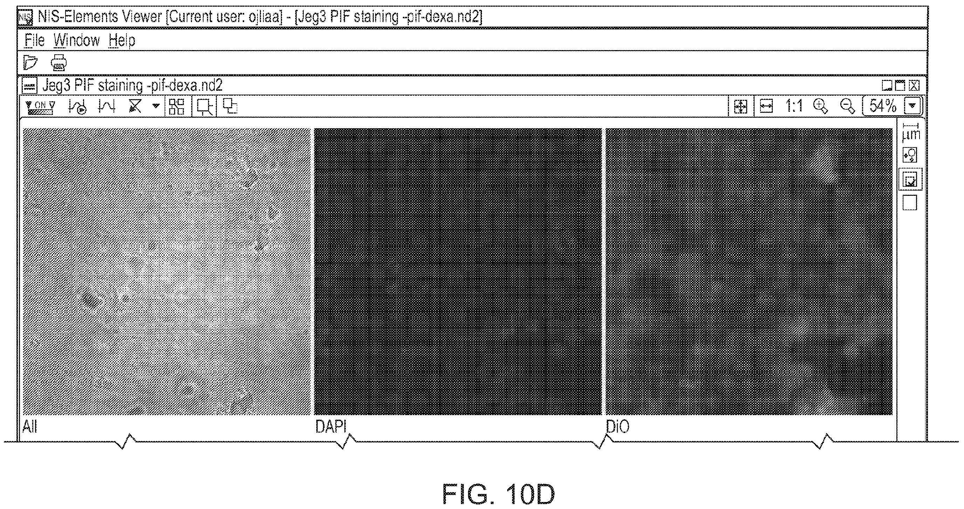

FIG. 10A-FIG. 10D illustrate the effect of PIF, Progesterone, and Dexamethasone on the expression of HLA-G on JEG-3 cells. JEG-3 cells were incubated with PIF (200 nM), Progesterone (1 µg/ml) and Dexamethasone (1 µg/ml) either alone or in combination as (PIF with Progesterone and PIF with Dexamethasone) for 24 hours. The cells were then incubated with the HLA-G specific mouse monoclonal antibody MEM-G9m followed by incubation with and Alexa Fluor 488 conjugated antibody preparations. Fluorescence was detected by using BD Accuri C6 flow cytometer. The data was analyzed using a Flowing Software V2.5.1. FIG. 10A shows representative histogram overlays of three independent experiments, where grey filled histogram indicates isotype control and line histogram represents fluorescence intensity of the treated samples. FIG. 10B is a bar graph showing the mean fluorescence intensity (MFI) of three independent experiments obtained following incubations with progesterone (Prog), PIF, dexamethasone (Dexa), PIF+Prog and PIF+Dexa. Error bar indicates standard deviation from the mean. Statistical analysis was performed with SigmaPlot software. One way Analysis of Variance was employed to test the significance of differences among groups. Double treatments PIF+Prog and PIF+Dexa were statistically significantly different (*) (P <0.05) from individual treatments. FIG. 10C shows image analysis demonstrating PIF added in combination with P4, and FIG. 10D shows image analysis demonstrating PIF added in combination with Dexa induced upregulation of surface expression of HLA-G in JEG-3 cells determined by confocal microscopy analysis. Acquisition was carried out in stacks, resulting in 3-D images. Cell only sample, DAPI blue stain, Fluorescent imaging.

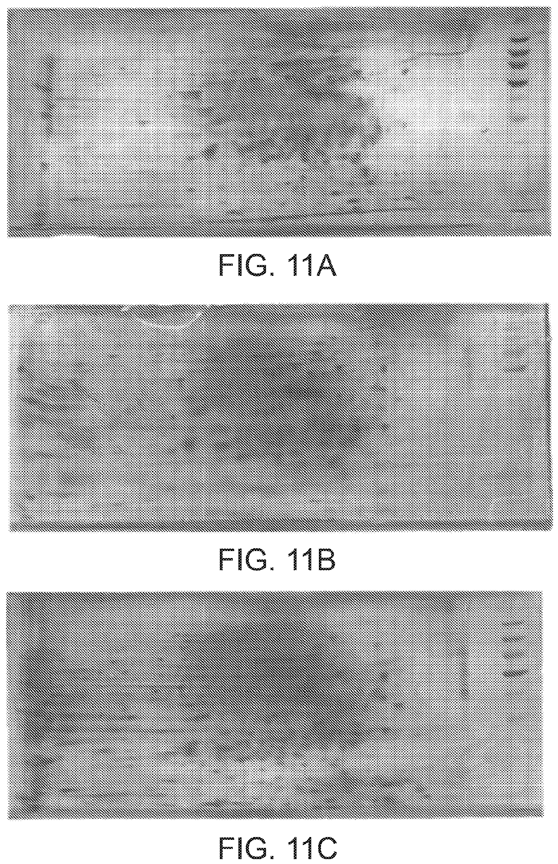

FIG. 11A-FIG. 11C are representative 2-DE displays of silver-stained protein extracts from subconfluent cultures of JEG-3 cells after incubation in DMEM/F-12 supplemented with 0.1% FCS under resting conditions (FIG. 11A), progesterone stimulation at a concentration of 1000 ng·ml⁻¹ (FIG. 11B), or PIF at a concentration of 200 nM for 24 hours (FIG. 11C). Mrmarkers ranging from 10-150 kDa are displayed on the right of each gel.

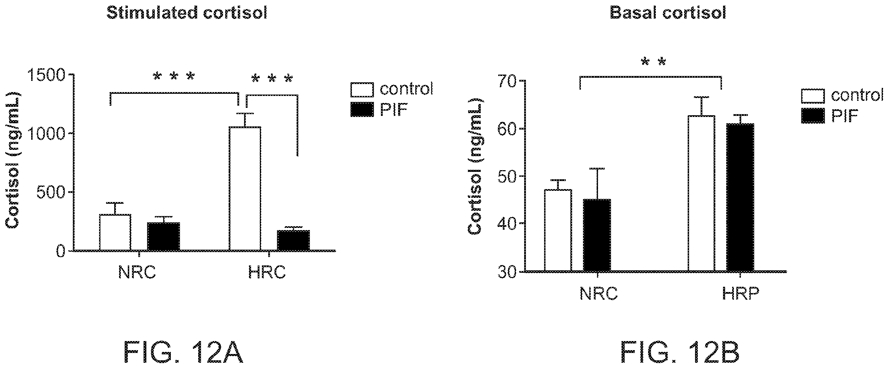

FIG. 12A-FIG. 12B illustrate the effect of PIF on cortisol production. FIG. 12A is a graph showing the influence of PIF on ACTH stimulated cortisol release. FIG. 12B is a graph showing the effect of PIF on basal cortisol production. All data presented as mean ± s.e.; n≥6 for each time point; *p≤0.05; **p ≤0.01; ***p≤0.001.

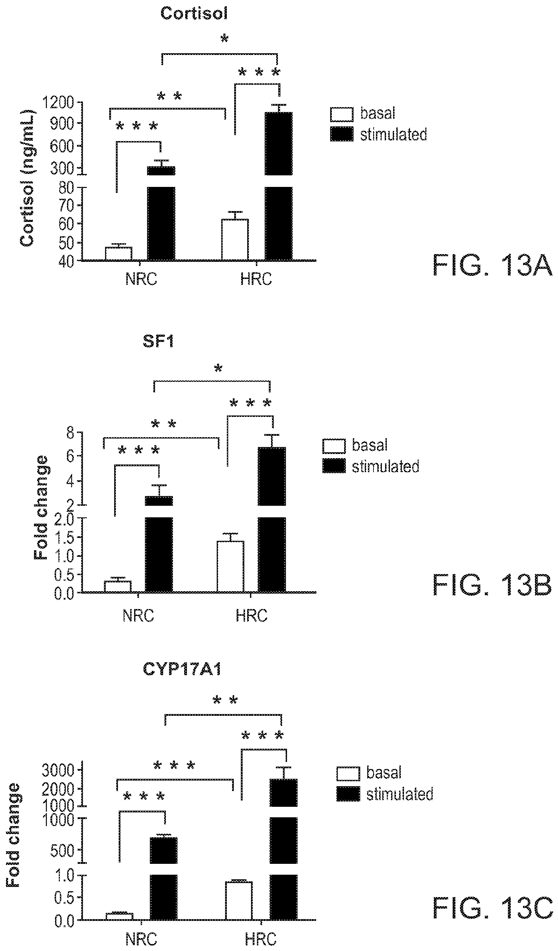

FIG. 13A-FIG. 13C illustrate the characteristic of the cells with different response to ACTH stimulation. FIG. 13A is a graph showing the characterization of normally and highly responsive cells by basal and ACTH stimulated cortisol production. The relative gene expression of SF1 is shown in FIG. 13B and of CYP17A1 is shown in FIG. 13C. All data presented as mean ± s.e.; n≥6 for each time point; *p≤0.05; **p ≤0.01; ***p≤0.001. Reference gene - *RPS9.*

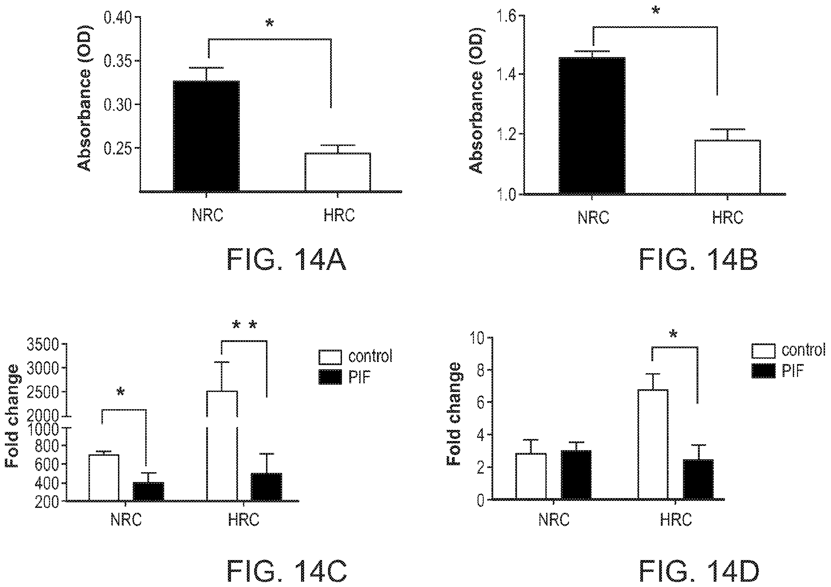

FIG. 14A-FIG. 14D illustrate the characteristic of the processes, occurring with the cells with different response on ACTH stimulation. FIG. 14A shows proliferation, FIG. 14B shows viability, FIG. 14C shows apoptosis, and FIG. 14D shows relative mRNA gene expression of IL-10 of normally and highly responsive cells. All data presented as mean ± s.e.; n≥6 for each time point; *p≤0.05.). All data presented as mean ± s.e.; n≥6 for each time point; *p≤0.05; **p ≤0.01; ***p≤0.001.

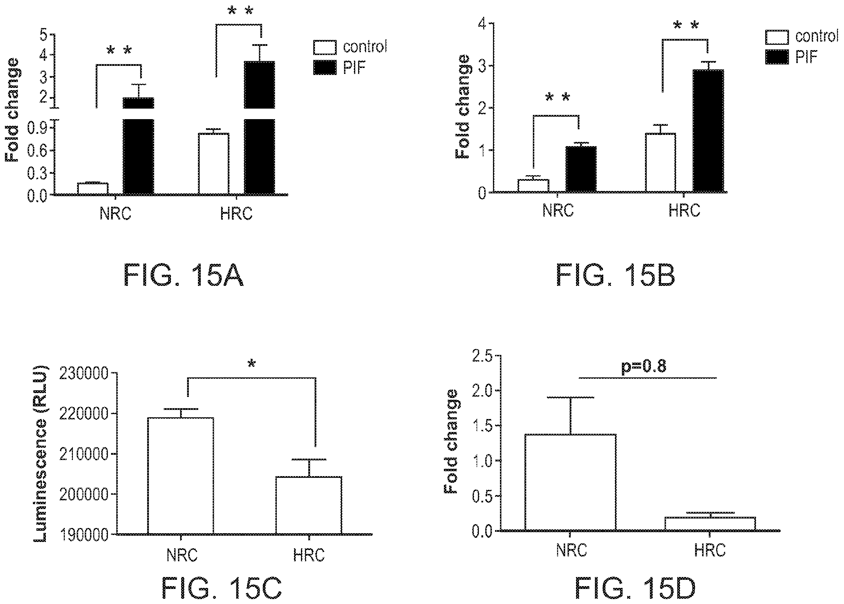

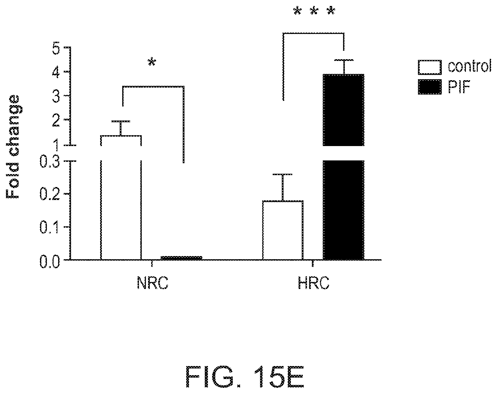

FIG. 15A-FIG. 15E illustrate the effect of PIF on mRNA gene expression. FIG. 15A is a graph showing the effect of PIF on basal gene expression of CYP17A1 and FIG. 15B is a graph showing the effect of PIF on basal gene expression of SF1. FIG. 15C shows the influence of PIF on ACTH stimulated gene expression of CYP17A1 and FIG. 15D shows the influence of PIF on ACTH stimulated gene expression of SF1. FIG. 15E shows the effect of PIF on expression of IL-10. All data presented as mean ± s.e.; n≥6 for each time point; *p≤0.05; **p ≤0.01; ***p≤0.001. Reference gene was *RPS9.*

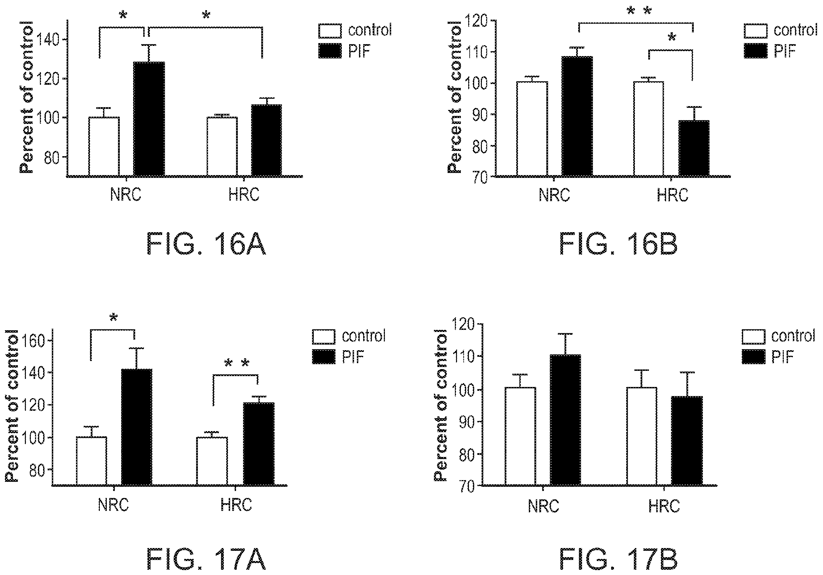

FIG. 16A-FIG. 16B illustrate the effect of PIF on cortisol secretion in encapsulated BACS until day 24. BACS culture were treated with PIF for three days. Subsequently BACS were encapsulated and cultured for additional 24 days. FIG. 16A shows the PIF effect on basal cortisol secretion by NRC and HRC. FIG. 16B shows the PIF effect on ACTH stimulated BACS. All data represented as mean± SEM; n>14 for each tome point; *p≤0.05; **p ≤0.01.
FIG. 17A-FIG. 17B illustrate the effect of PIF on cortisol secretion in encapsulated BACS on day 10. BACS culture were treated with PIF for three days. Subsequently BACS were encapsulated and cultured for additional 7 days. FIG. 17A shows the PIF effect on basal cortisol secretion by NRC and HRC. FIG. 17B shows the PIF effect on ACTH stimulated BACS. All data represented as mean± SEM; n>14 for each tome point; *p≤0.05; **p ≤0.01.

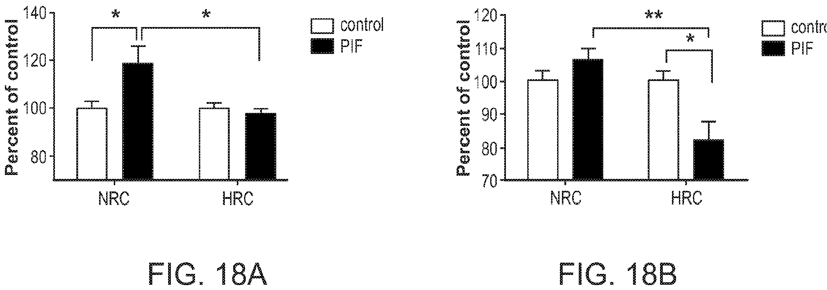

FIG. 18A-FIG. 18B illustrate the effect of PIF on cortisol secretion in encapsulated BACS on day 17-24. BACS cultures were treated with PIF for three days. Subsequently BACS were encapsulated and cultured for additional 14-24 days days. FIG. 18A shows the PIF effect on basal cortisol secretion by NRC and HRC. FIG. 18B shows the PIF effect on ACTH stimulated BACS. All data represented as mean± SEM; n>14 for each time point; *p≤0.05; **p ≤0.01.

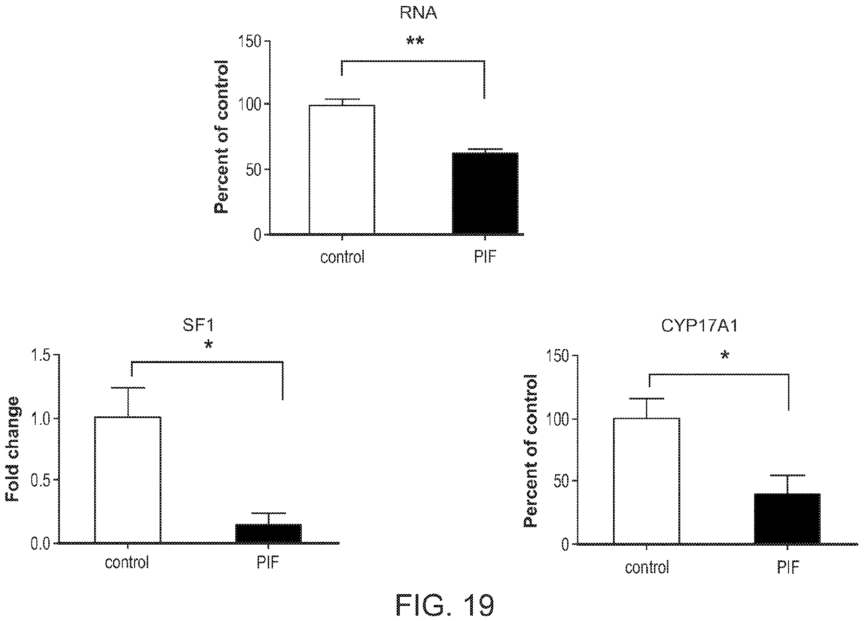

FIG. 19 illustrates the effect of PIF on mRNA gene expression on day 24. BACS cultures were treated with PIF for three days. Subsequently BACS were encapsulated and cultured for additional 24 days. Effect on global RNA, SF1, and CYP17A1 are shown. All data presented as mean ± SEM; n≥8 for each time point; *p≤0.05; **p ≤0.01; Reference gene was *RPS9.*

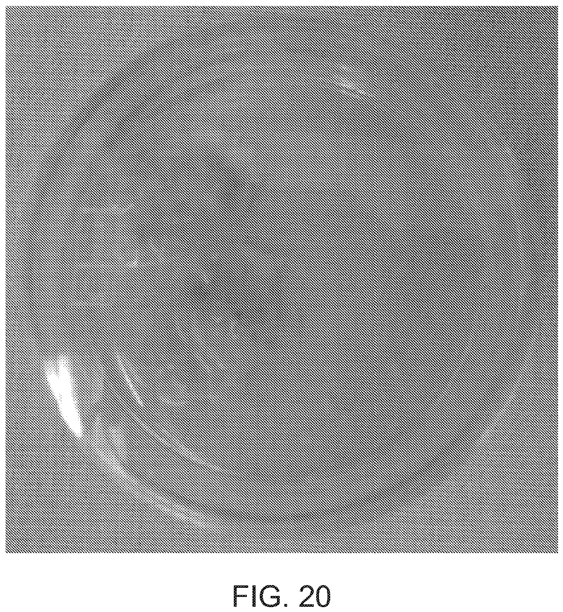

FIG. 20 is a picture of prepared thin ovarian cortical sections before transplantation.

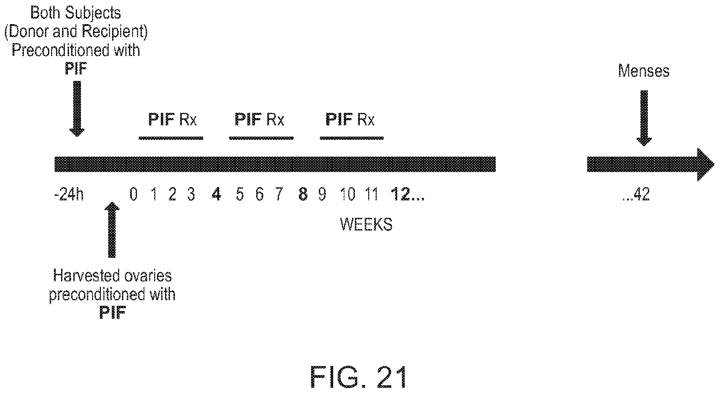

FIG. 21 is a chart depicting methods of PIF monotherapy as transplantation preconditioning and maintenance. Each baboon was preconditioned with PIF prior to ovariectomy and received PIF Rx bid (3 weeks on, 1 week off) for 12 weeks as transplant maintenance. Both baboons were monitored for additional 6 months without any added treatment. Return to function (menstruation) was documented in one subject at week 42.

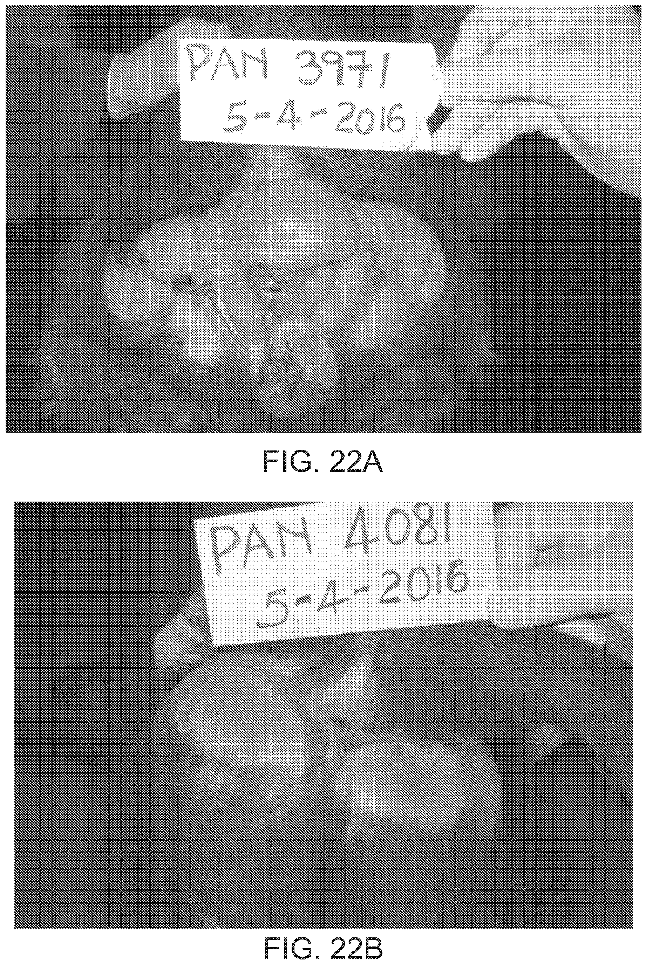

FIG. 22 are pictures documenting the return to menstrual function as it is evidenced by the typical swelling of the peritoneum after transplantation procedure of endocrine tissue.

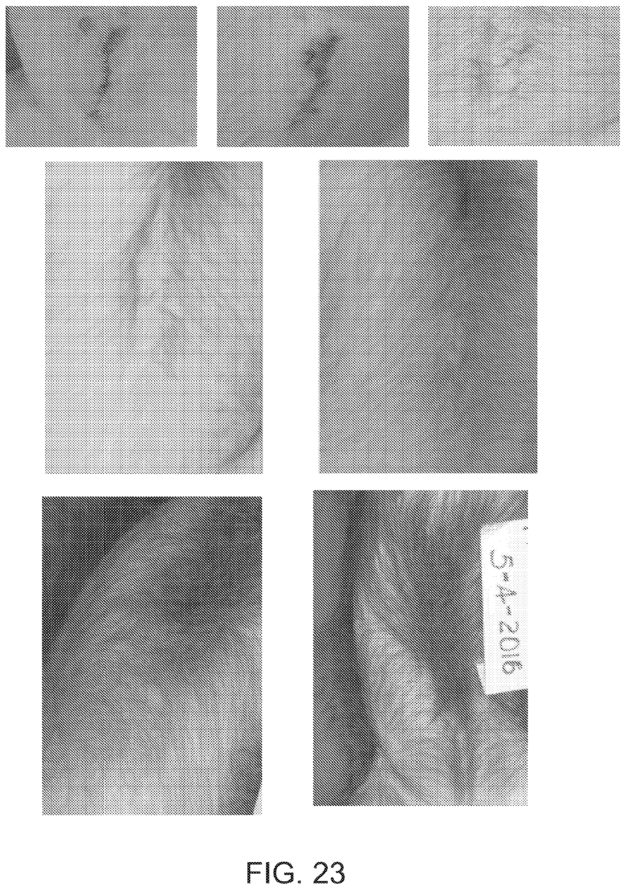

FIG. 23 is a series of pictures showing how PIF promotes laparotomy scar healing. The scarless wound also led to restored hair growth.

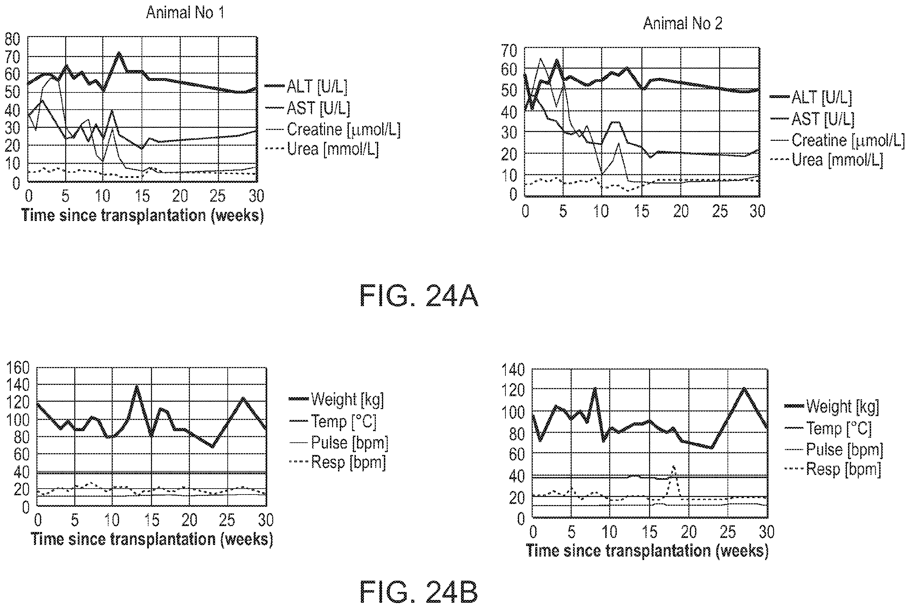

FIG. 24A-FIG. 24B are graphs illustrating post-transplantation biochemical (FIG. 24A) and clinical (FIG. 24B) parameters. Abbreviations: ALT = Alanine Aminotransferase, AST = Asparate Aminotransferase; Temp = Temperature, Resp = Respiration.

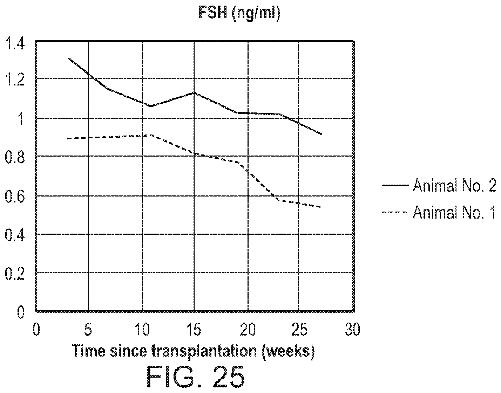

FIG. 25 is a graph showing FSH levels after transplantation which declined with time and was associated with increased E2.

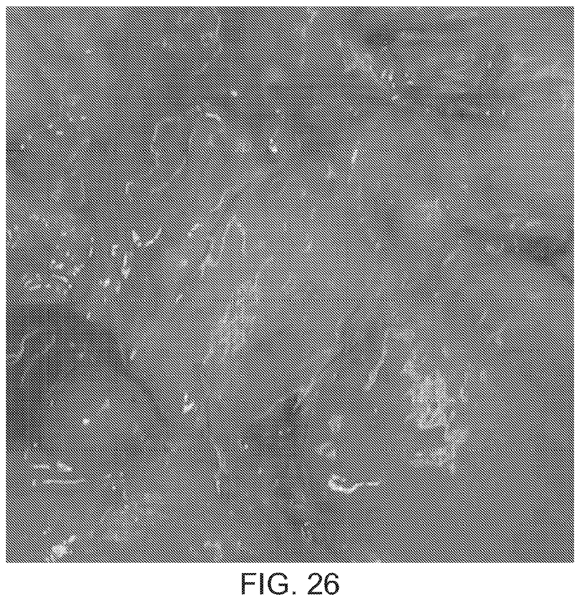

FIG. 26 is a picture showing the appearance of ovarian grafts in situ 324 days after transplantation. There was no evidence of local rejection or fibrosis.

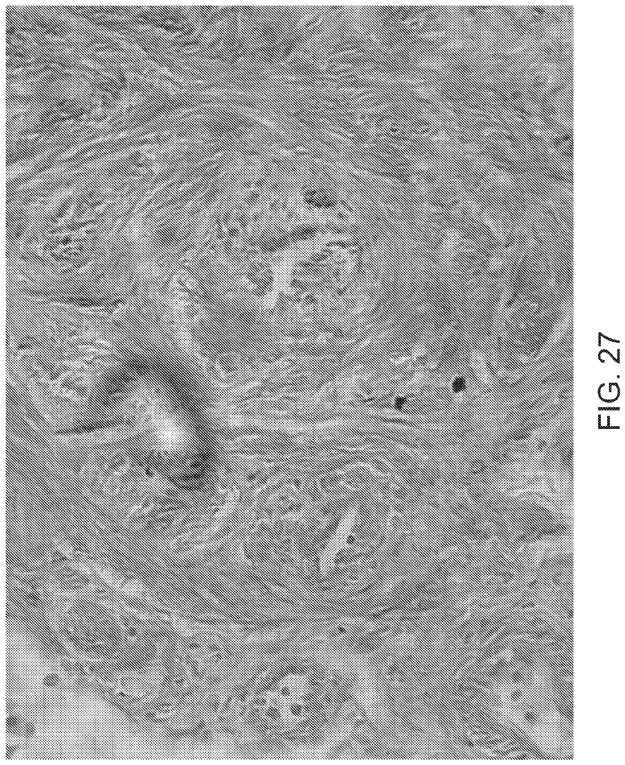

FIG. 27 is a picture showing follicles present in the ovary cortex. There was evidence for persistent follicular activity even after 9 nine months of study.

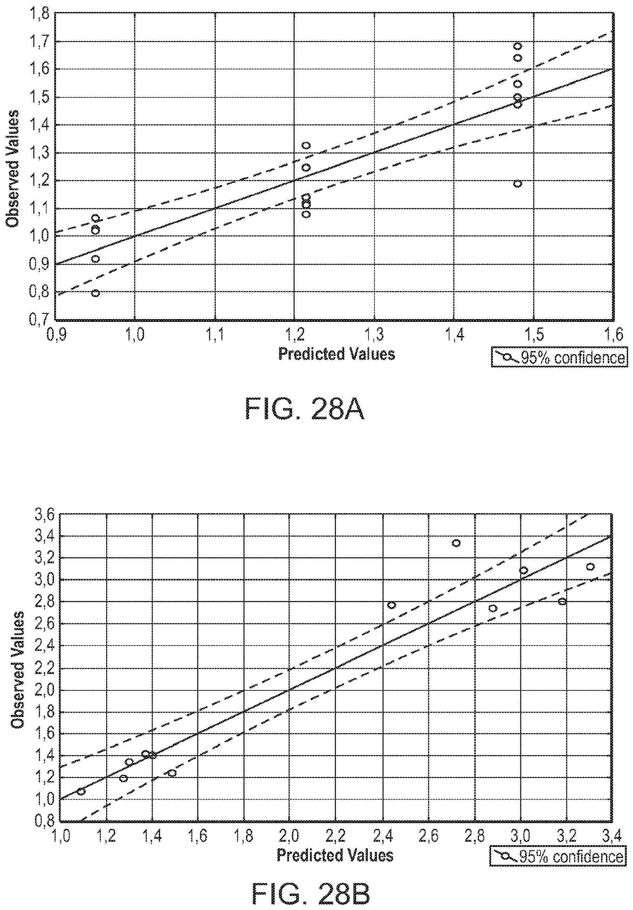

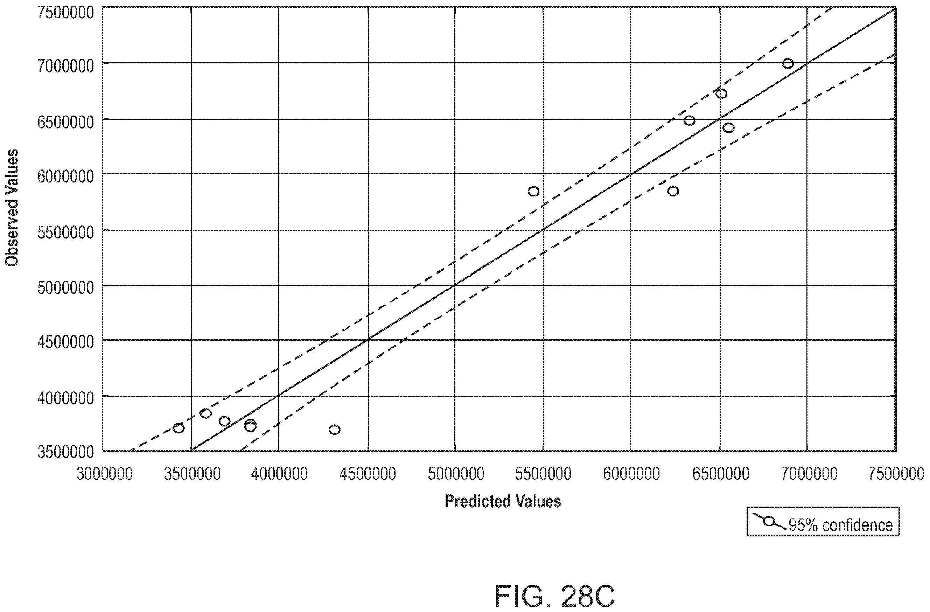

FIG. 28A-FIG. 28C are a series of graphs showing how the amount of viable cells strongly depend on the length of cultivation period (p<0.05). FIG. 28A shows the interdependence of cell viability from time of cultivation. FIG. 28B shows how cell viability strongly depends on apoptosis (p<0.05) and less on proliferation (p>0.1). FIG. 28C shows how the intensity of apoptosis in cell culture is significantly interconnected with cell proliferation activity (p<0.05).

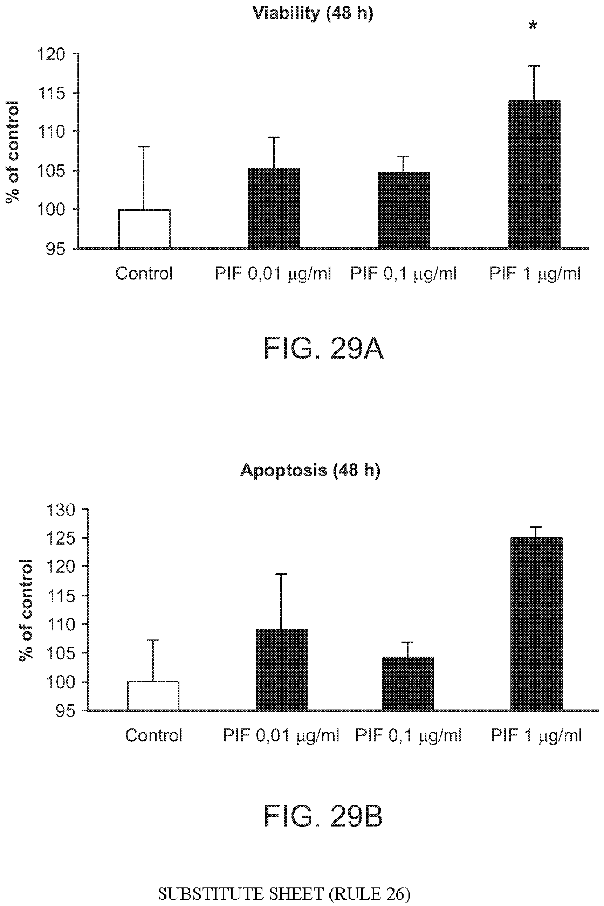

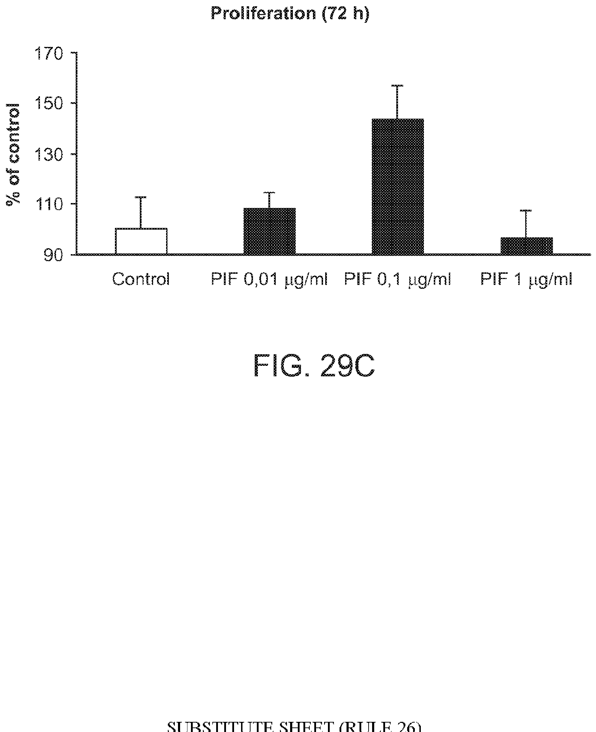

FIG. 29A is a graph showing the PIF effect on INS-1 cell viability. Data shows that at 48 hours of culture, INS-1 cell viability increased at 1 ug/ml. FIG. 29B is a graph showing the PIF effect on apoptosis. Data showed that at 48 hours of culture, INS-1 cell apoptosis increased at 1 ug/ml. FIG. 29C is a graph showing the PIF effect on proliferation. Data showed that at 72 hours of culture, INS-1 cell apoptosis at low 0.1 ug/ml increased.

### DETAILED DESCRIPTION OF EMBODIMENTS

Before the present compositions and methods are described, it is to be understood that this invention is not limited to the particular processes, compositions, or methodologies described, as these may vary. It is also to be understood that the terminology used in the description is for the purpose of describing the particular versions or embodiments only, and is not intended to limit the scope of the present invention which will be limited only by the appended claims. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. All publications mentioned herein are incorporated by reference in their entirety. Nothing herein is to be construed as an admission that the invention is not entitled to antedate such disclosure by virtue of prior invention.

It must also be noted that as used herein and in the appended claims, the singular forms "a", "an", and "the" include plural reference unless the context clearly dictates otherwise. Thus, for example, reference to a "peptide" is a reference to one or more peptides and equivalents thereof known to those skilled in the art, and so forth.

As used herein, the term "about" means plus or minus 10% of the numerical value of the number with which it is being used. Therefore, about 50% means in the range of 40%-60%.

"Administering" when used in conjunction with a therapeutic means to administer a therapeutic directly into or onto a target organ, tissue or cell or to administer a therapeutic to a patient, whereby the therapeutic positively impacts the organ, tissue or cell to which it is targeted. Thus, as used herein, the term "administering", when used in conjunction with pre-implantation factor (PIF), can include, but is not limited to, providing PIF into or onto the target organ, tissue or cell; providing PIF systemically to a patient by, e.g., intravenous injection whereby the therapeutic reaches the target organ, tissue or cell; providing PIF in the form of the encoding sequence thereof to the target tissue (e.g., by so-called gene-therapy techniques). "Administering" may be accomplished by parenteral, oral or topical administration, or by such methods in combination with other known techniques.

The terms "animal," "patient," and "subject" as used herein include, but are not limited to, humans and non-human vertebrates such as wild, domestic and farm animals. In some embodiments, the terms "animal," "patient," and "subject" may refer to humans. In some embodiments, the terms "animal," "patient," and "subject" may refer to non-human mammals. In some embodiments, the terms "animal," "patient," and "subject" may refer to any or combination of: dogs, cats, pigs, cows, horses, goats, sheep or other domesticated non-human mammals. In some embodiments, the subject is a human patient that has been diagnosed or is suspected of having a malignant form of cancer. In some embodiments, the subject is a human patient that has been diagnosed or is suspected of having organ failure. In some embodiments, the subject is a human patient that has been diagnosed or is suspected of having liver failure, kidney failure, any disease associated with an imbalance of cortisol levels, juvenile or adult diabetes (type I or II), or heart failure. In some embodiments, the subject is a human patient that has been identified as requiring or suspected of requiring an Islet cell transplant, a kidney transplant, or adrenal cell transplant, a blood cell transplant, a bone marrow transplant, or a heart transplant.

"Immunomodulating" refers to the ability of a compound of the present invention to alter (modulate) one or more aspects of the immune system. The immune system functions to protect the organism from infection and from foreign antigens by cellular and humoral mechanisms involving lymphocytes, macrophages, and other antigen-presenting cells that regulate each other by means of multiple cell-cell interactions and by elaborating soluble factors, including lymphokines and antibodies, that have autocrine, paracrine, and endocrine effects on immune cells.

The term "improves" is used to convey that the present invention changes either the appearance, form, characteristics and/or the physical attributes of the subject, organ, tissue or cell to which it is being provided, applied or administered. For example, the change in form may be demonstrated by any of the following alone or in combination: a decrease in one or more symptoms of a disease or disorder; increased engraftment of transplanted organs, tissues, or cells; increased acceptance of transplanted organs, tissues, or cells; reduction of host immune response to graft associated with autologous transplant, allogeneic transplant, semi-allogeneic transplant, or xenotransplant; increased graft v. leukemia; increase in graft v. leukemia with no or with minimal graft v. host disease; reduction or elimination of the need for immune suppressive agents; and faster recovery from chemotherapy and radiation therapy.

The term "inhibiting" includes the administration of a compound of the present invention to prevent the onset of the symptoms, alleviating the symptoms, or eliminating the disease, condition or disorder.

As used herein, the terms "peptide," "polypeptide" and "protein" are used interchangeably and refer to two or more amino acids covalently linked by an amide bond or non-amide equivalent. In some embodiments, peptides are also peptidomimetics, salts or functional fragemtns of peptides disclosed herein. The peptides of the invention can be of any length. For example, the peptides can have from about two to about 100 or more residues, such as, 5 to 12, 12 to 15, 15 to 18, 18 to 25, 25 to 50, 50 to 75, 75 to 100, or more in length. Preferably, peptides are from about 2 to about 18 residues. The peptides of the invention include L- and D-isomers, and combinations of L- and D-isomers. The peptides can include modifications typically associated with post-translational processing of proteins, for example, cyclization (e.g., disulfide or amide bond), phosphorylation, glycosylation, carboxylation, ubiquitination, myristylation, or lipidation. In some embodiments, the peptides of the disclosure comprise only D-isomers. In some embodiments, the peptides comprise only L-isomers.

By "pharmaceutically acceptable," it is meant the carrier, diluent or excipient must be compatible with the other ingredients of the formulation or composition and not deleterious to the recipient thereof.

As used herein, the term "therapeutic" means an agent utilized to treat, combat, ameliorate, prevent or improve an unwanted condition or disease of a patient. In part, embodiments of the present invention are directed to decreasing one or more symptoms of ARS, an increase in acceptance of transplanted organs, tissues, or cells in autologous transplants, allogeneic transplants, semi-allogeneic transplants, or xenotransplants, and/or a decrease in the rejection of organs, tissues, or cells in autologous transplants, allogeneic transplants, semi-allogeneic transplants, or xenotransplants.

A "therapeutically effective amount" or "effective amount" of a composition (e.g, a PIF peptide) is a predetermined amount calculated to achieve the desired effect, i.e., to improve, increase, or allow the acceptance of organs, tissues, or cells in autologous transplantation, allogeneic transplantation, semi-allogeneic transplantation, or xenotransplantation, and/or to decrease one or more symptoms of ARS, graft-versus host disease (GVHD) or increase the viability of donor organs, tissues, or cell before and after they are transplanted. The activity contemplated by the present methods includes both medical therapeutic and/or prophylactic treatment, as appropriate. The specific dose of a compound administered according to this invention to obtain therapeutic and/or prophylactic effects will, of course, be determined by the particular circumstances surrounding the case, including, for example, the compound administered, the route of administration, and the condition being treated. The compounds are effective over a wide dosage range and, for example, dosages per day will normally fall within the range of from about 0.001 to about 10 mg/kg, more usually in the range of from about 0.01 to about 1 mg/kg. In some embodiments, the therapeutically effective dose of PIF or PIF analog or peptide is about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, and about 1 mg/kg. However, it will be understood that the effective amount administered will be determined by the physician in the light of the relevant circumstances including the condition to be treated, the choice of compound to be administered, and the chosen route of administration, and therefore the above dosage ranges are not intended to limit the scope of the invention in any way. A therapeutically effective amount of compound of embodiments of this invention is typically an amount such that when it is administered in a physiologically tolerable excipient composition, it is sufficient to achieve an effective systemic concentration or local concentration in the tissue.

The terms "treat," "treated," or "treating" as used herein refers to both therapeutic treatment and/or prophylactic or preventative measures, wherein the object is to prevent or slow down (lessen) an undesired physiological condition, disorder or disease, or to obtain beneficial or desired clinical results. For the purposes of this invention, beneficial or desired clinical results include, but are not limited to, alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment.

Generally speaking, the term "tissue" refers to any aggregation of similarly specialized cells which are united in the performance of a particular function.

The disclosure relates to compositions and pharmaceutical compositions comprising polypeptides described herein, which, in some embodiments, act as agonists of PIF-mediated signal transduction via the receptor or receptors of PIF. These compositions or pharmaceutical compositions modulate signaling pathways that provide significant therapeutic benefit in the treatment of a recipient of transplanted tissue. The disclosure of the present disclosure may exist in unsolvated forms as well as solvated forms, including hydrated forms of the polypeptides disclosed herein. The compounds of the present disclosure also are capable of forming both pharmaceutically acceptable salts, including but not limited to acid addition and/or base addition salts. Furthermore, compounds of the present disclosure may exist in various solid states including an amorphous form (non-crystalline form), and in the form of clathrates, prodrugs, polymorphs, bio-hydrolyzable esters, racemic mixtures, non-racemic mixtures, or as purified stereoisomers including, but not limited to, optically pure enantiomers and diastereomers. In general, all of these forms can be used as an alternative form to the free base or free acid forms of the compounds, as described above and are intended to be encompassed within the scope of the present disclosure.

A "polymorph" refers to solid crystalline forms of a compound. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Different physical properties of polymorphs can affect their processing. In some embodiments, the pharmaceutical composition comprises at least one polymorph of any of the compositions disclosed herein.

As noted above, the compositions or pharmaceutical compositions of the present disclosure can be administered, inter alia, as pharmaceutically acceptable salts, esters, amides or prodrugs. The term "salts" refers to inorganic and organic salts of compounds of the present disclosure. The salts can be prepared in situ during the final isolation and purification of a compound, or by separately reacting a purified compound in its free base or acid form with a suitable organic or inorganic base or acid and isolating the salt thus formed. Representative salts include the hydrobromide, hydrochloride, sulfate, bisulfate, nitrate, acetate, oxalate, palmitiate, stearate, laurate, borate, benzoate, lactate, phosphate, tosylate, citrate, maleate, fumarate, succinate, tartrate, naphthylate, mesylate, glucoheptonate, lactobionate, and laurylsulphonate salts, and the like. The salts may include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. See, for example, S. M. Berge, et al., "Pharmaceutical Salts," J Pharm Sci, 66: 1-19 (1977). The term "salt" refers to acidic salts formed with inorganic and/or organic acids, as well as basic salts formed with inorganic and/or organic bases. Examples of these acids and bases are well known to those of ordinary skill in the art. Such acid addition salts will normally be pharmaceutically acceptable although salts of non-pharmaceutically acceptable acids may be of utility in the preparation and purification of the compound in question. Salts include those formed from hydrochloric, hydrobromic, sulphuric, phosphoric, citric, tartaric, lactic, pyruvic, acetic, succinic, fumaric, maleic, methanesulphonic and benzenesulphonic acids.

In some embodiments, salts of the compositions comprising either a PIF or PIF analog or PIF mutant may be formed by reacting the free base, or a salt, enantiomer or racemate thereof, with one or more equivalents of the appropriate acid. In some embodiments, pharmaceutical acceptable salts of the present disclosure refer to analogs having at least one basic group or at least one basic radical. In some embodiments, pharmaceutical acceptable salts of the present disclosure comprise a free amino group, a free guanidino group, a pyrazinyl radical, or a pyridyl radical that forms acid addition salts. In some embodiments, the pharmaceutical acceptable salts of the present disclosure refer to analogs that are acid addition salts of the subject compounds with (for example) inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxybenzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid. When several basic groups are present mono- or poly-acid addition salts may be formed. The reaction may be carried out in a solvent or medium in which the salt is insoluble or in a solvent in which the salt is soluble, for example, water, dioxane, ethanol, tetrahydrofuran or diethyl ether, or a mixture of solvents, which may be removed in vacuo or by freeze drying. The reaction may also be a metathetical process or it may be carried out on an ion exchange resin. In some embodiments, the salts may be those that are physiologically tolerated by a patient. Salts according to the present disclosure may be found in their anhydrous form or as in hydrated crystalline form (i.e., complexed or crystallized with one or more molecules of water).

Examples of pharmaceutically acceptable esters of the compounds of the present disclosure include C₁-C₈ alkyl esters. Acceptable esters also include C₅-C₇ cycloalkyl esters, as well as arylalkyl esters such as benzyl. C₁-C₄ alkyl esters are commonly used. Esters of compounds of the present disclosure may be prepared according to methods that are well known in the art. Examples of pharmaceutically acceptable amides of the compounds of the present disclosure include amides derived from ammonia, primary C₁-C₈ alkyl amines, and secondary C₁-C₈ dialkyl amines. In the case of secondary amines, the amine may also be in the form of a 5 or 6 membered heterocycloalkyl group containing at least one nitrogen atom. Amides derived from ammonia, C₁-C₃ primary alkyl amines and C₁-C₂ dialkyl secondary amines are commonly used. Amides of the compounds of the present disclosure may be prepared according to methods well known to those skilled in the art.

As used herein, "conservative" amino acid substitutions may be defined as set out in Tables A, B, or C below. The PIF peptides of the disclosure include those wherein conservative substitutions (from either nucleic acid or amino acid sequences) have been introduced by modification of polynucleotides encoding polypeptides of the disclosure. Amino acids can be classified according to physical properties and contribution to secondary and tertiary protein structure. A conservative substitution is recognized in the art as a substitution of one amino acid for another amino acid that has similar properties. In some embodiments, the conservative substitution is recognized in the art as a substitution of one nucleic acid for another nucleic acid that has similar properties, or, when encoded, has similar binding affinities.

The disclosure also relates to compositions and pharmaceutical compositions comprising one or a plurality of amino acid structural and functional analogs of a PIF peptide, for example, peptidomimetics having synthetic or non-natural amino acids (such as a norleucine) or amino acid analogues or non-natural side chains, so long as the mimetic shares one or more functions or activities of compounds of the disclosure. The compounds of the disclosure therefore include "mimetic" and "peptidomimetic" forms. As used herein, a "non-natural side chain" is a modified or synthetic chain of atoms joined by covalent bond to the α-carbon atom, β-carbon atom, or γ-carbon atom which does not make up the backbone of the polypeptide chain of amino acids. The peptide analogs may comprise one or a combination of non-natural amino-acids chosen from: norvaline, tert-butylglycine, phenylglycine, He, 7-azatryptophan, 4-fluorophenylalanine, N-methyl-methionine, N-methyl-valine, N-methyl-alanine, sarcosine, N-methyl-tert-butylglycine, N-methyl-leucine, N-methyl-phenylglycine, N-methyl-isoleucine, N-methyl-tryptophan, N-methyl-7-azatryptophan, N-methyl-phenylalanine, N-methyl-4-fluorophenylalanine, N-methyl-threonine, N-methyl-tyrosine, N-methyl-valine, N-methyl-lysine, homocysteine, and Tyr; Xaa2 is absent, or an amino acid selected from the group consisting of Ala, D-Ala, N-methyl-alanine, Glu, N-methyl-glutamate, D-Glu, Gly, sarcosine, norleucine, Lys, D-Lys, Asn, D-Asn, D-Glu, Arg, D-Arg, Phe, D-Phe, N-methyl-phenylalanine, Gin, D-Gln, Asp, D-Asp, Ser, D-Ser, N-methyl-serine, Thr, D-Thr, N-methyl-threonine, D-Pro D-Leu, N-methyl-leucine, D-Ile, N-methyl-isoleucine, D-Val, N-methyl-valine, tert-butylglycine, D-tert-butylglycine, N-methyl-tert-butylglycine, Trp, D-Trp, N-methyl-tryptophan, D-Tyr, N-methyl-tyrosine, 1-aminocyclopropanecarboxylic acid, 1-aminocyclobutanecarboxylic acid, 1-aminocyclopentanecarboxylic acid, 1-aminocyclohexanecarboxylic acid, 4-aminotetrahydro-2H-pyran-4-carboxylic acid, aminoisobutyric acid, (5)-2-amino-3-(IH-tetrazol-5-yl)propanoic acid, Glu, Gly, N-methyl-glutamate, 2-amino pentanoic acid, 2-amino hexanoic acid, 2-amino heptanoic acid, 2-amino octanoic acid, 2-amino nonanoic acid, 2-amino decanoic acid, 2-amino undecanoic acid, 2-amino dodecanoic acid, octylglycine, tranexamic acid, aminovaleric acid, and 2-(2-aminoethoxy)acetic acid. The natural side chain, or R group, of an alanine is a methyl group. In some embodiments, the non-natural side chain of the composition is a methyl group in which one or more of the hydrogen atoms is replaced by a deuterium atom. Non-natural side chains are disclosed in the art in the following publications: WO/2013/172954, WO2013123267, WO/2014/071241, WO/2014/138429, WO/2013/050615, WO/2013/050616, WO/2012/166559, US Application No. 20150094457, Ma, Z., and Hartman, M.C. (2012). In Vitro Selection of Unnatural Cyclic Peptide Libraries via mRNA Display. In J.A. Douthwaite & R.H. Jackson (Eds.), Ribosome Display and Related Technologies: Methods and Protocols (pp. 367-390). Springer New York., all of which are incorporated by reference in their entireties.

The terms "mimetic," "peptide mimetic" and "peptidomimetic" are used interchangeably herein, and generally refer to a peptide, partial peptide or non-peptide molecule that mimics the tertiary binding structure or activity of a selected native peptide or protein functional domain (e.g., binding motif or active site). These peptide mimetics include recombinantly or chemically modified peptides, as well as non-peptide agents such as small molecule drug mimetics, as further described below. The term "analog" refers to any polypeptide comprising at least one α-amino acid and at least one non-native amino acid residue, wherein the polypeptide is structurally similar to a naturally occurring full-length PIF protein and shares the biochemical or biological activity of the naturally occurring full-length protein upon which the analog is based. In some embodiments, the compositions, pharmaceutical compositions and kits comprise a peptide or peptidomimeic sharing share no less than about 70%, about 75%, about 79%, about 80%, about 85%, about 86%, about 87%, about 90%, about 93%, about 94% about 95%, about 96%, about 97%, about 98%, about 99% homology with any one or combination of PIF sequences; and wherein one or a plurality of amino acid residues is a non-natural amino acid residue or an amino acid residue with a non-natural sidechain. In some embodiments, peptide or peptide mimetics are provided, wherein a loop is formed between two cysteine residues. In some embodiments, the peptidomimetic may have many similarities to natural peptides, such as: amino acid side chains that are not found among the known 20 proteinogenic amino acids, non-peptide-based linkers used to effect cyclization between the ends or internal portions of the molecule, substitutions of the amide bond hydrogen moiety by methyl groups (N-methylation) or other alkyl groups, replacement of a peptide bond with a chemical group or bond that is resistant to chemical or enzymatic treatments, N- and C-terminal modifications, and conjugation with a non-peptidic extension (such as polyethylene glycol, lipids, carbohydrates, nucleosides, nucleotides, nucleoside bases, various small molecules, or phosphate or sulfate groups). As used herein, the term "cyclic peptide mimetic" or "cyclic polypeptide mimetic" refers to a peptide mimetic that has as part of its structure one or more cyclic features such as a loop, bridging moiety, and/or an internal linkage. As used herein, the term "bridging moiety" refers to a chemical moiety that chemically links one or a combination of atoms on an amino acid to any other atoms outside of the amino acid residue. For instance, in the case of amino acid tertiary structure, a bridging moiety may be a chemical moiety that chemically links one amino acid side chain with another sequential or non-sequential amino acid side chain.

Ultimately, a novel embryo-derived peptide, PIF, creates a tolerogenic state at low doses following short-term treatment leading to long-term protection from tissue rejection after transplantation. This effect is exerted without apparent toxicity and is exerted, in some embodiment, as a monotherapy without other active agents that may modulate the immune system. In some embodiments, the methods of treatment are performed without administration of steroids.

For therapeutic treatment of the specified indications, a PIF peptide may be administered as such, or can be compounded and formulated into pharmaceutical compositions in unit do sage form for parenteral, transdermal, rectal, nasal, local intravenous administration, or, preferably, oral administration. Such pharmaceutical compositions are prepared in a manner well known in the art and comprise at least one active PIF peptide associated with a pharmaceutically carrier. The term "active compound", as used throughout this specification, refers to at least one composition comprising one or a plurality of selected from compounds of the formulas or pharmaceutically acceptable salts thereof.

In such a composition, the active compound is known as the "active ingredient." In making the compositions, the active ingredient will usually be mixed with a carrier, or diluted by a carrier, or enclosed within a carrier that may be in the form of a capsule, sachet, paper or other container. When the carrier serves as a diluent, it may be a solid, semisolid, or liquid material that acts as a vehicle, excipient of medium for the active ingredient. Thus, the composition can be in the form of tablets, pills, powders, lozenges, sachets, cachets, elixirs, emulsion, solutions, syrups, suspensions, soft and hard gelatin capsules, sterile injectable solutions, and sterile packaged powders.

The terms "pharmaceutical preparation" and "pharmaceutical composition" include preparations suitable for administration to mammals, e.g., humans. When the compounds of the present disclosure are administered as pharmaceuticals to mammals, e.g., humans, they can be given per se or as a pharmaceutical composition containing, for example, from about 0.1 to about 99.5% of active ingredient in combination with a pharmaceutically acceptable carrier.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. In some embodiments, the pharmaceutical compositions comprising a PIF peptide, mimetic or pharmaceutically acceptable salt thereof and at least one pharmaceutically acceptable carrier.

The phrase "pharmaceutically acceptable carrier" is art recognized and includes a pharmaceutically acceptable material, composition or vehicle, suitable for administering compounds of the present disclosure to mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations. Suitable pharmaceutical carriers are described in "Remington's Pharmaceutical Sciences" by E. W. Martin, which is incorporated herein by reference in its entirety. In some embodiments, the pharmaceutically acceptable carrier is sterile and pyrogen-free water. In some embodiments, the pharmaceutically acceptable carrier is Ringer's Lactate, sometimes known as lactated Ringer's solution.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, .alpha.-tocopherol, and the like; and metal chelating agents, such as citric acid, ethylenediamine tetraacetic acid (EDTA), sorbitol, tartaric acid, phosphoric acid, and the like.

Formulations of the present disclosure include those suitable for oral, nasal, topical, buccal, sublingual, rectal, vaginal and/or parenteral administration. The formulations may conveniently be presented in unit dosage form and may be prepared by any methods well known in the art of pharmacy. The amount of active ingredient that can be combined with a carrier material to produce a single dosage form will generally be that amount of the compound that produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Some examples of suitable carriers, excipients, and diluents include lactose, dextrose, sucrose, sorbitol, mannitol, starches, gum acacia, calcium phosphate alginates, calcium salicate, microcrystalline cellulose, polyvinylpyrrolidone, cellulose, tragacanth, gelatin, syrup, methyl cellulose, methyl- and propylhydroxybenzoates, tale, magnesium stearate, water, and mineral oil. The formulations can additionally include lubricating agents, wetting agents, emulsifying and suspending agents, preserving agents, sweetening agents or flavoring agents. The compositions may be formulated so as to provide quick, sustained, or delayed release of the active ingredient after administration to the patient by employing procedures well known in the art.

For oral administration, a compound can be admixed with carriers and diluents, molded into tablets, or enclosed in gelatin capsules. The mixtures can alternatively be dissolved in liquids such as 10% aqueous glucose solution, isotonic saline, sterile water, or the like, and administered intravenously or by injection.

The local delivery of inhibitory amounts of active compound for the treatment of immune disorders can be by a variety of techniques that administer the compound at or near the targeted site. Examples of local delivery techniques are not intended to be limiting but to be illustrative of the techniques available. Examples include local delivery catheters, site specific carriers, implants, direct injection, or direct applications, such as topical application.

Local delivery by an implant describes the surgical placement of a matrix that contains the pharmaceutical agent into the affected site. The implanted matrix releases the pharmaceutical agent by diffusion, chemical reaction, or solvent activators.

For example, in some aspects, the disclosure is directed to a pharmaceutical composition comprising a PIF peptide, and a pharmaceutically acceptable carrier or diluent, or an effective amount of pharmaceutical composition comprising a PIF peptide.

Specific modes of administration will depend on the indication. The selection of the specific route of administration and the dose regimen is to be adjusted or titrated by the clinician according to methods known to the clinician in order to obtain the optimal clinical response. The amount of compound to be administered is that amount which is therapeutically effective. The dosage to be administered will depend on the characteristics of the subject being treated, e.g., the particular mammal or human treated, age, weight, health, types of concurrent treatment, if any, and frequency of treatments, and can be easily determined by one of skill in the art (e.g., by the clinician).

Pharmaceutical formulations containing the compounds of the present disclosure and a suitable carrier can be solid dosage forms which include, but are not limited to, tablets, capsules, cachets, pellets, pills, powders and granules; topical dosage forms which include, but are not limited to, solutions, powders, fluid emulsions, fluid suspensions, semi-solids, ointments, pastes, creams, gels, jellies, and foams; and parenteral dosage forms which include, but are not limited to, solutions, suspensions, emulsions, and dry powder; comprising an effective amount of a polymer or copolymer of the present disclosure. It is also known in the art that the active ingredients can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's The Pharmaceutical Basis of Therapeutics, 6th Edition, MacMillan Publishing Co., New York (1980) can be consulted.

The compositions or pharmaceutical compositions of the present disclosure can be formulated for parenteral administration by injection, e.g., by bolus injection or continuous infusion. The compounds can be administered by continuous infusion subcutaneously over a predetermined period of time. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

For oral administration, the compounds can be formulated readily by combining these compounds with pharmaceutically acceptable carriers well known in the art. Such carriers enable the compounds of the disclosure to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding the resulting mixture, and processing the mixture of granules, alter adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragecanth, methyl cellulose, hydroxypropylmethyl-celllose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations that can be used orally include, but are not limited to, push-fit capsules made of gelatin, as well as soft, scaled capsules made of gelatin and a plasticizer, such as glycerol or sorbitol. The push-fit capsules can contain the active ingredients in admixture with filler such as, e.g., lactose, binders such as, e.g., starches, and/or lubricants such as, e.g., talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the compositions can take the form of, e.g., tablets or lozenges formulated in a conventional manner.

For administration by inhalation, the compounds for use according to the present disclosure are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The compounds of the present disclosure can also be formulated in rectal compositions such as suppositories or retention enemas, e.g., containing conventional suppository bases such as cocoa butter or other glycerides.

In addition to the formulations described previously, the compounds of the present disclosure can also be formulated as a depot preparation. Such long acting formulations can be administered by implantation (for example subcutaneously or intramuscularly) or by intramuscular injection.

Depot injections can be administered at about 1 to about 6 months or longer intervals. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

In transdermal administration, the compounds of the present disclosure, for example, can be applied to a plaster, or can be applied by transdermal, therapeutic systems that are consequently supplied to the organism.

Pharmaceutical compositions comprising any one or plurality of compounds disclosed herein also can comprise suitable solid or gel phase carriers or excipients. Examples of such carriers or excipients include but are not limited to calcium carbonate, calcium phosphate, various sugars, starches, cellulose derivatives, gelatin, and polymers such as, e.g., polyethylene glycols.

For parenteral administration, a composition or pharmaceutical composition can be, for example, formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques. For example, a parenteral composition suitable for administration by injection is prepared by dissolving 1.5% by weight of analog in 0.9% sodium chloride solution.

The present disclosure relates to routes of administration include intramuscular, sublingual, intravenous, intraperitoneal, intrathecal, intravaginal, intraurethral, intradermal, intrabuccal, via inhalation, via nebulizer and via subcutaneous injection. Alternatively, the pharmaceutical composition may be introduced by various means into cells that are removed from the individual. Such means include, for example, microprojectile bombardment and liposome or other nanoparticle device.

Solid dosage forms for oral administration include capsules, tablets, pills, powders and granules. In solid dosage forms, the composition or pharmaceutical compositions are generally admixed with at least one inert pharmaceutically acceptable carrier such as sucrose, lactose, starch, or other generally regarded as safe (GRAS) additives. Such dosage forms can also comprise, as is normal practice, an additional substance other than an inert diluent, e.g., lubricating agent such as magnesium state. With capsules, tablets, and pills, the dosage forms may also comprise a buffering agent. Tablets and pills can additionally be prepared with enteric coatings, or in a controlled release form, using techniques know in the art.

Liquid dosage forms for oral administration include pharmaceutically acceptable emulsions, solutions, suspensions and syrups, with the elixirs containing an inert diluent commonly used in the art, such as water. These compositions can also include one or more adjuvants, such as wetting agent, an emulsifying agent, a suspending agent, a sweetening agent, a flavoring agent or a perfuming agent.

In another embodiment of the invention the composition of the invention is used to treat a patient before, contemporaneous with, or after receiving tissue from a donor. In some embodiments, the tissue comprises cells from any type of tissue capable of having endocrine function. In some embodiments, the tissue is a whole organ capable of endocrine function. In some embodiments, the tissue is a graft of cells or tissue or part of an organ, such partial organ or such cells being sufficient to restore or partially restore systemic endocrine function in a subject. In some embodiments, the therapy is a monotherapy. In some embodiments, the therapy also simultaneously treats a subject susceptible to or diagnosed with or having Type I adult or juvenile diabetes, graft versus host disease from a previous transplant procedure, multiple sclerosis, Crohn's, or autoimmune hepatitis.

In some embodiments, the disclosure relates to a method of treating congenital adrenal hyperplasia (CAH) comprising administering to a subject in need thereof a therapeutically effective amount of a PIF peptide, compositions thereof, mimetics thereof, pharmaceutically acceptable salts thereof, or combinations thereof of any of the aforementioned.

In some aspects, the disclosure relates to a method of preventing an acute adrenal crisis in a subject in need thereof having adrenal tissue dysfunction comprising administering to the subject a therapeutically effective amount of a PIF peptide, compositions thereof, mimetics thereof, pharmaceutically acceptable salts thereof, or combinations thereof of any of the aforementioned.

One of skill in the art will recognize that the appropriate dosage of the compositions and pharmaceutical compositions may vary depending on the individual being treated and the purpose. For example, the age, body weight, and medical history of the individual patient may affect the therapeutic efficacy of the therapy. Further, a lower dosage of the composition may be needed to produce a transient cessation of symptoms, while a larger dose may be needed to produce a complete cessation of symptoms associated with the disease, disorder, or indication. A competent physician can consider these factors and adjust the dosing regimen to ensure the dose is achieving the desired therapeutic outcome without undue experimentation. It is also noted that the clinician and/or treating physician will know how and when to interrupt, adjust, and/or terminate therapy in conjunction with individual patient response. Dosages may also depend on the strength of the particular composition, pharmaceutical composition, salt or analog chosen for the pharmaceutical composition.

The dose of the composition or pharmaceutical compositions may vary. The dose of the composition may be once per day. In some embodiments, multiple doses may be administered to the subject per day. In some embodiments, the total dosage is administered in at least two application periods. In some embodiments, the period can be an hour, a day, a month, a year, a week, or a two-week period. In an additional embodiment of the invention, the total dosage is administered in two or more separate application periods, or separate doses over the course of an hour, a day, a month, a year, a week, or a two-week period.

Dosage may be measured in terms of mass amount of polypeptide, salt, or analog per liter of liquid formulation prepared. One skilled in the art can increase or decrease the concentration of the polypeptide, salt, or analog in the dose depending upon the strength of biological activity desired to treat or prevent any above-mentioned disorders associated with the treatment of subjects in need thereof. For instance, some embodiments of the invention can include up to 0.00001 grams of polypeptide, salt, or analog per 5 mL of liquid formulation and up to about 10 grams of polypeptide, salt, or analog per 5 mL of liquid formulation.

The disclosure relates generally to the a composition comprising a therapeutically effective amount of one or a plurality of: a PIF peptide, a pharmaceutically acceptable salt thereof, a peptidomimetic thereof or a functional fragment thereof or combinations of any of the foregoing. If in combination and in some embodiments, the composition comprises a first, second, third, fourth or more different PIF peptides or salts, functional fragments or peptidomimetics thereof. In some embodiments, the compositions of the disclosure relate to a pharmaceutical composition comprising a therapeutically effective amount of a PIF peptide PIF peptides or salts, functional fragments or peptidomimetics thereof; and a pharmaceutically acceptable carrier, such as an excipient. A PIF peptide may be defined as one of the following: PIF or analogs of any PIF sequence set forth in Table Z that share no less than about 70%, about 75%, about 79%, about 80%, about 85%, about 86%, about 87%, about 90%, about 93%, about 94% about 95%, about 96%, about 97%, about 98%), about 99% homology with any one or combination of PIF sequences set forth in Table Z. In some embodiments, PIF may refer to an amino acid sequence selected from SEQ ID NOs: 1, 2, 3, 4, 5, 6, 7, 8, 9, or 10, or a functional fragment thereof that is about 70%, 75%, 80%, 85%, 90%, 91%, 92%, 93%, 94%, 95%, 96%, 97%, 98%, or 99% homologous to any such amino acid sequence. In some embodiments, PIF may refer to an amino acid sequence comprising, consisting essentially of, or consisting of a sequence that is at least about 70%, 75%, 80%, 85%, 86%, 87%, 90%, 91%, 92%, 93%, 94%, 95%, 96%, 97%, 98%, or 99% homologous to SEQ ID. NO: 1. In some embodiments, the PIF mutant comprises a sequence selected from: MVXIKPGSANKPSDD, MVXIKPGSANKPSD, MVXIKPGSANKPS, MVXIKPGSANKP, MVXIKPGSANK, MVXIKPGSAN, MVXIKPGSA, MVXIKPGS, MVXIKPG, MVXIK, MVXI, or MVX wherein X is a non-natural amino acid or a naturally occurring amino acid. In some embodiments, the PIF peptide is an amino acid sequence comprising MVRIKPGSANKPSDD, or a functional fragment thereof that is at least about 70%, 75%, 80%, 85%, 90%, 91%, 92%, 93%, 94%, 95%, 96%, 97%, 98%, or 99% homologous to any such amino acid sequence. In some embodiments, the PIF peptide is an amino acid sequence comprising MVRIK, or a functional fragment thereof that is at least about 70%, 75%, 80%, 85%, 90%, 91%, 92%, 93%, 94%, 95%, 96%, 97%, 98%, or 99% homologous to any such amino acid sequence. In some embodiments, the PIF peptide is an amino acid sequence comprising MVXIK, or a functional fragment thereof that is at least about 70%, 75%, 80%, 85%, 90%, 91%, 92%, 93%, 94%, 95%, 96%, 97%, 98%, or 99% homologous to any such amino acid sequence, wherein X is a non-natural amino acid or a naturally occurring amino acid.
In some embodiments, the PIF peptide is an amino acid sequence comprises PGSANKPSDD, or a functional fragment thereof that is at least about 70%, 75%, 80%, 85%, 90%, 91%, 92%, 93%, 94%, 95%, 96%, 97%, 98%, or 99% homologous to any such amino acid sequence.

Peptides disclosed herein further include compounds having amino acid structural and functional analogs, for example, peptidomimetics having synthetic or non-natural amino acids (such as a norleucine) or amino acid analogues or non-natural side chains, so long as the mimetic shares one or more functions or activities of compounds of the disclosure. The compounds of the disclosure therefore include "mimetic" and "peptidomimetic" forms. As used herein, a "non-natural side chain" is a modified or synthetic chain of atoms joined by covalent bond to the a-carbon atom, β-carbon atom, or γ-carbon atom which does not make up the backbone of the polypeptide chain of amino acids. The peptide analogs may comprise one or a combination of non-natural amino-acids chosen from: norvaline, tert-butyl glycine, phenylglycine, He, 7-azatryptophan, 4-fluorophenylalanine, N-methyl-methionine, N-methyl-valine, N-methyl-alanine, sarcosine, N-methyl -tert-butyl glycine, N-methyl-leucine, N-methyl-phenylglycine, N-methyl-isoleucine, N-methyl -tryptophan, N-methyl-7-azatryptophan, N-methyl-phenylalanine, N-methyl-4-fluorophenylalanine, N-methyl-threonine, N-methyl-tyrosine, N-methyl -valine, N-methyl-lysine, homocysteine, and Tyr; Xaa2 is absent, or an amino acid selected from the group consisting of Ala, D-Ala, N-methyl-alanine, Glu, N-methyl-glutamate, D-Glu, Gly, sarcosine, norleucine, Lys, D-Lys, Asn, D-Asn, D-Glu, Arg, D-Arg, Phe, D-Phe, N-methyl-phenylalanine, Gin, D-Gln, Asp, D-Asp, Ser, D-Ser, N-methyl-serine, Thr, D-Thr, N-methyl-threonine, D-Pro D-Leu, N-methyl-leucine, D-Ile, N-methyl-isoleucine, D-Val, N-methyl-valine, tert-butylglycine, D-tert-butylglycine, N-methyl-tert-butyl glycine, T , D-Tϕ, N-m ethyl -tryptophan, D-Tyr, N-m ethyl -tyrosine, 1-aminocyclopropanecarboxylic acid, 1-aminocyclobutanecarboxylic acid, 1-aminocyclopentanecarboxylic acid, 1-aminocyclohexanecarboxylic acid, 4-aminotetrahydro-2H-pyran-4-carboxylic acid, aminoisobutyric acid, (5)-2-amino-3-(IH-te trazol-5-yl)propanoic acid, Glu, Gly, N-methyl-glutamate, 2-amino pentanoic acid, 2-amino hexanoic acid, 2-amino heptanoic acid, 2-amino octanoic acid, 2-amino nonanoic acid, 2-amino decanoic acid, 2-amino undecanoic acid, 2-amino dodecanoic acid, octylglycine, tranexamic acid, aminovaleric acid, and 2-(2-aminoethoxy)acetic acid. The natural side chain, or R group, of an alanine is a methyl group. In some embodiments, the non-natural side chain of the composition is a methyl group in which one or more of the hydrogen atoms is replaced by a deuterium atom. Non-natural side chains are disclosed in the art in the following publications: WO/2013/172954, WO2013123267, WO/2014/071241, WO/2014/138429, WO/2013/050615, WO/2013/050616, WO/2012/166559, US Application No. 20150094457, Ma, Z., and Hartman, M.C. (2012). In Vitro Selection of Unnatural Cyclic Peptide Libraries via mRNA Display. In J. A. Douthwaite & R.H. Jackson (Eds.), Ribosome Display and Related Technologies: Methods and Protocols (pp. 367-390). Springer New York., all of which are incorporated by reference in their entireties. In some embodiments, the PIF peptides of the disclosure are modified to produce peptide mimetics by replacement of one or more naturally occurring side chains of the 20 genetically encoded amino acids (or D amino acids) with other side chains, for instance with groups such as alkyl, lower alkyl, cyclic 4-, 5-, 6-, to 7 membered alkyl, amide, amide lower alkyl, amide di (lower alkyl), lower alkoxy, hydroxy, carboxy and the lower ester derivatives thereof, and with 4-, 5-, 6-, to 7 membered heterocyclics. For example, proline analogs can be made in which the ring size of the proline residue is changed from 5 members to 4, 6, or 7 members. Cyclic groups can be saturated or unsaturated, and if unsaturated, can be aromatic or nonaromatic. Heterocyclic groups can contain one or more nitrogen, oxygen, and/or sulphur heteroatoms. Examples of such groups include the furazanyl,furyl, imidazolidinyl, imidazolyl, imidazolinyl, isothiazolyl, isoxazolyl, morpholinyl (e.g. morpholino), oxazolyl, piperazinyl (e.g. 1-piperazinyl), piperidyl (e.g. 1-piperidyl, piperidino ), pyranyl, pyrazinyl, pyrazolidinyl, pyrazolinyl, pyrazolyl, pyridazinyl, pyridyl, pyrimidinyl, pyrrolidinyl (e.g. 1- pyrrolidinyl), pyrrolinyl, pyrrolyl, thiadiazolyl, thiazolyl, thienyl, thiomo holinyl (e.g. thiomorpholino ), and triazolyl. These heterocyclic groups can be substituted or unsubstituted. Where a group is substituted, the substituent can be alkyl, alkoxy, halogen, oxygen, or substituted or unsubstituted phenyl. Peptidomimetics may also have amino acid residues that have been chemically modified by phosphorylation, sulfonation, biotinylation, or the addition or removal of other moieties.

In a further embodiment a compound of the formula R1-R2-R3-R4-R5-R6-R7-R8-R9-R10-R11-R12-R13-R14-R15, wherein R1 is Met or a mimetic of Met, R₂ is Val or a mimetic of Val, R₃ is Arg or a mimetic of Arg, or any amino acid, R₄ is Ile or a mimetic of Ile, R₅ is Lys or a mimetic of Lys, R₆ is Pro or a mimetic of Pro, R₇ is Gly or a mimetic of Gly, R₈ is Ser or a mimetic of Ser, R₉ is Ala or a mimetic of Ala, R₁₀ is Asn or a mimetic of Asn, Kₙ is Lys or a mimetic of Lys, R₁₂ is Pro or a mimetic of Pro, R₁₃ is Ser or a mimetic of Ser, R14 is Asp or a mimetic of Asp and R₁₅ is Asp or a mimetic of Asp is provided. In a further embodiment, a compound comprising the formula R₁-R₂-R₃-R₄-R₅-Rₑ-R₇-R₈-R₉-R₁₀-R₁₁-R₁₂-R₁₃-R₁₄-R₁₅, wherein R₁ is a mimetic of the naturally occurring residue at position 1 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₂ is a mimetic of the naturally occurring residue at position 2 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₃ is a mimetic of the naturally occurring residue at position 3 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₄ is a mimetic of the naturally occurring residue at position 4 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₅ is a mimetic of the naturally occurring residue at position 5 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₆ is a mimetic of the naturally occurring residue at position 6 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₇ is a mimetic of the naturally occurring residue at position 7 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₈ is a mimetic of the naturally occurring residue at position 5 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 1 or the residue at that position of such sequences; wherein R₉ is a mimetic of the naturally occurring residue at position 9 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₁₀ is a mimetic of the naturally occurring residue at position 10 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein R₁₁ is a mimetic of the naturally occurring residue at position 11 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein Rᵢ₂ is a mimetic of the naturally occurring residue at position 12 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein Rᵢ₃ is a mimetic of the naturally occurring residue at position 13 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein Ri₄ is a mimetic of the naturally occurring residue at position 14 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences; wherein Ri5 is a mimetic of the naturally occurring residue at position 15 of SEQ ID NO: 1; SEQIDNO:2; SEQIDNO:3; SEQIDNO:4; SEQIDNO:5; SEQIDNO:6; SEQIDNO:7; SEQ ID NO: 8; SEQ ID NO: 9; or SEQ ID NO: 10 or the residue at that position of such sequences.

In some embodiments, the pharmaceutical composition comprising the formula R₁-R₂-R₃-R₄-R₅-R₆-R₇-R₈- R₉-R₁₀-R₁₁-R₁₂-R₁₃-R₁₄-R₁₅-R₁₆-R₁₇-R₁₈, wherein R₁ is Ser or a mimetic of Ser, R₂ is Gly or a mimetic of Gly, R₃ is Ile or a mimetic of Ile, R₄ is Val or a mimetic of Val, R₅ is Ile or a mimetic of Ile, R₆ is Tyr or a mimetic of Tyr, R₇ is Gin or a mimetic of Gin, R₈ is Tyr or a mimetic of Tyr, R₉ is Met or a mimetic of Met, R₁₀ is Asp or a mimetic of Asp, R11 is Asp or a mimetic of Asp, R₁₂ is Arg or a mimetic of Arg, R13 is Tyr or a mimetic of Tyr, R₁₄ is Val or a mimetic of Val, R15 is Gly or a mimetic of Gly, R16 is Ser or a mimetic of Ser, R₁₇ is Asp or a mimetic of Asp and R18 is Leu or a mimetic of Leu; and a compound comprising the formula R1-R2-R3-R4-R5-R6-R7-R8-R9, wherein Ri is Val or a mimetic of Val, R₂ is Ile or a mimetic of Ile, R₃ is Ile or a mimetic of Ile, R₄ is He or a mimetic of He, R5 is Ala or a mimetic of Ala, R₆ is Gin or a mimetic of Gin, R₇ is Tyr or a mimetic of Tyr, R₈ is Met or a mimetic of Met, and R₉ is Asp or a mimetic of Asp is provided. In some embodiments, R₃ is not Arg or a mimetic of Arg.

A variety of techniques are available for constructing peptide mimetics with the same or similar desired biological activity as the corresponding native but with more favorable activity than the peptide with respect to solubility, stability, and/or susceptibility to hydrolysis or proteolysis (see, e.g., Morgan & Gainor, Ann. Rep. Med. Chem. 24,243-252,1989).

Certain peptidomimetic compounds are based upon the amino acid sequence of the peptides of the disclosure. Often, peptidomimetic compounds are synthetic compounds having a three dimensional structure (i.e. a "peptide motif) based upon the three-dimensional structure of a selected peptide. The peptide motif provides the peptidomimetic compound with the desired biological activity, i.e., binding to PIF receptors, wherein the binding activity of the mimetic compound is not substantially reduced, and is often the same as or greater than the activity of the native peptide on which the mimetic is modeled. Peptidomimetic compounds can have additional characteristics that enhance their therapeutic application, such as increased cell permeability, greater affinity and/or avidity and prolonged biological half-life.

Peptidomimetic design strategies are readily available in the art (see, e.g., Ripka & Rich, Curr. Op. Chern. Bioi. 2,441-452, 1998; Hruby et al., Curr. Op.Chem. Bioi. 1,114-119, 1997; Hruby & Baise, Curr.Med. Chern. 9,945-970,2000). One class of peptidomimetics a backbone that is partially or completely non-peptide, but mimics the peptide backbone atom-for atom and comprises side groups that likewise mimic the functionality of the side groups of the native amino acid residues. Several types of chemical bonds, e.g., ester, thioester, thioamide, retroamide, reduced carbonyl, dimethyl ene and ketomethylene bonds, are known in the art to be generally useful substitutes for peptide bonds in the construction of protease-resistant peptidomimetics. Another class of peptidomimetics comprises a small non-peptide molecule that binds to another peptide or protein, but which is not necessarily a structural mimetic of the native peptide. Yet another class of peptidomimetics has arisen from combinatorial chemistry and the generation of massive chemical libraries. These generally comprise novel templates which, though structurally unrelated to the native peptide, possess necessary functional groups positioned on a nonpeptide scaffold to serve as "topographical" mimetics of the original peptide (Ripka & Rich, 1998, supra). In some embodiments, the pharmaceutical composition comprising the formula R₁-R₂-R₃-R₄-R₅-R₆-R₇-R₈- R₉-R₁₀-R₁₁-R₁₂-R₁₃-R₁₄-R₁₅-R₁ₑ-R₁₇-R₁₈, wherein R₁ is Ser or a mimetic of Ser or salt thereof, R₂ is Gly or a mimetic of Gly or salt thereof, R₃ is Ile or a mimetic of Ile or salt thereof, R₄ is Val or a mimetic of Val or salt thereof, R₅ is Ile or a mimetic of Ile or salt thereof, R₆ is Tyr or a mimetic of Tyr or salt thereof, R₇ is Gin or a mimetic of Gin or salt thereof, R₈ is Tyr or a mimetic of Tyr or salt thereof, R₉ is Met or a mimetic of Met or salt thereof, R₁₀ is Asp or a mimetic of Asp or salt thereof, R₁₁ is Asp or a mimetic of Asp or salt thereof, R₁₂ is Arg or a mimetic of Arg or salt thereof, R₁₃ is Tyr or a mimetic of Tyr or salt thereof, R₁₄ is Val or a mimetic of Val or salt thereof, R₁₅ is Gly or a mimetic of Gly or salt thereof, R₁₆ is Ser or a mimetic of Ser or salt thereof, R₁₇ is Asp or a mimetic of Asp or salt thereof; and R₁₈ is Leu or a mimetic of Leu or salt thereof; and a compound comprising the formula R₁-R₂-R₃-R₄-R₅-R₆-R₇-R₈- R₉,wherein R₁ is Val or a mimetic of Val or salt thereof, R₂ is Ile or a mimetic of Ile or salt thereof, R₃ is Ile or a mimetic of Ile or salt thereof, R₄ is Ile or a mimetic of Ile or salt thereof, R₅ is Ala or a mimetic of Ala or salt thereof, R₆ is Gin or a mimetic of Gin or salt thereof, R₇ is Tyr or a mimetic of Tyr or salt thereof, R₈ is Met or a mimetic of Met or salt thereof, and Rg is Asp or a mimetic of Asp or salt thereof, is provided. In some embodiments, R₃ is not Arg or a mimetic of Arg or a salt thereof.

A variety of techniques are available for constructing peptide mimetics with the same or similar desired biological activity as the corresponding native but with more favorable activity than the peptide with respect to solubility, stability, and/or susceptibility to hydrolysis or proteolysis (see, e.g., Morgan & Gainor, Ann. Rep. Med. Chern. 24,243-252,1989). Certain peptidomimetic compounds are based upon the amino acid sequence of the peptides of the disclosure. Often, peptidomimetic compounds are synthetic compounds having a three dimensional structure (i.e. a "peptide motif') based upon the three-dimensional structure of a selected peptide. The peptide motif provides the peptidomimetic compound with the desired biological activity, i.e., binding to PIF receptors, wherein the binding activity of the mimetic compound is not substantially reduced, and is often the same as or greater than the activity of the native peptide on which the mimetic is modeled. Peptidomimetic compounds can have additional characteristics that enhance their therapeutic application, such as increased cell permeability, greater affinity and/or avidity and prolonged biological half-life.

Peptidomimetic design strategies are readily available in the art (see, e.g., Ripka & Rich, Curr. Op. Chern. Bioi. 2,441-452,1998; Hruby et al., Curr. Op.Chem. Bioi. 1,114-119,1997; Hruby & Baise, Curr.Med. Chern. 9,945-970,2000). One class of peptidomimetics a backbone that is partially or completely non-peptide, but mimics the peptide backbone atom-for atom and comprises side groups that likewise mimic the functionality of the side groups of the native amino acid residues. Several types of chemical bonds, e.g., ester, thioester, thioamide, retroamide, reduced carbonyl, dimethylene and ketomethylene bonds, are known in the art to be generally useful substitutes for peptide bonds in the construction of protease-resistant peptidomimetics. Another class of peptidomimetics comprises a small non-peptide molecule that binds to another peptide or protein, but which is not necessarily a structural mimetic of the native peptide. Yet another class of peptidomimetics has arisen from combinatorial chemistry and the generation of massive chemical libraries. These generally comprise novel templates which, though structurally unrelated to the native peptide, possess necessary functional groups positioned on a nonpeptide scaffold to serve as "topographical" mimetics of the original peptide (Ripka & Rich, 1998, *supra).*

A list of PIF amino acid sequences are provided below in Table Z. Antibodies to various PIF peptides and scrambled PIF peptides are also provided.

**Table Z. PIF Peptides**

| (SEQ ID NO) | Peptide | Amino Acid Sequence |
|---|---|---|
| SEQ ID NO:1 | nPIF-1₁₅ | MVRIKPGSANKPSDD |
| isolated native, matches region of Circumsporozoite protein (Malaria) | | |
| SEQ ID NO:2 | nPIF -1₍₁₅₋ₐₗₜₑᵣ₎ | MVRIKYGSYNNKPSD |
| isolated native, matches region of Circumsporozoite protein (Malaria) | | |
| SEQ ID NO:3 | nPIF-1₍₁₃₎ | MVRIKPGSANKPS |
| isolated native, matches region of Circumsporozoite protein (Malaria) | | |
| SEQ ID NO:4 | nPIF-1₍₉₎ | MVRIKPGSA |
| isolated native, matches region of Circumsporozoite protein (Malaria) | | |
| SEQ ID NO:5 | scrPIF-1₁₅ | GRVDPSNKSMPKDIA |
| synthetic, scrambled amino acid sequence from region of Circumsporozoite protein Malaria | | |
| SEQ ID NO:6 | nPIF-2₍₁₀₎ | SQAVQEHAST |
| isolated native, matches region of human retinoid and thyroid hormone receptor-SMRT | | |
| SEQ ID NO:7 | nPIF-2₍₁₃₎ | SQAVQEHASTNMG |
| isolated native, matches region of human retinoid and thyroid hormone receptor (SMRT) | | |
| SEQ ID NO:8 | scrPIF-2₍₁₃₎ | EVAQHSQASTMNG |
| synthetic, scrambled amino acid sequence from region of human retinoid and thyroid hormone receptor SMRT | | |
| SEQ ID NO:9 | scrPIF-2₍₁₄₎ | GQASSAQMNSTGVH |
| SEQ ID NO:10 | nPIF-3₍₁₈₎ | SGIVIYQYMDDRYVGSDL |
| isolated native, matches region of Rev Trans | | |
| SEQ ID NO:11 | Neg control for negPIF-1₍₁₅₎ | GMRELQRSANK |
| synthetic, scrambled amino acid sequence from region of Circumsporozoite protein Malaria | | |
| SEQ ID NO:12 | nPIF-4₍₉₎ | VIIIAQYMD |
| isolated native, matches region of Rev Trans | | |
| antibody of native isolated nPIF-1₁₅ | AbPIF-1₍₁₅₎ | |
| (SEQ ID NO:13) | sPIF-1₍₁₅₎ | MVRIKPGSANKPSDD |
| synthetic, amino acid sequence from region of Circumsporozoite protein Malaria | | |
| (SEQ ID NO:14) | sPIF-2₍₁₃₎ | SQAVQEHASTNMG |
| synthetic, amino acid sequence from of human retinoid and thyroid hormone receptor SMRT | | |
| (SEQ ID NO:15) | sPIF-3₍₁₈₎ | SGIVIYQYMDDRYVGSDL |
| synthetic, amino acid sequence from region of Circumsporozoite protein Malaria | | |
| (SEQ ID NO: 16) | sPIF-1₍₉₎ | MVRIKPGSA |
| synthetic, amino acid sequence from region of Circumsporozoite protein Malaria | | |
| antibody of native isolated nPIF-2₍₁₃₎ | AbPIF-2₍₁₃₎ | |
| antibody of native isolated nPIF -3₍₁₈₎ | AbPIF-3₍₁₈₎ | |
| (SEQ ID NO: 17) | sPIF-4₍₉₎ | VIIIAQYMD |
| Synthetic | | |
| SEQ ID NO: 18 | sPIF-1₍₅₎ | MVRIK |
| Synthetic | | |
| SEQ ID NO: 19 | sPIF-1₍₄₎ | PGSA |
| Synthetic | | |
| SEQ ID NO: 20 | PIF (-3) | MVXIKPGSANKPSDD |
| SEQ ID NO: 21 | PIF (-1) | XVRIKPGSANKPSDD |
| SEQ ID NO: 22 | PIF (-1, -3) | XVXIKPGSANKPSDD |
| SEQ ID NO: 23 | PIF (-6) | MVRIKXGSANKPSDD |
| SEQ ID NO: 24 | PIF (-4) | MVRXKPGSANKPSDD |
| SEQ ID NO: 25 | PIF (-2) | MXRIKPGSANKPSDD |
| SEQ ID NO: 26 | mut1 | MVRIKEGSANKPSDD |
| SEQ ID NO: 27 | mut3 | MVRGKPGSANKPSDD |
| SEQ ID NO: 28 | mut4 | MERIKPGSANKPSDD |
| SEQ ID NO: 29 | mut5 | A VRIKPGSANKPSDD |
| n=native, s= synthetic, scr =scrambled, same AA, ()= number of AA, Ab=antibody, X = any amino acid, except arginine | | |

In a further embodiment, PIF is a compound of the formula R₁-R₂-R₃-R₄-R₅-R₆-R₇-R₈- R₉-R₁₀-R₁₁-R₁₂-R₁₃-R₁₄-R₁₅, wherein R₁ is Met or a mimetic of Met or salt thereof, R₂ is Val or a mimetic of Val or salt thereof, R₃ is Arg or a mimetic of Arg, or any amino acid or salt thereof, R₄ is Ile or a mimetic of lie or salt thereof, R₅ is Lys or a mimetic of Lys or salt thereof, R₆ is Pro or a mimetic of Pro or salt thereof, R₇ is Gly or a mimetic of Gly or salt thereof, R₈ is Ser or a mimetic of Ser or salt thereof, Rg is Ala or a mimetic of Ala or salt thereof, R₁₀ is Asn or a mimetic of Asn or salt thereof, R₁₁ is Lys or a mimetic of Lys or salt thereof, R₁₂ is Pro or a mimetic of Pro or salt thereof, R₁₃ is Ser or a mimetic of Ser or salt thereof, R₁₄ is Asp or a mimetic of Asp or salt thereof and R₁₅ is Asp or a mimetic of Asp or salt thereof. In a further embodiment, a compound comprising the formula R₁-R₂-R₃-R₄-R₅-R₆-R₇-R₈- R₉-R₁₀-R₁₁-R₁₂-R₁₃-R₁₄-R₁₅, wherein R₁ is a mimetic of the naturally occurring residue at position 1 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₂ is a mimetic of the naturally occurring residue at position 2 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₃ is a mimetic of the naturally occurring residue at position 3 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₄ is a mimetic of the naturally occurring residue at position 4 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₅ is a mimetic or salt thereof of the naturally occurring residue at position 5 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₆ is a mimetic or salt thereof of the naturally occurring residue at position 6 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₇ is a mimetic or salt thereof of the naturally occurring residue at position 7 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₈ is a mimetic or salt thereof of the naturally occurring residue at position 5 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein Rg is a mimetic or salt thereof of the naturally occurring residue at position 9 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₀ is a mimetic or salt thereof of the naturally occurring residue at position 10 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₁ is a mimetic or salt thereof of the naturally occurring residue at position 11 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₂ is a mimetic or salt thereof of the naturally occurring residue at position 12 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₃ is a mimetic or salt thereof of the naturally occurring residue at position 13 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₄ is a mimetic or salt thereof of the naturally occurring residue at position 14 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₅ is a mimetic or salt thereof of the naturally occurring residue at position 15 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences.

In some embodiments the pharmaceutical compositions of the claimed invention comprises at least one or a plurality of active agents otherthan the PIF peptide, polypeptide, salt, or analog of pharmaceutically acceptable salt thereof. In some embodiments the active agent is covalently linked to the PIF peptide or PIF polypeptide, salt, or analog disclosed herein optionally by a protease cleavable linker (including by not limited to Pro-Pro or Cituline-Valine di-α-amino acid linkers). In some embodiments, the one or plurality of active agents includes one or a combination of compounds chosen from: an anti-inflammatory compound, alpha-adrenergic agonist, antiarrhythmic compound, analgesic compound, and an anesthetic compound.

**Table 5**

| Examples of anti-inflammatory compounds include: | |
|---|---|
| | aspirin |
| | celecoxib |
| | diclofenac |
| | diflunisal |
| | etodolac |
| | ibuprofen |
| | indomethacin |
| | ketoprofen |
| | ketorolac nabumetone |
| | naproxen |
| | oxaprozin |
| | piroxicam |
| | salsalate |
| | sulindac |
| | tolmetin |

| Examples of alpha-adrenergic agonists include: | |
|---|---|
| | Methoxamine |
| | Methylnorepinephrine |
| | Midodrine |
| | Oxymetazoline |
| | Metaraminol |
| | Phenylephrine |
| | Clonidine (mixed alpha2-adrenergic and imidazoline-11 receptor agonist) |
| | Guanfacine, (preference for alpha2A-subtype of adrenoceptor) |
| | Guanabenz (most selective agonist for alpha2-adrenergic as opposed to imidazoline-11) |
| | Guanoxabenz (metabolite of guanabenz) |
| | Guanethidine (peripheral alpha2-receptor agonist) |
| | Xylazine, |
| | Tizanidine |
| | Medetomidine |
| | Methyldopa |
| | Fadolmidine |
| | Dexmedetomidine |

| Examples of antiarrhythmic compounds include: | |
|---|---|
| | Amiodarone (Cordarone, Pacerone) |
| | Bepridil Hydrochloride (Vascor) |
| | Disopyramide (Norpace) |
| | Dofetilide (Tikosyn) |
| | Dronedarone (Multaq) |
| | Flecainide (Tambocor) |
| | Ibutilide (Corvert) |
| | Lidocaine (Xylocaine) |
| | Procainamide (Procan, Procanbid) |
| | Propafenone (Rythmol) |
| | Propranolol (Inderal) |
| | Quinidine (many trade names) |
| | Sotalol (Betapace) |
| | Tocainide (Tonocarid) |

| Examples of analgesic compound include: | |
|---|---|
| | codeine |
| | hydrocodone (Zohydro ER), |
| | oxycodone (OxyContin, Roxicodone), |
| | methadone |
| | hydromorphone (Dilaudid, Exalgo), |
| | morphine (Avinza, Kadian, MSIR, MS Contin), and |
| | fentanyl (Actiq, Duragesic) |

| Examples of anesthetic compounds include: | |
|---|---|
| | Desflurane |
| | Isoflurane |
| | Nitrous oxide |
| | Sevoflurane |
| | Xenon |

The compounds of the present disclosure can also be administered in combination with other active ingredients, such as, for example, adjuvants, or other compatible drugs or compounds where such combination is seen to be desirable or advantageous in achieving the desired effects of the methods described herein. When exposing the PIF peptide to any cells, tissue, or organ prior to transplantation, exposure may be anywhere from about 1 to about 12 hours. In some embodiments, the step of exposing PIF to pre-condition cells prior to transplant is from about 2 to about 4 hours. In some embodiments, the step of exposing PIF to pre-condition cells prior to transplant is about 1, 2, 3, 4, 5, 6, 7, 8, 9, 10, 11, or 12 or more hours. In some embodiments, the step of exposing PIF to the organ, tissue, or cells prior to transplant occurs any where from about 1 to about 48 hours before transplant. In some embodiments, the step of exposing PIF to the organ, tissue, or cells prior to transplant occurs is about 1, 2, 3, 4, 5, 6, 7, 8, 9, 10, 11, 12, 15, 20, 25, 35, 45, or about 50 hours before transplant occurs. In some embodiments, the PIF peptide is exposed to the organ, tissue or cells for a time and under conditions sufficient to increase the viability of the organ, tissue or cells, increase the likelihood of successful transplantation, reduce recipient acceptance. In some embodiments, the organ, tissue or cells is exposed to one or a combination of pharmaceutical compositions disclosed herein for no less than 1, 2, 3, 4, 5, 6, 7, 8, 9, 10 hours and/or at about room temperature. In some embodiments, the organ, tissue or cells is exposed to one or a combination of pharmaceutical compositions disclosed herein for no less than 1, 2, 3, 4, 5, 6, 7, 8, 9, 10 hours and/or at about 4 degree Celsius. In some embodiments, the organ, tissue or cells is exposed to one or a combination of pharmaceutical compositions disclosed herein at from about 4 degrees Celsius to about 40 degrees Celsius.

As used herein, the term "islet cells" refers to cells from any hormone-producing region of the pancreas.

As used herein, the term "adrenal cells" refers to any cell from any hormone-producing region of the adrenal gland.

As used herein, the term "pre-condition" refers to the process of treating an organ, tissue, or cell prior to its transplantation or use. Any organ, tissue, or cell may be pre-conditioned one or more times with any one or combination of PIF peptides, functional fragment, salts or peptidomimetics thereof.

In some embodiments, the PIF peptide is administered or is pre-exposed in a therapeutically effective amount. In some embodiments, the PIF peptide is administered after the subject undergoes transplant, before the subject undergoes transplant, while the subject undergoes transplant, or a combination thereof. In some embodiments, the subject may receive secondary treatment, which may include secondary administration of a PIF peptide, before the subject undergoes transplant, while the subject undergoes transplant, or a combination thereof.

In the foregoing embodiments, the PIF peptide may be administered at a dose of about 0.01 mg/kg/day, about 0.1 mg/kg/day, about 0.5 mg/kg/day, about 0.75 mg/kg/day, about 1 mg/kg/day, about 2 mg/kg/day, about 3 mg/kg/day, about 4 mg/kg/day, about 6 mg/kg/day, about 8 mg/kg/day, about 10 mg/kg/day, about 15 mg/kg/day, about 20 mg/kg/day, or any range between any of these values, including endpoints. Such doses may be administered as a single dose or as divided doses in a single day. In some embodiments, if administered once a day, the daily regimen may be repeated over two days, three day, four days, five days, six days, 7 days or more. In some embodiments, the daily regimen or regimens are repeated once a month or once every other month. In some embodiments, the dosage regimen may begin at one dose such as 1 mg/kg for any period of time in days or months and then slowly become reduced over time or escalate depending upon the health of the subject.

In the foregoing embodiments, the composition or pharmaceutical composition may be administered once, for a limited period of time or as a maintenance therapy (over an extended period of time until the condition is ameliorated, cured or for the life of the subject). A limited period of time may be for 1 week, 2 weeks, 3 weeks, 4 weeks and up to one year, including any period of time between such values, including endpoints. In some embodiments, the composition or pharmaceutical composition may be administered for about 1 day, for about 3 days, for about 1 week, for about 10 days, for about 2 weeks, for about 18 days, for about 3 weeks, or for any range between any of these values, including endpoints

In the foregoing embodiments, the composition or pharmaceutical composition may be administered once daily, twice daily, three times daily, four times daily or more.

In some embodiments, the composition or pharmaceutical composition is administered or provided as a pharmaceutical composition comprising a PIF peptide, as defined above, and a pharmaceutically acceptable carrier or diluent, or an effective amount of a pharmaceutical composition comprising a compound as defined above.

The methods disclosed herein can be used with any of the compounds, compositions, preparations, and kits disclosed herein. Methods of the disclosure include methods of treating a transplant recipient, methods of restoring endocrine function in a transplant recipient, method of enhancing endocrine function in a subject deficient in endocrine function and methods of increasing the acceptance of transplanted tissue in a subject for more than 15, 16, 17, 18, 19 or 20 days or more, by, in each case administering a therapeutically effective amount of composition comprising PIF, a PIF analog, PIF mimetic or any salt thereof to a subject in need of such treatment for a period of time sufficient to improve or restore endocrine function.

In some embodiments the methods comprise methods of transplanting ovarian tissue such that the donor tissue is biologically functional both on the local level within the subject (capable of restoring menses and ovulation) and systemically (i.e. capable of restoring endocrine crosstalk between the tissue and the nervous system of the patient). In some embodiments, the methods are successful to restore local and systemic function of the donor tissue in the subject for longer than about one month of time. In some embodiments, the methods are successful to restore local and systemic function of the donor tissue in the subject for longer than about two months of time or more. In some embodiments, the methods are successful to restore local and systemic function of the donor tissue in the subject for longer than about three months of time or more. In some embodiments, the tissue is ovarian tissue engrafted on an ovary of the subject or transplant of an entire ovary. In some embodiments, the biological function of the subject is improved or restored using transplanted tissue from a species that is not a species of the subject, such transplant procedure being recognized as a xenotransplant. In some embodiments, the step of administering the composition or pharmaceutical composition is preceded by a step of culturing cells (donor tissue) from a species that is not the species of the subject. Insome embodiments, the donor cells or donor tissues is pre-conditioned with a composition comprising a PIF peptide, mimetic, salt or functional fragment thereof.

In some embodiments, the pharmaceutical composition consists of a single active agent that is PIF, PIF functional fragment, PIF mimetic or salt thereof.

In some aspects, the disclosure relates to a pharmaceutical composition comprising a PIF peptide, PIF analog, PIF mimetic or salt thereof, plus a therapeutically effective amount of another active agent. In some embodiments, the other active agent is an agent from Table 5. In some embodiments, the other active agent is a steroid. Non-limiting examples of steroids are set forth in Table 4.

In some aspects, the disclosure relates to a method of restoring endocrine function in a subject comprising administering to the subject a therapeutically effective amount of a PIF peptide, compositions thereof, mimetics thereof, pharmaceutically acceptable salts thereof, or combinations thereof.

In some aspects, the disclosure relates to a method of restoring menstruation in a mammal in need of restoration comprising administering to the mammal a therapeutically effective amount of a PIF peptide, compositions thereof, mimetics thereof, pharmaceutically acceptable salts thereof, or combinations thereof.

In some embodiments, the disclosure relates to a method of treating congenital adrenal hyperplasia (CAH) comprising administering to a subject in need thereof a therapeutically effective amount of a PIF peptide, compositions thereof, mimetics thereof, pharmaceutically acceptable salts thereof, or combinations thereof of any of the aforementioned.

In some aspects, the disclosure relates to a method of preventing an acute adrenal crisis in a subject in need thereof having adrenal tissue dysfunction comprising administering to the subject a therapeutically effective amount of a PIF peptide, compositions thereof, mimetics thereof, pharmaceutically acceptable salts thereof, or combinations thereof of any of the aforementioned methods and/or doses.

In some aspects, the disclosure relates to a method of inducing wound healing comprising a therapeutically effective amount of a PIF peptide, compositions thereof, mimetics thereof, pharmaceutically acceptable salts thereof, or combinations thereof of any of the aforementioned.

The disclosure relates, among other things, to the treatment of a transplant receipient with one or more compositions provided herein prior to the transplantation of a donor tissue, organ or cells. The methods relate to administration of a subject with any one or multiple doses of the PIF peptide, salt, peptidomimetic, or functional fragment thereof with at a therapeutic level. In some embodiments, the methods involve the use of the PIF peptides as a monotherapy, meaning that the PIF peptide administered is the only active agent administered to the individual. In some of the method embodiments, the recipient is transplanted with any of the organs, tissue or cells dsisclosed herein, and, optionally, such cells organs or tissues are pre-conditioned with a solution comprising the one or plurality iof PIF peptides. Soaking the cells, tissue, or organ in a solution comprising PIF before transplantation improves the acceptance of the cells, tissue or organs after transplantation. The methods disclosed herein are completely distinguishable from methods of treating graft-versus-host disease or other immunological problems resulting from toxicity of transplanted tissue. The disclosure relates to rendering any transplanted tissue, cells or organs more agreeable to acceptance by relying on the immune regulatory function of PIF before the transplant takes place. Administration of PIF to the subject induces expression of class I HLA molecules, such as HLA-E and HLA-F such that any transplantation tissue recipient is less likely to respond to reject the donor tissue or cells. This method is a treatment for transplant recipients of endocrine tissue and the balanced immuneoregulatory environment allows fr restortiation of transplanted tossue function for day week and even moneths at a time. In some embodiments, the result is achieved and any and all of the methods comprise steps without the use or free of administration of immunocompromising agents or procedures, such as steroids or immune depletion techniques. As an enchanced bonus and other surpsing finding, even if you co administer steroid, such as dexamethoasone or a steroid such as a dexamethasone analog, and a PIF peptide, the necessary amounts of steroid that have a therapeutic effect are lowered (the effects are potentiated) as compared to a subject receiving steroid alone. Another surprising feature is that pre-coinditioning any transplant tissue prior to trnasplaantaiton in any of the disclosed methods, increases the tolerance of the recipient in receiving the donor cells, tissue or organs. Exposure to PIF before and after transplantation also allows the tissue to remain unattacked and functional by the host immune response for months after a transplantation.

The dose of the composition or pharmaceutical compositions may vary. The dose of the composition may be once per day. In some embodiments, multiple doses may be administered to the subject per day. In some embodiments, the total dosage is administered in at least two application periods. In some embodiments, the period can be an hour, a day, a month, a year, a week, or a two-week period. In an additional embodiment of the invention, the total dosage is administered in two or more separate application periods, or separate doses over the course of about an hour, 2 hours, 3 hours, 4 hours, 5 hours, 6 hours, 7 hours, 8 hours, 9 hours, 10 hours, 11 hours, 12 or more hours, a day, a month, a year, a week, or a two-week period.

In some embodiments, subjects can be administered the composition in which the composition is provided in a daily dose range of about 0.0001 mg/kg to about 5000 mg/kg of the weight of the subject. The dose administered to the subject can also be measured in terms of total amount of PIF peptide or PIF analog or pharmaceutically acceptable salt thereof administered per day. In some embodiments, a subject is administered from about 0.001 to about 3000 milligrams of PIF peptide or PIF analog or pharmaceutically acceptable salt thereof per day. In some embodiments, a subject is administered up to about 2000 milligrams of PIF peptide or PIF analog or pharmaceutically acceptable salt thereof per day. In some embodiments, a subject is administered up to about 1800 milligrams of PIF peptide or PIF analog or pharmaceutically acceptable salt thereof per day. In some embodiments, a subject is administered up to about 1600 milligrams of PIF peptide or PIF analog or pharmaceutically acceptable salt thereof per day. In some embodiments, a subject is administered up to about 1400 milligrams of PIF peptide or PIF analog or pharmaceutically acceptable salt thereof per day. In some embodiments, a subject is administered up to about 1200 milligrams of PIF peptide or PIF analog or pharmaceutically acceptable salt thereof per day. In some embodiments, a subject is administered up to about 1000 milligrams of PIF peptide or PIF analog or pharmaceutically acceptable salt thereof per day. In some embodiments, a subject is administered up to about 800 milligrams of PIF peptide or PIF analog or pharmaceutically acceptable salt thereof per day. In some embodiments, a subject is administered from about 0.001 milligrams to about 700 milligrams of PIF peptide or PIF analog or pharmaceutically acceptable salt thereof per dose. In some embodiments, a subject is administered up to about 700 milligrams of PIF peptide or PIF analog per dose. In some embodiments, a subject is administered up to about 600 milligrams of PIF peptide or PIF analog or pharmaceutically acceptable salt thereof per dose. In some embodiments, a subject is administered up to about 500 milligrams of PIF peptide or PIF analog or pharmaceutically acceptable salt thereof per dose. In some embodiments, a subject is administered up to about 400 milligrams of PIF peptide or PIF analog or pharmaceutically acceptable salt thereof per dose. In some embodiments, a subject is administered up to about 300 milligrams of PIF peptide or PIF analog or pharmaceutically acceptable salt thereof per dose. In some embodiments, a subject is administered up to about 200 milligrams of PIF peptide or PIF analog or pharmaceutically acceptable salt thereof per dose. In some embodiments, a subject is administered up to about 100 milligrams of PIF peptide or PIF analog or pharmaceutically acceptable salt thereof per dose. In some embodiments, a subject is administered up to about 50 milligrams of PIF peptide or PIF analog or pharmaceutically acceptable salt thereof per dose.

In some embodiments, subjects can be administered the composition in which the composition comprising a PIF peptide or PIF analog or pharmaceutically acceptable salt thereof is administered in a daily dose range of about 0.0001 mg/kg to about 5000 mg/kg of the weight of the subject. In some embodiments, the composition comprising a PIF analog or pharmaceutically acceptable salt thereof is administered in a daily dosage of up to about 450 mg/kg of the weight of the subject. In some embodiments, the composition comprising a PIF peptide or PIF analog or pharmaceutically acceptable salt thereof is administered in a daily dosage of up to about 400 mg/kg of the weight of the subject. In some embodiments, the composition comprising a PIF peptide or PIF analog or pharmaceutically acceptable salt thereof is administered in a daily dosage of up to about 350 mg/kg of the weight of the subject. In some embodiments, the composition comprising a PIF peptide or PIF analog or pharmaceutically acceptable salt thereof is administered in a daily dosage of up to about 300 mg/kg of the weight of the subject. In some embodiments, the composition comprising a PIF peptide or PIF analog or pharmaceutically acceptable salt thereof is administered in a daily dosage of up to about 250 mg/kg of the weight of the subject. In some embodiments, the composition comprising PIF peptide or a PIF analog or pharmaceutically acceptable salt thereof is administered in a daily dosage of up to about 200 mg/kg of the weight of the subject. In some embodiments, the composition comprising PIF peptide or a PIF analog or pharmaceutically acceptable salt thereof is administered in a daily dosage of up to about 150 mg/kg of the weight of the subject. In some embodiments, the composition comprising a PIF peptide or a PIF analog or pharmaceutically acceptable salt thereof is administered in a daily dosage of up to about 100 mg/kg of the weight of the subject. In some embodiments, the composition comprising a PIF peptide or a PIF analog or pharmaceutically acceptable salt thereof is administered in a daily dosage of up to about 50 mg/kg of the weight of the subject. In some embodiments, the composition comprising PIF peptide or a PIF analog or pharmaceutically acceptable salt thereof is administered in a daily dosage of up to about 25 mg/kg of the weight of the subject.

In some embodiments, the composition comprising a PIF peptide or a PIF analog or pharmaceutically acceptable salt thereof is administered in a daily dosage of up to about 10 mg/kg of the weight of the subject. In some embodiments, the composition comprising PIF peptide or a PIF analog or pharmaceutically acceptable salt thereof is administered in a daily dosage of up to about 5 mg/kg of the weight of the subject. In some embodiments, the composition comprising PIF peptide or a PIF analog or pharmaceutically acceptable salt thereof is administered in a daily dosage of up to about 1 mg/kg of the weight of the subject. In some embodiments, the composition comprising a PIF peptide or a PIF analog or pharmaceutically acceptable salt thereof is administered in a daily dosage of up to about 0.1 mg/kg of the weight of the subject. In some embodiments, the composition comprising a PIF analog or pharmaceutically acceptable salt thereof is administered in a daily dosage of up to about 0.01 mg/kg of the weight of the subject. In some embodiments, the composition comprising a PIF analog or pharmaceutically acceptable salt thereof is administered in a daily dosage of up to about 0.001 mg/kg of the weight of the subject. The dose administered to the subject can also be measured in terms of total amount of a PIF peptide or PIF analog administered per day.

In some embodiments, a subject in need thereof is administered from about 1 ng to about 500 µg of analog or pharmaceutically salt thereof per day. In some embodiments, a subject in need thereof is administered from about 1 ng to about 10 ng of analog or pharmaceutically salt thereof per day. In some embodiments, a subject in need thereof is administered from about 10 ng to about 20 ng of analog or pharmaceutically salt thereof per day. In some embodiments, a subject in need thereof is administered from about 10 ng to about 100 ng of analog or pharmaceutically salt thereof per day. In some embodiments, a subject in need thereof is administered from about 100 ng to about 200 ng of analog or pharmaceutically salt thereof per day. In some embodiments, a subject in need thereof is administered from about 200 ng to about 300 ng of analog or pharmaceutically salt thereof per day. In some embodiments, a subject in need thereof is administered from about 300 ng to about 400 ng of analog or pharmaceutically salt thereof per day. In some embodiments, a subject in need thereof is administered from about 400 ng to about 500 ng of analog or pharmaceutically salt thereof per day. In some embodiments, a subject in need thereof is administered from about 500 ng to about 600 ng of analog or pharmaceutically salt thereof per day. In some embodiments, a subject in need thereof is administered from about 600 ng to about 700 ng of analog or pharmaceutically salt thereof per day. In some embodiments, a subject in need thereof is administered from about 800 ng to about 900 ng of analog or pharmaceutically salt thereof per day. In some embodiments, a subject in need thereof is administered from about 900 ng to about 1 µg of analog or pharmaceutically salt thereof per day. In some embodiments, a subject in need thereof is administered from about 1 µg to about 100 µg of analog or pharmaceutically salt thereof per day. In some embodiments, a subject in need thereof is administered from about 100 µg to about 200 µg of analog or pharmaceutically salt thereof per day. In some embodiments, a subject in need thereof is administered from about 200 µg to about 300 µg of analog or pharmaceutically salt thereof per day. In some embodiments, a subject in need thereof is administered from about 300 µg to about 400 µg of analog or pharmaceutically salt thereof per day. In some embodiments, a subject in need thereof is administered from about 400 µg to about 500 µg of analog or pharmaceutically salt thereof per day. In some embodiments, a subject in need thereof is administered from about 500 µg to about 600 µg of analog or pharmaceutically salt thereof per day. In some embodiments, a subject in need thereof is administered from about 600 µg to about 700 µg of analog or pharmaceutically salt thereof per day. In some embodiments, a subject in need thereof is administered from about 800 µg to about 900 µg of analog or pharmaceutically salt thereof per day. In some embodiments, a subject in need thereof is administered from about 900 µg to about 1 mg of analog or pharmaceutically salt thereof per day. In some embodiments, a subject in need thereof is administered from about .0001 to about 3000 milligrams of a PIF peptide or PIF analog or pharmaceutically salt thereof per day. In some embodiments, a subject is administered up to about 2000 milligrams of a PIF peptide or PIF analog or pharmaceutically salt thereof day. In some embodiments, a subject is administered up to about 1800 milligrams of a PIF peptide or PIF analog or pharmaceutically salt thereof per day. In some embodiments, a subject is administered up to about 1600 milligrams of a PIF peptide or PIF analog or pharmaceutically salt thereof per day. In some embodiments, a subject is administered up to about 1400 milligrams of a PIF peptide or PIF analog or pharmaceutically salt thereof per day. In some embodiments, a subject is administered up to about 1200 milligrams of a PIF peptide or PIF analog or pharmaceutically salt thereof per day. In some embodiments, a subject is administered up to about 1000 milligrams of a PIF peptide or PIF analog or pharmaceutically salt thereof per day. In some embodiments, a subject is administered up to about 800 milligrams of a PIF peptide or PIF analog or pharmaceutically salt thereof per day. In some embodiments, a subject is administered from about 0.0001 milligrams to about 700 milligrams of a PIF peptide or PIF analog or pharmaceutically salt thereof per dose. In some embodiments, a subject is administered up to about 700 milligrams of a PIF peptide or PIF analog or pharmaceutically salt thereof per dose. In some embodiments, a subject is administered up to about 600 milligrams of a PIF peptide or PIF analog or pharmaceutically salt thereof per dose. In some embodiments, a subject is administered up to about 500 milligrams of a PIF peptide or PIF analog or pharmaceutically salt thereof per dose. In some embodiments, a subject is administered up to about 400 milligrams of a PIF peptide or PIF analog or pharmaceutically salt thereof per dose. In some embodiments, a subject is administered up to about 300 milligrams of a PIF peptide or PIF analog or pharmaceutically salt thereof per dose. In some embodiments, a subject is administered up to about 200 milligrams of a PIF peptide or PIF analog or pharmaceutically salt thereof per dose. In some embodiments, a subject is administered up to about 100 milligrams of a PIF peptide or PIF analog or pharmaceutically salt thereof per dose. In some embodiments, a subject is administered up to about 50 milligrams of a PIF peptide or PIF analog or pharmaceutically salt thereof per dose. In some embodiments, a subject is administered up to about 25 milligrams of a PIF peptide or PIF analog or pharmaceutically salt thereof per dose. In some embodiments, a subject is administered up to about 15 milligrams of a PIF peptide or PIF analog or pharmaceutically salt thereof per dose.

In some embodiments, a subject is administered up to about 10 milligrams of a PIF peptide or PIF analog or pharmaceutically salt thereof per dose. In some embodiments, a subject is administered up to about 5 milligrams of a PIF peptide or PIF analog or pharmaceutically salt thereof per dose. In some embodiments, a subject is administered up to about 1 milligram of a PIF peptide or PIF analog or pharmaceutically salt thereof per dose. In some embodiments, a subject is administered up to about 0.1 milligrams of a PIF peptide or PIF analog or pharmaceutically salt thereof per dose. In some embodiments, a subject is administered up to about 0.001 milligrams of a PIF peptide or PIF analog or pharmaceutically salt thereof per dose.

The dose administered to the subject can also be measured in terms of total amount of a PIF peptide or PIF analog or pharmaceutically salt thereof administered per ounce of liquid prepared. In some embodiments, the PIF peptide or PIF analog or pharmaceutically salt thereof is at a concentration of about 2.5 grams per ounce of solution. In some embodiments, the PIF peptide or PIF analog or pharmaceutically salt thereof is at a concentration of about 2.25 grams per ounce of solution. In some embodiments, the PIF peptide or PIF analog or pharmaceutically salt thereof is at a concentration of about 2.25 grams per ounce of solution. In some embodiments, the PIF peptide or PIF analog or pharmaceutically salt thereof is at a concentration of about 2.0 grams per ounce of solution. In some embodiments, the PIF peptide or PIF analog or pharmaceutically salt thereof is at a concentration of about 1.9 grams per ounce of solution. In some embodiments, the PIF peptide or PIF analog or pharmaceutically salt thereof is at a concentration of about 1.8 grams per ounce of solution. In some embodiments, the PIF analog or pharmaceutically salt thereof is at a concentration of about 1.7 grams per ounce of solution. In some embodiments, the PIF peptide or PIF analog or pharmaceutically salt thereof is at a concentration of about 1.6 grams per ounce of solution. In some embodiments, the PIF peptide or PIF analog or pharmaceutically salt thereof is at a concentration of about 1.5 grams per ounce of solution. In some embodiments, the PIF peptide or PIF analog or pharmaceutically salt thereof is at a concentration of about 1.4 grams per ounce of solution. In some embodiments, the PIF peptide or PIF analog or pharmaceutically salt thereof is at a concentration of about 1.3 grams per ounce of solution.

In some embodiments, the PIF peptide or PIF analog or pharmaceutically salt thereof is at a concentration of about 1.2 grams per ounce of solution. In some embodiments, the PIF peptide or PIF analog or pharmaceutically salt thereof is at a concentration of about 1.1 grams per ounce of solution. In some embodiments, the PIF peptide or PIF analog or pharmaceutically salt thereof is at a concentration of about 1.0 grams per ounce of solution.

In some embodiments, the PIF peptide or PIF analog or pharmaceutically salt thereof is at a concentration of about 0.9 grams per ounce of solution. In some embodiments, the PIF peptide or PIF analog or pharmaceutically salt thereof is at a concentration of about 0.8 grams per ounce of solution. In some embodiments, the PIF peptide or PIF analog or pharmaceutically salt thereof is at a concentration of about 0.7 grams per ounce of solution. In some embodiments, the PIF peptide or PIF analog or pharmaceutically salt thereof is at a concentration of about 0.6 grams per ounce of solution. In some embodiments, the PIF peptide or PIF analog or pharmaceutically salt thereof is at a concentration of about 0.5 grams per ounce of solution. In some embodiments, the PIF peptide or PIF analog or pharmaceutically salt thereof is at a concentration of about 0.4 grams per ounce of solution. In some embodiments, the PIF peptide or PIF analog or pharmaceutically salt thereof is at a concentration of about 0.3 grams per ounce of solution. In some embodiments, the PIF peptide or PIF analog or pharmaceutically salt thereof is at a concentration of about 0.2 grams per ounce of solution. In some embodiments, the PIF peptide or PIF analog or pharmaceutically salt thereof is at a concentration of about 0.1 grams per ounce of solution. In some embodiments, the PIF peptide or PIF analog or pharmaceutically salt thereof is at a concentration of about 0.01 grams per ounce of solution. In some embodiments, the PIF peptide or PIF analog or pharmaceutically salt thereof is at a concentration of about 0.001 grams per ounce of solution prepared. In some embodiments, the PIF peptide or PIF analog or pharmaceutically salt thereof is at a concentration of about 0.0001 grams per ounce of solution prepared. In some embodiments, the PIF peptide or PIF analog or pharmaceutically salt thereof is at a concentration of about 0.00001 grams per ounce of solution prepared. In some embodiments, the PIF peptide or PIF analog or pharmaceutically salt thereof is at a concentration of about 0.000001 grams per ounce of solution prepared.

The disclosure relates to any method containing a transplantation step of transplanting tissues, organs or cells that are preconditioned with a composition comprising PIF peptide, salt, peptidomimetic or functional fragment thereof prior to the step of transplantation. In some embodiments, the step of preconditioning comprising exposing the cells, tissues, or organs with a pharmaceutical composition comprising an amount of PIF from about 0.1 to about 100 mg/mL.

In some embodmients, the step of preconditioning comprising exposing the cells, tissues, or organs with a pharmaceutical composition comprising an amount of PIF of about 1.0 mg/mL. In some embodmients, the step of preconditioning comprising exposing the cells, tissues, or organs with a pharmaceutical composition comprising an amount of PIF of about 2.0 mg/mL.In some embodmients, the step of preconditioning comprising exposing the cells, tissues, or organs with a pharmaceutical composition comprising an amount of PIF of about 3.0 mg/mL.In some embodmients, the step of preconditioning comprising exposing the cells, tissues, or organs with a pharmaceutical composition comprising an amount of PIF of about 4.0 mg/mL.In some embodmients, the step of preconditioning comprising exposing the cells, tissues, or organs with a pharmaceutical composition comprising an amount of PIF of about 5.0 mg/mL.In some embodmients, the step of preconditioning comprising exposing the cells, tissues, or organs with a pharmaceutical composition comprising an amount of PIF of about 6.0 mg/mL.In some embodmients, the step of preconditioning comprising exposing the cells, tissues, or organs with a pharmaceutical composition comprising an amount of PIF of about 7.0 mg/mL.In some embodmients, the step of preconditioning comprising exposing the cells, tissues, or organs with a pharmaceutical composition comprising an amount of PIF of about 8.0 mg/mL.In some embodmients, the step of preconditioning comprising exposing the cells, tissues, or organs with a pharmaceutical composition comprising an amount of PIF of about 9.0 mg/mL.In some embodmients, the step of preconditioning comprising exposing the cells, tissues, or organs with a pharmaceutical composition comprising an amount of PIF of about 10.0 mg/mL. In some embodmients, the step of preconditioning comprising exposing the cells, tissues, or organs with a pharmaceutical composition comprising an amount of PIF of about 0.5 mg/mL.

The disclosure also relates to a method of potentiating steroid treatment of a subject by adminstering a PIF peptide before, contemporaneous with or after administration of the steroid. In some embodiments, the steroid is dexamethasone or an analog thereof.

The disclosure also relates to reducing cortisol secretion in a subject by administering to the subject a therapeutically effective amount of a PIF peptide, compositions thereof, mimetics thereof, pharmaceutically acceptable salts thereof, or combinations thereof.

In some embodiments, the disclosure relates to a method of restoring endocrine function in a subject deficient in endorcrine function comprising:
(i) transplanting one or a plurality of endocrine cells, tissues, or organs in the subject; and
(ii) administering to the subject a therapeutically effective amount of a PIF peptide, compositions thereof, mimetics thereof, pharmaceutically acceptable salts thereof, or combinations thereof. In some embodiments, the subject is administered in any route of administration disclosed herein and is administered one or more time before the transplantation of the one or plurality of endocrine cells. In some embodiments, the one or plurality of endocrine cells are selected fron one or a combination of pancreatice islet cells, adrenal cells or ovarian cells. In some embodiments, the one or plurality of cells are ovarian, adrenal gland, or pancreatic tissues. In some embodidments, the one or plurality of edocrine cells are a ovary or an adrenal gland. In some embodiments, the one or plurality of cells are pre-conditioned with the PIF peptide, compositions thereof, mimetics thereof, pharmaceutically acceptable salts thereof, or combinations thereof prior to transplantation.

All referenced journal articles, patents, and other publications are incorporated by reference herein in their entirety.

### EXAMPLES

### Example 1

### Prelmplantation Factor (PIF) promotes HLA-G, -E, -F, -C expression in JEG-3 choriocarcinoma cells and endogenous progesterone activity

### Abstract

BACKGROUND: Pregnancy success requires mandatory maternal tolerance of the semi/allogeneic embryo involving embryo-derived signals. Expression levels of Prelmplantation Factor (PIF), a novel peptide secreted by viable embryos, correlate with embryo development, and its early detection in circulation correlates with a favorable pregnancy outcome. PIF enhances endometrial receptivity to promote embryo implantation. Via the p53 pathway, it increases trophoblast invasion, improving cell survival / immune privilege. PIF also reduces spontaneous and LPS-induced fetal death in immune naive murine model.

AIMS: To examine if PIF affects gene expression of human leukocyte antigen (HLA)-G, -E -F and -C in JEG-3 choriocarcinoma cells, and to examine the influence of PIF on local progesterone activity.

METHODS: PIF and progesterone (P4) effects on JEG-3 cells surface and intracellular HLA molecules was tested using monoclonal antibodies, flow cytometry, and Western blotting. PIF and IL17 effects on P4 and cytokines secretion was determined by ELISA. PIF and P4 effects on JEG-3 cells proteome was examined using 2D gel staining followed by spot analysis, mass spectrometry and bioinformatic analysis.

RESULTS: In cytotrophoblastic JEG-3 cells PIF increased intracellular expression of HLA-G, HLA-F, HLA-E and HLA-C and surface expression of HLA-G, HLA-E and HLA-C in dose and time dependent manner. In case of HLA-E, F confirmed also by Western blotting. Proteome analysis confirmed an increase in HLA-G, pro-tolerance FOXP3+ regulatory T cells (Tregs), coagulation factors and complement regulator. In contrast, PIF reduced PRDX2 and HSP70s to negate oxidative stress and protein misfolding. PIF enhanced local progesterone activity, increasing steroid secretion and the receptor protein. It also promoted the secretion of the Th1/Th2 cytokines (IL-10, IL-1β, IL-8, GM-CSF and TGF-β1), resulting in improved maternal signaling.

CONCLUSION: PIF can generate a pro-tolerance milieu by enhancing the expression of HLA molecules and by amplifying endogenous progesterone activity. A Fast-Track clinical trial for autoimmune disease has been satisfactorily completed. The acquired data warrants PIF use for the treatment of early pregnancy disorders.

### Introduction

Mammalian pregnancy involves the successful transplant of a semi-allogeneic or allogeneic graft, whether originating through natural conception, donor embryo acceptance or cross-species embryo transfer. Paradoxically, the maternal immune system remains competent and active during pregnancy and does not reject the fetus, as it would any other transplant [1]. Embryo rejection, in fact, indicates a pregnancy complication. It is also noteworthy that autoimmune conditions, unless severe, improve during pregnancy only to recur post-partum, indicating the existence of a unique temporary immunological milieu specific to pregnancy [2-4]. Despite extensive investigations, an inclusive explanation as to why the fetus is offered special immunologic privilege has not been forthcoming [5, 6]. It is assumed, however, that pregnancy-specific compounds play an important role [7].

Placental trophoblast cells play a key role in maintaining tolerance to the fetus [8]. Extravillous trophoblasts (EVTs), which invade the decidual stroma (interstitial invasion) and open the uterine spiral arteries [9-11], selectively express the non-classical class Ib antigens (Ag) HLA-G, HLA-E and HLA-F, and HLA-C, a non-classical class Ia Ag [10]. However, HLA-A and -B, both T-cell related HLA ligands [12] are absent from EVT, which may prevent attack by maternal cytotoxic CD8+ T lymphocytes. Progesterone (P4) promotes trophoblast invasion [13], and it increases HLA-G expression in primary trophoblasts and JEG-3 cells [14-16]. In JEG-3 cells, P4 is able to induce heterotypic associations between HLA-G and -E and cell-surface expression of HLA-C, -E and -G [15, 17]. However, early in gestation P4 is of corpus luteum origin, and the level of circulating P4 is low [18]. Effective local steroid production is only taken over by the placenta by week 12 of gestation [19]. Thus, the role of pregnancy specific endogenous compound(s) in regulating trophoblast class I HLA molecules remains currently incomplete. Our premise is that immune modulation and embryo/foetus acceptance are specifically embryo-derived and embryo-driven, in coordination with the maternal immune response.

Prelmplantation Factor (PIF), a small peptide secreted by viable embryos, is likely to play an important role in maternal recognition that leads to tolerance for the semi/allogenic embryo [20-22]. PIF is detectable as early as the two-cell stage and is associated with embryo development [23, 24]. Circulating levels of PIF during early pregnancy are associated with a favorable pregnancy outcome [25]. PIF has an essentially autotrophic effect on embryo development, which is blocked by anti-PIF antibody [23]. In the embryo, PIF targets protein-disulfide isomerase/thioredoxin and heat shock proteins (HSPs), promoting embryo development and protecting against serum toxicity [23, 24, 26]. Additionally, PIF lowers natural killer (NK) cell toxicity [27]. PIF promotes endometrial receptivity to support embryo implantation [28-30]. It regulates both systemic and local maternal immunity, creating Th2 bias while preserving an effective anti-pathogenic Th1 response [31-33]. These findings have been translated to treatment of diverse immune disorders, transplantation, and brain injury models outside of pregnancy. Recently, a Fast-Track FDA clinical trial evaluating PIF for the treatment of an autoimmune disorder has been completed satisfactorily (NCT02239562) [34-41].

PIF expression in the placenta is highest shortly post-implantation, and declines around term [20, 25, 42]. A premature decline in PIF has been associated with preeclampsia and intrauterine growth retardation, thus evidencing the peptide's important role in maintaining effective placental function [41, 43]. PIF promotes invasion by extra villous cells (EVTs), without affecting these cells' proliferation [42, 44]. This was shown with transformed trophoblasts and confirmed by using primary human EVTs. This in line with data showing that EVTs HLA-G+ are drivers of the immune response through their interaction with decidual immunity [45]. PIF's effect on EVTs invasion is dependent on increased metalloproteinase 9 and reduction of its inhibitor and integrin regulation. Pathway analysis demonstrated that PIF action is dependent on the MAPK, PI3K, and JAK-STAT pathways [42]. PIF acts on, and its effect is dependent on, critical apoptosis regulating the p53 pathway [46]. Relevance of the p38 MAPK/ERK signaling pathway also in polychlorinated biphenyls -induced apoptosis of human transformed EVT was reported [47]. Presently, there is limited information on local compounds that regulate HLA expression, especially during the earliest stages of pregnancy when it is most critical. Both PIF and HLA-G are secreted by viable embryos [23, 48] and interact intimately from the earliest stages of embryo development. This interaction continues, with PIF and HLA-G present in the circulation in later pregnancy. PIF and HLA-G are expressed by the trophoblast, as PIF is expressed by the viable embryo immediately post-fertilization and by the trophoblast shortly after implantation [23, 42]. Both ligands may therefore play a local regulatory role in trophoblast HLA class I function.

HLA-G expression in different trophoblastic cells was previously examined showing that JEG-3 cytotrophoblast cells expression is higher than Bewo and Jar cells [16]. In the present study, the effect of PIF on the expression of the HLA class I molecules HLA-G, -C, -E and -F in JEG-3 cells are examined using a novel and validated co-localization and image processing approach [15]. Results were confirmed by proteome analysis. The effect of PIF was compared to that of progesterone (P4), a known HLA-G/HLA-E regulator. Whether PIF regulates endogenous P4 activity and Th1/Th2 cytokine secretion was also determined. Herein we reveal that PIF up-regulates several HLAs, potentiates progesterone action and promotes Th1/Th2 cytokine secretion by trophoblast cells. Since PIF is being tested clinically, our observations support its use for the treatment of early pregnancy disorders.

### Materials and Methods

### Test compounds

Synthetic PIF (MVRIKPGSANKPSDD) was obtained from Biosynthesis, Lewisville, NJ USA. Peptide had >95% purity, documented by mass spectrometry before use. PIF was dissolved in PBS with 0.01% dimethyl sulfoxide (DMSO) (SIGMA, Missouri, USA). Progesterone (P4) (Sigma-Aldrich, Missouri, USA) was dissolved by using absolute ethanol. IL-17RA (Life Technologies) was dissolved in Millipore water.

### Monoclonal Antibodies (mAbs)

HLA-G and HLA-E antibody specificity was previously validated through FACS at the Third International Conference on HLA-G (Paris, July 2003) [47] and through separation validation studies, as reported by Palmisano [48] and Zhao [49]. (Table 1) MEM-G/09 (EXBIO Praha, Vestec, Czech Republic), the IgG1 conformational antibody against HLA-G1 and HLA-G5, previously defined for fluorescence-activated cell sorting (FACS) and immunohistochemistry (IHC) staining, was used. For Western blotting, the MEM-G/01 (IgG1) (EXBIO Praha) clone, which recognizes the denatured HLA-G heavy chain of all isoforms, was used. For detection of the HLA-E molecule using Western blotting, MEM-E/02, (IgG1) (EXBIO Praha), which reacts specifically with all denatured HLA-E molecules and does not cross-react with HLA-A, -B, -C or -G, was used. For FACS and IHC staining, MEM-E/07 (IgG1) (EXBIO Praha), which recognizes the native surface HLA-E molecules, was used; this however is reported to cross-react with the classical MHC class I molecules HLA-B7, HLA-B8, HLA-B27 and HLA-B44. Anti-HLA-F clone 3D11 (IgG1), which recognizes the native and denatured forms of HLA-F and does not cross react with any other HLA-F type, was kindly provided by Dr. Daniel Geraghty (Seattle), and used for FACS, Western blotting and IHC staining. L31 (IgG1) (Media Pharma, Chieiti, Italy) antibody is known to bind to an epitope present on all HLA-C alleles (CW1 through to CW8), and is also known to react with HLA-B alleles (HLA-B7, -B8, -B35, -B51 and others). It was used for both FACS and microscopy, whilst the HLA-C clone D-9 (Biolegend, San Diego, CA, U.S.A) was used for Western blotting.

### Testing the influence of PIF and P4 on the expression of HLA class I molecules

For determining the effect of PIF and P4 and in combination as well as combination of PIF with Dexamethasone was examined on HLA class I molecule expression, JEG-3 cells were passaged and cultured at a density of 1×10⁶ cells/ml in complete medium. After 24 h, the cells were serum starved by replacing the medium with DMEM-F12 supplemented with 0.1% FCS. Cells were incubated for 6 h, after which the medium was refreshed and supplemented with PIF (0-1000 nM), added for 24-72 h, or P4 (0-1ug/ml), added for 24h, as recently described [15]. Cells without PIF or P4, or serum free cultured cells, were used as control. The collected cells were further tested for expression for class I HLA molecules by using specific monoclonal antibodies.

### Protein extraction and analysis by SDS-PAGE

Treated and untreated JEG-3 cells, following exposure to test agents were detached, counted and pelleted as described previously [14]. They were immediately lysed using SDS-lysis buffer, vortexed and heated at 95°C for 5 min. Cell lysates were stored at -20°C until used for protein analysis. To quantify the final concentration of the proteins we used the Bradford assay following the previously described protocol with some modifications [15]. Briefly, bovine serum albumin (BSA), at a concentration of 4 mg/ml, was used as a calibration standard. Five µl of BSA was diluted sequentially in a 96-well microplate prefilled with PBS to produce the standard curve. Five µl of each protein sample was then diluted 1:1 with PBS and incubated with the reading reagent at room temperature for 5 min. Finally, the protein concentration was determined using a spectrophotometer, extrapolating each absorbance value over the previously created standard curve. Samples were diluted in SDS-sample buffer, heated for 5 min at 95°C and loaded onto a polyacrylamide gel (12% resolving gel and 4% stacking gel). The samples were run for 30 min at 30 V, following increase to 100 V until the gel finished running. A pre-stained standard protein marker (Li-Cor Bioscience) was also loaded onto the gel and run parallel to the protein samples to be analyzed.

### Western blot analysis of HLAs

The proteins resolved using SDS-PAGE were then transferred onto a PVDF membrane (Immobilion Millipore Inc.), using a Mini Trans-Blot Cell (Bio Rad). Briefly, before transfer the SDS-PAGE gel was incubated in gel running buffer (25 mM Tris/HCI, 250 mM glycine, 0.1% SDS) for 15 min. The PVDF membrane was hydrated in absolute methanol for 10 s and immediately washed with molecular biology grade water. A stack consisting of sponge, Whatman paper soaked in transfer buffer (20mM Na₂PO₄, 2% Methanol, 0.05% SDS), the PVDF membrane, the SDS-PAGE gel, Whatman paper and sponge, was then made. This stack was placed on the electro blotter and transferred to the blotting system. The transfer was done for 1 h at 110 mA and 40 V. After transfer, the membrane was washed with molecular biology grade water and incubated in blocking buffer (0.1% Tween, 3% dried skimmed milk and PBS) for a 1 h at room temperature or overnight at 4°C. After blocking, the membrane was washed and incubated with the primary monoclonal antibody for HLA-G, -E, -C and -F overnight at 4°C. The membrane was then washed three times (10 min each wash) and incubated with secondary antibody (IRDye 800CW^{©} Donkey anti-Mouse IgG from Li-Cor Biosciences) for 1 hour at room temperature. Membranes were read using an Odyssey^{©} infrared imaging system (Li-Cor Biosciences). Semi-quantification of each antigen studied using this technique was attained by comparing loading control band (BSA) brightness and thickness using ImageJ software (http://imagej.net/).

### Flow cytometry analysis of HLAs

For surface antigen expression analysis, cells were detached using Accutase and washed with PBS. Cells were counted and at least 1×10⁶ cells were used per sample. Each sample was blocked with 0.1% BSA in PBS for 30 min at room temperature. For intracellular staining, after detaching and washing cells, the pellet was fixed with 4% paraformaldehyde on ice. Cells were then washed with 0.1% saponin-BSA in PBS, and permeabilized for 10 min at room temperature using 0.3% saponin in PBS.

Cells were washed with PBS and incubated with saturating concentrations of primary HLA antibodies, followed by washing and labelling with a conjugated secondary antibody. Cells were then re-suspended in 500 µl of PBS and at least 10,000 events were acquired using a BD FACS Aria I equipped with the FACS Diva software (BD Biosciences). The raw data analysis was performed using FlowJo Vx software (Tree Star Inc.).

### Surface HLA antigen quantification

For surface antigen quantification, a previously described protocol was followed [15]. Briefly, we used the Qifikit beads kit (Dako). The cells were prepared following the same step as for flow cytometry up to the primary HLA antibody staining stage. For detection of antibody staining cell samples, set up beads and calibration beads were all stained with FITC conjugated secondary antibody. A FACS Aria I was calibrated for the cell isotype and setup beads. A calibration curve was constructed for mean fluorescence intensity for each population of beads. The cell antigen-binding capacity was calculated by extrapolation on the calibration curve.

### PIF and IL-17 effect on P4 secretion

The effect of PIF (200nM) and IL-17 (0-100 ng/ml) on P4 secretion was tested by measuring culture supernatant P4. JEG-3 cells were cultured for 24-72 h, after which supernatants were collected and kept at -20°C until determining P4 levels by ELISA. Cells cultured in incomplete media (DMEM/Ham's F12) were used as controls

### PIF-induced HLA-G staining of JEG-3 cells

JEG-3 cells were seeded in 8-well Lab-Tek chambers (Thermo Fisher Scientific) at a density of 8×10³ per well. Cells were grown in complete medium up to 60% confluence, after which they were incubated in a serum-starved medium (0.1% FCS) for 24 h. The cells were then treated with PIF at a concentration of 200nM for 24 h followed by fixing with 4% PFA at 4°C and permeation with 0.25% Triton X-100 in PBS. The reaction was then treated with 2% BSA in PBS to block non-specific binding, for 1h at room temperature. Cells were then incubated with the primary antihuman monoclonal antibodies anti-HLA-G (clone MEM-G/09) and anti-HLA-E (clone MEM-E/07) at a dilution of 2 µg/100 µl in PBS for 1 h and anti-HLA-C (clone L31) and anti-HLA-F (clone 3D11) at a dilution of 1 µg/100µl in PBS for 1 h. Cells were washed with PBS, after which they were incubated with anti-mouse IgG conjugated with Alexa Fluor 488 or Alexa Fluor 555 (Invitrogen, Carlsbad, CA, USA) at a dilution of 0.25 µg/100 µl for 1 h at room temperature. Cells were washed once again and air dried. The cells were then mounted and the cell nuclei stained using Vectashield mounting medium with DAPI (Vector Laboratories, Burlingame, CA, USA), covered with coverslips (Chance proper LTD, West Midlands, England) and sealed with Marabu Fixogum rubber cement (Marabuwerke GmbH & Co. KG, Tamm, Germany).

### Two-dimensional electrophoresis (2-DE) and staining

Cell lysates and their protein concentration were prepared and assessed as previously described [50, 51] Frozen cell pellets were dissolved in hot lysis buffer (1% SDS, 100 mM Tris-HCI), and sonicated. Five percent of 2-mercaptoethanol was added. Samples were then dissolved in solubilisation buffer (8 M urea, 2.5 M thiourea, 4% 3-[(3-cholamidopropyl) dimethylammonio]-1-propanesuflate, 50 mM DTT, 24mM spermine tetrachloride) at room temperature for 1 h. The samples were centrifuged at 12000 x g for 30 min. To concentrate the protein samples, acetone precipitation was used. Once the supernatant was discarded the protein pellets were washed with a mixture of ice cold methanol-chloroform, and re-suspended in isoelectric focusing buffer (IEF) (8 M urea, 2.5 M thiourea, 4% 3-[(3-cholamidopropyl) dimethylammonio]-1-propanesuflate).

For first dimensional isoelectric focusing buffer (IEF), immobilized pH Gradient (IPG) dry strip gel pH 3-10NL (Bio-Rad), was rehydrated for 24 h at room temperature with a mixture containing 55 µg of protein lysate dissolved in IEF buffer. The IPG strips were then focused using an IPGphor system (BioRad, USA). Rehydrated filter wicks were placed between the electrodes and the IPG strip. Separation of proteins in the first dimension was carried out at 20°C for 24 h, following the manufacturer's protocol. After IEF, strips were rinsed in deionized water, and stored at -80°C until the second dimensional IEF.

For second dimensional IEF, IPG strips were incubated in equilibrium buffer 1 [6 M/I urea, 20% w/v glycerol, 4% w/vSDS, 0.375 M/I Tris-HCL (pH 8.8), 5% 2-mercaptoethanol] for 15 min at room temperature. After that the strips were moved to equilibrium buffer 2 [6 M/I urea, 20% w/v glycerol, 4% w/v SDS, 0.375 M/I Tris-HCL (pH 8.8), 2.5% 2-mercaptoethanol] and incubated for another 15 min at room temperature. Strips were then rinsed in electrophoresis buffer (60.57 g Tris base, 288.27 g glycine, 20 g SDS, double distilled H₂O up to 20 L). The second dimensional analysis was carried out using a Criterion gel system (Bio-Rad). Each strip was placed into the well of a 12% SDS-PAGE gel (Bio-Rad), and sealed with agarose solution. Electrophoresis was carried out at 50 V for 30 min, and following this at 150 V for 4-5 h. The gel was then fixed overnight in fixing solution (50% methanol, 5% acetic acid, 45% water), and stained using the silver staining protocol [52, 53].

### Spot analysis

The gels were scanned and analyzed using SameSpots analysis software (Nonlinear dynamics Ltd). Digitized images from 12 silver stained gels were analyzed for spot detection and quantification. Image analysis included spot detection, editing, background subtraction and spot matching. A master image was then created, and all spots in the other gels were matched to this master image both manually and digitally. The size of a protein, which approximates the volume of the spot, was calculated by using the software. Spots identified as differentially present were excised and analyzed using mass spectrometry (MS) analysis.

### Mass spectrometry and bioinformatics

Identified spots were manually excised from the 2-DE gels using a disposable sterile scalpel spot cutter and washed in molecular biology grade water. Each spot was then subjected to in gel digestion alkylation, along with tryptic digestion so as to yield peptide fragments for MS analysis following previously described protocols [54].

Peptide digests were subjected to MS analysis at the sub-picomole level through the use of a matrix assisted laser desorption ionization-time of flight (MALDI-TOF MS) so as to generate peptide mass fingerprinting (PMF). The MALDI-TOF analysis was carried out using a Bruker Daltonics Reflex IV instrument using a linear mode with a laser power attenuation setting at 30, as described previously [55]. Once the data were examined and calibrated using the program M/Z, the results were transferred to the MASCOT peptide search engine (Matrix Science Limited) for protein identification.

### Differential Spot Expression Visualization

The expression data were pre-processed by mean-centering (by division and medians), and over median normalized data. A heatmap was generated via hierarchical clustering for examining protein expression and treatment (http://cran.r-project.org). The generation of a heatmap for median centered protein expression data with horizontal hierarchical clustering of different proteins utilized the median linkage agglomeration method and vertical hierarchical clustering of treatment conditions, using complete linkage agglomeration. A correlation distance metric was used for clustering data.

Fold change of PIF treated vs. control and P4 treated vs control was applied on the raw expression data. (The spot volume data used followed the assumption that spot volume correlates and is function of the protein expression level.) The expression ratios and the corresponding p-values obtained from the statistical tests applied over the differential expression data were imported into EGAN (http://akt.ucsf.edu/EGAN/). The proteins (Hugo Symnol represented) that mapped to the Entrez Genes reference were subjected to further analysis and network visualization. Selected proteins were used for related associated nodes enrichment, thus obtaining the linked annotation; GO, REACTOME, NCI-Harvard Pathways and others.

### ELISA for cytokine determination

In preliminary studies the cytokine secretion profiles of JEG-3 and ACH-P3 trophoblastic cells were determined. Subsequently, the effect of PIF and P4 on JEG-3 cell cytokines was examined, comparing against unstimulated cell culture supernatant. At the end of the experiments the supernatant was collected and analyzed for cytokine levels. The concentrations of tumor necrosis factor alpha (TNF-α), interleukin (IL)-1, IL-8, IL-10, interferon gamma (IFN-γ), transforming growth factor beta 1 (TGF-β1) and granulocyte macrophage colony-stimulating factor (GM-CSF) were measured using ELISA kits (eBioscience), following the manufacturer's instructions.

### Statistical analysis

Statistical analysis was performed using the software SigmaPlot version 12.5 (Systat Software Inc., India). The descriptive statistics and normality test were done as a first step. If the population followed a normal distribution (Gauss' Bell), Student's t-test was used to compare the different groups (treated vs. non-treated, surface vs. intracellular antigen expression, etc.). If the population did not follow a normal distribution Mann-Whitney U test was used. The multiparametric comparison was performed by ANOVA. The p<0.05 value is considered significant.

### Comparison between JEG-3 and ACH-3P

*Cell lines culture.* Human trophoblast-derived cell lines JEG-3 and ACH-3P were used for this study. They were cultured in a fully humidified atmosphere at 5% CO₂ and 37°C. The JEG-3 cell line was maintained in Dulbecco's modified Eagle's medium (DMEM) Ham's F-12, supplemented with 10% (v/v) of fetal calf serum (FCS). The new hybrid cell line ACH-3P, derived from the human first trimester trophoblast, was maintained in Ham's F-12 with Glutamax^{®} medium supplemented with 10% (v/v) FCS. All media were from (PAA-GE Healthcare) while sera was (Hyclone).

Cells were detached at an approximately 70-80% confluence. Briefly, once the culture media was removed, cells were washed twice with room temperature PBS following Accutase^{™} treatment for 2-8 minutes at 37°C. Accutase was inhibited with cold culture media, and detached cells were centrifuged at 3,000 rpm for 5 minutes. The supernatant was aspirated off and cell pellet re-suspended in fresh media. The seeding density was between 1.0×10⁵ to 1.5×10⁵ cells/ml. Both JEG-3 or ACH-3P trophoblastic cell lines showed similar growth pattern with population doubling times of 24 hours and 20 hours, respectively. After seeding, both cell lines entered the exponential growth phase on day three and reached the stationary phase around day seven. The growth pattern of JEG-3 and ACH-3P cells were similar, both grew in monolayers adhered to the culture flask. The JEG-3 and ACH-3P cells were studied for the typical trophoblast antigen expression panel: cytokeratin-7+, HLA-DR- and vimentin-. Neither expressed detectable levels of vimentin and HLA class II molecules while they were strongly positive for cytokeratin-7. The two trophoblast subpopulations comprising the ACH-3P cell line were separated using fluorescence-activated cell sorting (FACS) analysis based on their HLA-G expression. While the EVT is known to express HLA-G, the villous syncytiotrophoblast does not. **Fig 1A****,B and Table 1** show the results of the cell sort using the MEM-G/9 antibody. In the figure the histogram with a double peak to the left of the graph is representative of the original ACH-3P cell line tagged with the MEM-G/9; this cell line displays two peaks, representative of two subpopulations, whilst the single-peaked histogram is representative of the isotype control. Using the BDFacs Aria, the cell line was sorted based on its HLA-G expression; the double peaked histogram is the original ACH-3P cell line, while the single peaked histogram is representative of the EVT subpopulation; the small overlap between the two histograms is believed to be due to the low proportion of other subpopulations that are present in this polyclonal cell line.

### Results

### Comparison between JEG-3 and ACH-P

*HLA class I expression: Western blot analysis.* To determine which trophoblast cells are most suitable for analyzing the effect of PIF and P4 first the relative expression of global HLA class I, intra and extracellular, was explored by using Western blot. In all experiments 40 µg protein was loaded. HLA was analyzed using semi-quantitative estimation based on the thickness and brightness of the observed band vs. standard. Data showed that HLA-G, HLA-E and HLA-C expression were higher in JEG-3 cells as compared with ACH-3P cells. The expression of HLA-F expression was low in both cells. However, HLA-A or -B was not detected in any cell line.

*HLA class I expression: Flow cytometry analysis.* Intracellular and surface antigen expression was also studied by flow cytometry. Both cells lines expressed intracellular HLA-C, -E, -F and -G. However, HLA I expression in JEG-3 cells (**FIG.** 1A) was significantly higher compared with ACH-3P cells (**FIG.** 1B) At the cell surface, the expression pattern followed a similar behavior. HLA-C, -E and -G were detected while, HLA-F was not detected in either cell line. The expression of HLA-C and HLA-G between JEG-3 and ACH-3P cells was significant (P ≤ 0.05). Confirming Western blot data, JEG-3 cells showed a higher HLA class I molecules expression than ACH-3P.

*HLA class I expression: Surface antigen quantification.* The relative number of HLA class I molecules in JEG-3 and ACH-3P cells was assessed using a quantitative immunofluorescence assay. **(Table 2).** Highly significant differences in HLA-E (P ≤ 0.01) and HLA-G (P ≤ 0.05) between both cell lines were observed. HLA-F intracellular and surface expression was higher in JEG-3 cells. Importantly, HLA-C was only expressed in JEG-3 cells (P ≤ 0.01). The significant HLA-G expression in JEG-3 may be due to its clonal origin, while ACH-3P cells derive from cell two populations, where the extravillous cells (HLA-G+ cells) contribute only 40% to the ACH-3P cell line.¹ Low HLA-F expression² on the cell surface³ was found especially in ACH-3P cells. The low expression requires cell-cell or receptor/soluble ligand signaling, or due to limited number of peptides that bind to the respective HLA-F groove in culture. Compared to the ACH-3P, the JEG-3 cell line displayed higher levels of HLA I expression.

*JEG-3 and ACH-3P cells: cytokine profile.* Cytokines known to be expressed by the placenta were studied. Both ACH-3P and JEG-3 cell lines secreted IL-1β, IL-8, GM-CSF and TGF-β1 into the media. While TNF-α and IFN-γ levels were below the threshold considered negative. **(Table 3).** IL-1β cytokine level secreted by JEG-3 cells was twofold higher as compared with ACH-3P cells therefore early pregnancy is predominantly a pro-inflammatory state.^{4,5} If we consider that TGF-β inhibits the proliferation of T-cells, IL1-induced proliferation and activation of T-helper and cytotoxic cells combined with elevated HLA-G expression indicates that JEG-3 cells mimic accurately the immune-regulatory uterine environment.⁶ Consequently, further investigations were carried out only by using the JEG-3 cells. The next experiments aimed to determine the most effective concentration of progesterone on this cell line in view of comparing it with PIF effect.

### The effects of PIF on HLA class I molecules

*PIF's effect on JEG-3 cells: HLA-G expression:* PIF promotes trophoblast invasion and protects against apoptosis [46]. Whether PIF is involved in regulating HLA class I antigen expression to promote maternal tolerance is not known. The viable embryo secretes both PIF and soluble HLA-G into the culture media [16, 48]. PIF promotes invasion, regulates the immune response and has anti-apoptotic effects. Since PIF is expressed by the trophoblast shortly post-implantation its potential regulatory role on local HLA class I molecules was examined. This is relevant for understanding the development of tolerance from the earliest stages of gestation.

The effect of synthetic PIF (1--1000 nM) on HLA-G expression in JEG-3 cells was determined by using serum free media. JEG-3 cells are here shown to be superior to ACH-3P and therefore were used for all experiments in the current study (Supplemental Material). This was substantiated by the following observations. 1. ACH-3P cells are comprised of two populations of cells, however only in 40% of the cells HLA-G+ is expressed (Figure 1a, 1b); 2. The expression of HLA-C, -E, - F, G is higher in JEG-3 cells than in ACH-3P cells. (Table 2); 3. HLA-C was only expressed in JEG-3 cells. 4. IL-1α, IL-8, IL-10 and TGF-β1 levels were higher in JEG-3 cells than in ACH-3P cells. (Table 3).

To validate measurements of the mean fluorescence intensity (MFI) a calibration curve was constructed, plotting against antigen binding capacity. For validating
the MFI binding target data, a double logarithmic graph was used to provide the best fit, R²=0.99 (Fig. 2). This methodology was used in all subsequent experiments.

PIF was found to increase HLA-G levels in JEG-3 cells in a dose-dependent manner (Fig. 3A). PIF promoted HLA-G expression at all concentrations tested (up to 1uM). The highest effect was noted testing PIF at 200 nM concentration. In the time course experiment, 200 nM PIF added for 24 hours in culture induced the most pronounced increase in HLA-G expression (28 fold) (Fig. 3B). This supports the hypothesis that PIF's role is to be a driver of tolerance.

### The effect of PIF on HLA class I antigen expression by JEG-3 cells

HLA molecules are expressed both intracellularly and on the cell surface. The effect of PIF on intracellular HLA-G, -E, -F and -C expression by JEG-3 cells was determined (Fig. 3C). PIF increased the expression of all tested HLAs. HLA-G exhibited the highest level of expression followed by HLA-E. The increase in intracellular expression was coupled with an increase of cell surface HLA-G and HLA-E (p< 0.01) (Fig. 3D). HLA-C expression also increased on the cell surface (p< 0.05). However, PIF only minimally affected surface HLA-F expression. The time-dependent increase in HLA-F and HLA-E expression was confirmed using Western blotting, which demonstrated that the maximal increase was already attained at 24 h of culture (Fig. 4A-D) Thus PIF activates class I HLA molecules.

### PIF and P4 have a differential effect on HLA class I expression by JEG-3 cells

JEG-3 cells cultured with 1 µg/ml P4 for 24 hours produced increased levels of HLA-G, -E, -F and -C, with HLA-G expression being the most pronounced (Fig. 5A). Intracellular and surface measurements demonstrated significant P4-induced HLA expression (Fig. 5B, 5C). Significant differences between intra- and extracellular HLA expression were noted. The highest increase was noted with HLA-G, followed by HLA-E and a mild effect was observed on HLA-C expression (Fig. 5C). This confirmed that the 1 µg/ml dose at 24 h was most effective. This observation permitted comparison of the effect of PIF with the most effective concentration of P4.

The effect of 200 nM PIF was compared with 1 µg/ml P4 after incubation for 24 h. Western blot analysis showed that, compared to P4, PIF induced increase was more pronounced in both HLA-G and HLA-E expression (p<0.01). Basal expression of HLA-C and HLA-F was low. However, PIF induced a higher expression of both ligands as compared to P4 (Fig. 6A). Flow cytometry analysis and the antigen quantification data confirmed the Western blot data. The PIF-induced increase in both intracellular and surface HLA expression was also more pronounced compared to the effect of P4 (Fig. 6B). The most pronounced effect noted was on HLA-G and HLA-E expression (Fig. 6C). The increase in HLA expression at the intracellular level translated to an increase at the surface level, with PIF-treated cells displaying high MFI and surface antigens in comparison to P4-treated cells.

### PIF promotes P4 secretion

PIF promoted the expression of IL-17F, a pro-inflammatory cytokine in the trophoblast that plays an important role in angiogenesis [46]. Using a dose-dependent design, 1-100 ng/ml IL-17 promoted P4 secretion by JEG-3 cells, as determined by ELISA. The maximal effect was noted at 10 ng/ml IL-17 (p<0.01) (data not shown). Both 200 nM PIF and 10 ng/ml IL-17, after 6-72 h of culture, increased P4 secretion by JEG-3 cells, observed at 6 and 24 h, respectively. (Fig. 6D). Thus, PIF directly promotes P4 secretion through an IL-17-dependent pathway.

### Imaging analysis confirms that PIF promotes HLA-G expression

Following culture of JEG-3 cells until confluence, the cells were washed and cultured in serum-free media in the presence of 200 nM PIF. The effect of PIF on HLA-G expression was examined after 24 h of incubation. It was found that the expression of HLA-G in PIF-treated JEG-3 cells (anti-HLA-G mAb stained) increased (Fig. 7). Further, DAPI staining revealed an intact nuclear structure. This provided additional evidence that PIF promotes HLA-G expression.

### The effect of PIF and P4 on protein levels in JEG-3 cells

To determine whether PIF also regulates the placental proteome, the effect of 200 nM PIF and 1 µg/ml P4 on protein expression by JEG-3 cells was analyzed by 2-DE gel electrophoresis Proteins were separated in the first dimension based on their pl, which ranged from 3-10, and, based on their size, by SDS-PAGE in the second dimension. The analysis of the master gel detected 22 spots showing differences between treatment and control cells.

A comparison of the average spot volume (mean ± SD, 4 samples/group) of the 22 spots was carried out. Significant differences between PIF-treated and untreated JEG-3 cells were found. Fourteen spots from the PIF-treated group decreased, whereas nine significantly increased. Comparison of P4-treated cells with PIF's effect was also significant. The 22 protein spots identified by mass spectrometry analysis were validated by using a ProFound probability score of 0.99-1.00. Since not all spots could be confirmed further, bioinformatic and pathway analysis was performed only on the 19 validated proteins **(Table I**).

### Bioinformatic analysis of proteomic data

Fig. 8 shows the heat map visualized effect of PIF on the placental proteome. GO analysis of the biological functions of the 19 significant proteins (Fig. 2) showed that they are mainly related, in terms of response to stimulus, regulation of biological process, multicellular organismal process, cell communication, developmental process and cell proliferation. The proteomic data confirmed that PIF-induced an increase in HLA-G, validating the antibody based data. FOXP3+, an activation marker of pro-tolerance Tregs, also increased. Remarkably, PIF increased the P4 receptor (PRGR, 2.5 fold) protein coupled with the increase in P4 secretion (see above) which potentiates P4 action in trophoblasts. In addition, PIF increased levels of pro-coagulation factors DCBD2 V/VIII-Homology domain, TTP, Von Willebrand Factor-Cleaving Protease and CD59, involved in complement regulation. ATP5B, which is H+ transporting, mitochondrial F1 complex and ZSC21, a potent transcriptional activator, and CLIC3 and HBB, were overexpressed as well.

In contrast, proteins involved in oxidative stress and protein misfolding were reduced, including PDIA3, protein disulfide isomerase A3, PRDX2 peroxiredoxin-2, HS74L, (HSP70-4)- and HSP71 (HSP70-8). These are prime PIF targets that are also regulated *in vivo* [26, 33, 37]. Mitogenic effects of EGF were reduced by lowering its receptor levels, which is likely to support trophoblastic differentiation. Also, EPCAM, calcium-independent cell adhesion and LMNA and EF2 proteins were decreased, which catalyze the GTP-dependent ribosomal translocation step. Metabolic KPYM pyruvate kinase and G3P glycerol-3-phosphate dehydrogenase-1 proteins were decreased as well. Thus PIF, in contrast to P4, has a dual regulatory and protective action.

### Hierarchical clustering analysis

The detected differentially expressed proteins were analyzed, using hierarchical clustering of normalized protein expression and treatment conditions (PIF vs. P4) and Exploratory Gene Association Networks (EGAN) analysis to explore the involvement of PIF in differentially expressed networks. Hierarchical clustering revealed that PIF reciprocally down-regulated half the proteins compared to control cells. It also triggered up-regulation in nine cases, in contrast to P4. The top up-regulated cluster was ranging from HLAG to FOXP3. On the other hand, P4 up-regulated only five of 19 differentially expressed proteins, with the remaining down-regulated (Fig. 9).

When EGAN was applied, the CLIC3 protein differed only in terms of its expression mode, since PIF up-regulated while P4 down-regulated the same protein. This protein is a voltage-dependent chloride ion channel and participates in cell membrane potential stabilization. The increase in CLIC3 may protect against intrauterine growth retardation and preeclampsia, since PIF expression is low in this condition [46, 58]. EGAN performs a hypergeometric enrichment of linked annotations to the gene-related nodes, and in the case of PIF treatment, FOXP3 is up-regulated and linked to NFAT transcriptional control, while PDIA3 is linked to oxidative stress and calcium signaling, which is decreased. Similarly, several pathways and processes are affected by EGFR down-regulation, with few selected and represented, for example for signal transduction mediated by PTPB1, by IL1 and the related protein-protein interaction of EGFR with LMNA (increased Apoptosis related Caspases). PDIA3 (increased calreticulin based calcium signaling) and ATPB5 (decreased MHC protein binding) are also targeted by PIF [26]. Thus proteome data confirms PIF's promoting effect on HLA-G and the P4 receptor while reducing oxidative stress and protein misfolding.

### The effect of PIF and P4 on the cytokine profile of JEG-3 cells

Since PIF was effective in promoting HLA expression, particularly that of HLA-G, we examined whether such stimulation also extends to an effect on the production of cytokines needed to interact with the maternal milieu. The effect of 200 nM PIF on JEG-3 cell cytokine secretion was compared with 1 µg/ml P4 using serum-free media for 24 hours. PIF increased production of both clinically relevant Th1 and Th2 type cytokines (IL-10, IL-1β, IL-8, GM-CSF and TGF-β1). In contrast, P4 only increased IL-10 secretion **(Table 2).** The results show that PIF induces a balanced secretion of cytokines, promoting tolerance whilst sustaining anti-pathogen protection. However, the effect of P4 is limited.

### PIF synergy with P4 on HLA-G expression

Since each ligand alone led to increased HLA-G expression, it was important to determine whether the effect could be synergized. Testing with maximally effective ligands in combination showed that the effect can be further enhanced over the individual stimulants. This supports the view that such coordinated action could support embryo tolerance (Figs. 10 A-C).

### PIF synergy with Dexamethasone on HLA-G expression

Dexamethasone is a steroid that is used to mature the fetal lung in preparation for labor that occurs prematurely. PIF targets Kv1.3b channels which are the binding site of dexamethasone (Dexa). In view that Dexa is shown herein to promote HLA-G acting as an immune regulatory agent we tested whether the effect can be synergized with PIF. The data showed that PIF effect can be amplified when combined with Dexa evidenced by increased HLA-G expression. (Figure 10 A,B,D) This implies that action on the trophoblast could improve fetal wellbeing when combined with the steroid.

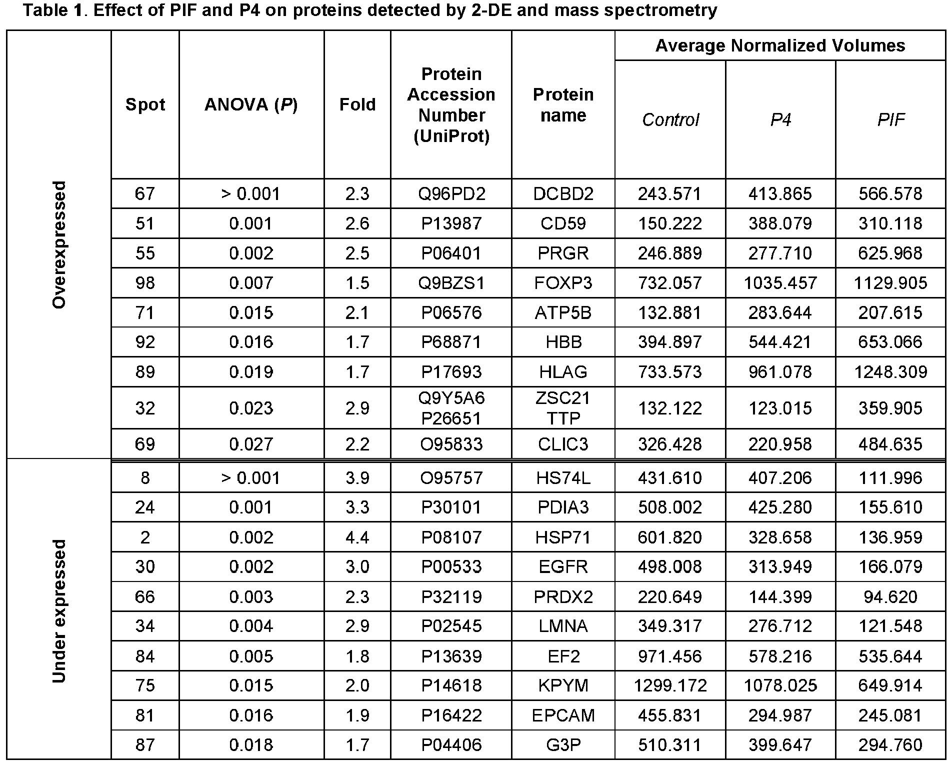

**Table 2. Effect of PIF and P4 on cytokine secretion by JEG-3 cells**

| | **JEG-3** | **JEG-3 (P4 1µg/ml)** | **JEG-3 (PIF 200nM)** |
|---|---|---|---|
| IL-1β | 289 ± 33.5 | 334 ± 27.7 | 436 ± 46.3* |
| IL-8 | 601.5 ± 43.7 | 685 ± 58.2 | 754 ± 50.7* |
| IL-10 | < 0.1 | 113 ± 15.6* | 201 ± 23.1* |
| GM-CSF | 226.2 ± 23.7 | 310 ± 36.3 | 398 ± 32.4* |
| TNF-α | < 1 | 1 | N.D. |
| IFN-γ | < 10 | 10 | N.D. |
| TGF-β1 | 321 ± 29.1 | 398 ± 32.4 | 462 ± 39.2* |

All results are given in pg/ml. Results shown are mean ± SEM from six independent experiments *P<0.05.

**Table 3. Comparison of JEG-3 cells expression of HLA-I molecules as compared with ACH-3P**

| | **JEG-3** | **ACH-3P** |
|---|---|---|
| HLA-G | 41 562 ± 1943 | 27 619 ± 1723 P<0.05 |
| HLA-E | 12 985 ± 1542 | 10 321 ± 1700 |
| HLA-F | | |
| HLA-C | 4824 ± 1230 | 3233 ± 1079 P<0.05 |

**Table 4. Steroid doses**

| **Steroid** | **Normal Dose** |
|---|---|
| Prednisone | 5-80 mg/day (40-60 mg common) |
| Betamethasone | 0.6-9.6 mg/day |
| Dexamethasone | 0.75-12 mg/day |
| Methylprednisolone | 4-64 mg/day |
| Triamcinolone | 4-64 mg/day |
| Prednisolone | 5-80 mg/day |
| Hydrocortisone | 20-320 mg/day |
| Cortisone | 25-400 mg/day |
| Deflazacort | 7.5-120 mg/day |
| Fludrocortisone | 0.05-0.2 mg/day |

### Discussion

Consistent with a prior study, the expression levels of HLAs and secreted cytokines in JEG-3 cells were superior to those in ACH-3P cells and the JEG-3 cell line was herein used [14]. We demonstrate that in JEG-3 cells PIF up-regulates the pivotal pro-tolerance molecule HLA-G and the associated class I molecules HLA-C, -E and -F. Proteome analysis confirmed up-regulation of HLA-G, pro-tolerance Tregs (FoxP3+), coagulation factors and complement, and the reduced expression of proteins involved in oxidative stress and protein misfolding. Reduced EGF receptor expression may limit the proliferative potential of the trophoblast. PIF's promoting effect on all of the HLAs and cytokines studied was more pronounced than P4's. Evidence for PIF-induced amplification of endogenous P4 action is shown by increased P4 secretion coupled with increased steroid receptor protein levels.

HLA and cytokine expression in JEG-3 cells creates a balance between a pro-inflammatory and anti-inflammatory environment. PIF increased HLA-G, HLA-E and HLA-C expression both intracellularly and at the cell surface, as evidenced by complementary methods of analysis. HLA-G up-regulation was confirmed by using HLA-G imaging and proteome analysis. Such robust multifaceted analyses provide support for PIF's important local regulatory role. The PIF-induced increase in HLA-E expression was of similar magnitude to HLA-G. The role of HLA-E at the feto-maternal interface can be complementary to HLA-G. Both can be co-expressed and induced by P4 in the trophectoderm of preimplantation embryos [15, 16]. Recognizing P4's important role in HLA regulation, a direct comparison with that of PIF shown confirmed PIF's higher efficacy in all side-by-side experiments. Both PIF and P4 increased intracellular and cell surface expression of HLA-G, -E, -F, and -C, but did not affect their relative proportion. This may be critical for a successful pregnancy; while not all HLA-G molecules need to form a heterodimer with HLA-E, the lack of heterodimer formation of HLA-E, combined with HLA-G, can lead to implantation failure [59]. Moreover, for its surface expression, HLA-E must interact with and be stabilized by a signal peptide, usually derived from other HLA class I alleles. In trophoblasts, it is derived from HLA-G and HLA-C [60]. HLA-E can also interact with uNK cells through the CD49/NKG2 receptor [61]. Together, HLA-G and HLA-E may inhibit NK cells cytotoxicity by interacting with the killer-cell immunoglobulin-like receptors (KIR)2DL4 and CD94/NKG2, respectively. Trophoblast protection from cell lysis is also achieved through interaction of HLA-G homodimers with the inhibitory NK receptors ILT2 and ILT4 [15]. Both PIF and P4 increased HLA-C expression, which also targets KIR molecules on uNK cells. KIR molecules regulate trophoblast invasion and uterine spiral artery blood flow in the inter-villous space [62]. Certain maternal KIR/HLA-C combinations can lead to defective trophoblast invasion or to an incomplete transformation of the spiral arteries, ultimately leading to pregnancy complications [59, 62]. The involvement of RAC1 and downstream b-catenin role in effective trophoblast invasion by promoting metalloproteinase 9 (MMP-9) was shown [63]. PIF also promoted MMP9 while reducing the inhibitor TIMP1 and regulating integrins expression [42]. Thus, the low PIF expression in preeclampsia and intrauterine growth retardation may lead to the low local MMP-9 expression [42, 46]. In addition, detailed proteomic analysis of syncytiotrophoblast extracellular vesicles identified differentially expressed proteins in the placenta of patients with preeclampsia among them increased pro-inflammatory S100-A8 as having a major role [64]. Also, it was shown that thrombin is an inducer of FMs-like tyrosine kinase 1 through increased ROS in transformed EVT supporting role in preeclampsia pathogenesis [65]. Importantly, we reported that in human immune cells PIF targets both thrombin and S100-A8, thus through local action PIF could mitigate such pathology [33]. PIF reduced systemic NK cells cytotoxicity and up-regulated local HLA-C expression, suggesting an integrated protective action [37]. PIF and P4 only mildly affected HLA-F expression, confirming previous observations [14]. PIF is expressed in trophoblasts during the earliest phase of gestation and is also present in maternal circulation nine days after insemination. Therefore, both endogenous and exogenous PIF may regulate trophoblastic HLA-G. Collectively, the observed potent PIF-induced up-regulation of HLA in trophoblasts reveals an essential role for PIF in promoting immune tolerance.

Our 2-DE proteome analysis (Figs. 11A-C) demonstrated the PIF-induced increase in P4 receptor levels in JEG-3 cells, coupled with increased P4 secretion [52, 53]. This reveals PIF's important role in endogenous P4 potentiation, which thus may facilitate the steroid's production overtake by the placenta. The stimulatory effect of hCG on the corpus luteum as well as on endogenous (trophoblast) P4 was also reported [66]. The PIF data also confirmed the increase in HLA-G and FOXP3, a marker of Treg activation, serving to amplify the pro-tolerance effect. Circulating Tregs increase prior to implantation in response to the presence of a viable embryo [67]. Effective coagulation control (through increased TTP and DCBD factors) is crucial for placental function, since an altered coagulation cascade can lead to placental abruption. Regulation of complement activation (CD59) prevents membrane attack complex-induced C9 polymerization that promotes adverse pore production on the cell surface [68]. The embryo and early pregnancy trophoblast are highly vulnerable to an oxygen rich environment. Thus, down-regulation of peroxidases and HSPs by PIF may support a protective role, as shown in the embryo and decidua [26, 29, 32, 33, 37, 42]. This protection is amplified by the ATPB protein involved in ATP production, which, together with HBB, further protects against free oxygen and nitrogen radical species. Reduced KPYM levels prevent caspase-independent cell death. Clustering analysis indicated that beyond the central role of HLA-G and FOXP3, PIF also reduced the EGF receptor, which interacts with harmful LMNA and PDIA proteins. This is relevant, since PIF's pro-receptive effect on the decidua was negated by EGF [30]. Hence, the proteome analysis substantiates PIF's multifaceted role in regulating the activity of the trophoblast, acknowledging that it is a transformed cell line.

Both Th1 and Th2 type cytokines were up-regulated by PIF. The increase in IL-10, a prime Th2 cytokine, was induced by both P4 and PIF in JEG-3 cells. IL-10 may be secreted by both Th1 and Th2 type cells; its function is to balance pro- and anti-inflammatory signals [69-71]. IL-10 enhances HLA-G transcription in first trimester human trophoblast cultures [72, 73]. In the endometrium PIF and P4 create a pro-inflammatory milieu by increasing IL-1β, IL-8, GM-CSF and IFN-γ secretion to promote embryo implantation [29, 30, 36]. Elevated TGF-β could promote IL-1-induced T-cell proliferation and trophoblast invasion by up-regulating integrin expression, as shown for PIF in the endometrium, independent of P4. Thus, PIF-induced secretion of diverse cytokines, in contrast to P4, which affected only IL10, supports PIF-induced trophoblast interaction with the maternal milieu.

The data generated when PIF effect was combined with P4 demonstrating synergistic effect on HLA-G support both ligands critical role in placental function in inducing tolerance. Further the synergy identified with Dexa indicates that although the both ligand acts on the same binding site in this instance they act in synergy which supports fetal wellbeing.

The current study is limited, since JEG-3 cytotrophoblastic cells and not primary trophoblastic cells were used. However, this model was validated with respect to the administration of P4, which was shown to be of physiologic value. In addition, exogenous PIF was administered. However, importantly, PIF targets the embryo and significant levels are present in the maternal circulation, which support the data generated with exogenous PIF administration [25, 26]. These are in vitro observations and therefore data using PIF in an in vivo setting would help to substantiate its potential clinical utility in treatment of pregnancy disorders. Recent data showed that in an immune intact murine model PIF administration reduces fetal death due to LPS administration [74]. Spontaneous pregnancy loss was also lowered.

In conclusion, PIF promotes the expression of HLA-G, -C, -E and mildly -F which are critical for immunological tolerance in JEG-3 choriocarcinoma cells. The effect of PIF was found to be superior to that of P4 in terms of promoting expression of the HLAs and cytokines studied. By promoting P4 secretion and receptor expression, PIF potentiates the endogenous steroid's effect. PIF regulates the trophoblast proteome, promotes tolerance by increasing HLA-G and FoxP3+ levels and affects coagulation and complement, while it reduces the level of proteins involved in oxidative stress and protein misfolding. PIF-induced increase in Th1/Th2 cytokine secretion favors trophoblast/maternal signaling. PIF successfully completed a Phase I FDA designated Fast-Track clinical trial for an autoimmune disease (Clinicaltrials.gov NCT02239562). Current data support comparable testing in early pregnancy disorders as well.

The current data is also in line with our observation where PIF was shown to promote allogeneic bone marrow transplantation (Azar et al, 2013) as well as in allo-ovarian primate transplantation (Feichtinger et al. 2017). In that context, the role of HLA-G expression following transplantation was examined. The data showed that following heart transplantation both serum and cardiac biopsies showed decreased rejection when HLA-G was expressed. Similar observations were in kidney transplants where the reduction of the titer led to better acceptance. Moreover, the higher titer of soluble HLA-G in the circulation the better acceptance of the bone marrow transplant and reduction in acute GVHD occurrence.
1. Zarnani AH, et al. Microenvironment of the feto-maternal interface protects the semiallogenic fetus through its immunomodulatory activity on dendritic cells. Fertil Steril 2008;90:781-788.
2. Paidas MJ, et al. Pregnancy and Multiple Sclerosis (MS): A Beneficial Association. Possible therapeutic application of embryo-specific Pre-implantation Factor (PIF*). Am J Reprod Immunol 2012;68:456-464.
3. Barnea ER, et al. Reproduction and autoimmune disease: important translational implications from embryo-maternal interaction. Immunotherapy 2013;5:769-780.
4. Tincani A, et al. Pregnancy, lupus and antiphospholipid syndrome (Hughes syndrome). Lupus 2006;15:156-160.
5. Thellin O, et al. Tolerance to the foeto-placental 'graft': ten ways to support a child for nine months. Curr Opin Immunol 2000;12:731-737.
6. Beagley KW, Gockel CM. Regulation of innate and adaptive immunity by the female sex hormones oestradiol and progesterone. FEMS Immunol Med Microbiol 2003;38:13-22.
7. Barnea ER. Signaling between Embryo and Mother in Early Pregnancy: Basis for Development of Tolerance; in Carp HJA (ed) Recurrent Pregnancy Loss - Causes, Controversies, and Treatment. Boca Raton, Florida, CRC Press, 2014, pp 17-28.
8. Genbacev O, et al. Establishment of human trophoblast progenitor cell lines from the chorion. Stem Cells 2011;29:1427-1436.
9. Hanna J, et al. Decidual NK cells regulate key developmental processes at the human fetal-maternal interface. Nat Med 2006;12:1065-1074.
10. Gregori S, et al. HLA-G Orchestrates the Early Interaction of Human Trophoblasts with the Maternal Niche. Frontiers in immunology 2015;6:128.
11. Lynge Nilsson L, et al. Controlling the Immunological Crosstalk during Conception and Pregnancy: HLA-G in Reproduction. Frontiers in immunology 2014;5:198.
12. Moffett A, Loke C. Immunology of placentation in eutherian mammals. Nat Rev Immunol 2006;6:584-594.
13. Barnea ER, et al. The Effect of Progesterone upon First Trimester Trophoblastic Cell Differentiation and Human Chorionic Gonadotropin Secretion. Hum Reprod 1991;6:905-909.
14. Yie SM, et al. Progesterone regulates HLA-G gene expression through a novel progesterone response element. Hum Reprod 2006;21:2538-2544.
15. Jabeen A, et al. Quantified colocalization reveals heterotypic histocompatibility class I antigen associations on trophoblast cell membranes: relevance for human pregnancy. Biol Reprod 2013;89:94.
16. Shaikly V, et al. Cell bio-imaging reveals co-expression of HLA-G and HLA-E in human preimplantation embryos. Reprod Biomed Online 2010;20:223-233.
17. Spencer PS, et al. Key cellular components and interactive histocompatibility molecules regulating tolerance to the fetal allograft. Am J Reprod Immunol 2012;68:95-99.
18. Barnea ER, et al. Progesterone, Estradiol, and Alpha-Human Chorionic Gonadotropin Secretion in Patients with Ectopic Pregnancy. J Clin Endocrinol Metab 1986;62:529-531.
19. Morel Y, et al. Evolution of steroids during pregnancy: Maternal, placental and fetal synthesis. Ann Endocrinol (Paris) 2016;77:82-89.
20. Barnea ER. Applying Embryo-Derived Immune Tolerance to the Treatment of Immune Disorders. Annals of the New York Academy of Sciences 2007;1110:602-618.
21. Barnea ER. Insight into early pregnancy events: the emerging role of the embryo. Am J Reprod Immunol 2004;51:319-322.
22. Than NG, et al. Embryo-Placento-Maternal Interaction and Biomarkers: From Diagnosis to Therapy - A Workshop Report; Trophoblast Research, 2007, vol 21, pp S1-S4.
23. Stamatkin CW, et al. PreImplantation Factor (PIF) correlates with early mammalian embryo development-bovine and murine models. Reprod Biol Endocrinol 2011;9:63.
24. Stamatkin CW, et al. Preimplantation factor negates embryo toxicity and promotes embryo development in culture. Reprod Biomed Online 2011;23:517-524.
25. Ramu S, et al. PreImplantation factor (PIF) detection in maternal circulation in early pregnancy correlates with live birth (bovine model). Reprod Biol Endocrinol 2013;11:105.
26. Roussev RG, et al. Preimplantation factor inhibits circulating natural killer cell cytotoxicity and reduces CD69 expression: implications for recurrent pregnancy loss therapy. Reprod Biomed Online 2013;26:79-87.
27. Barnea ER, et al. Insight into PreImplantation Factor (PIF*) mechanism for embryo protection and development: target oxidative stress and protein misfolding (PDI and HSP) through essential RIKP binding site. PLoS One 2014;9:e100263.
28. Paidas MJ, et al. A genomic and proteomic investigation of the impact of preimplantation factor on human decidual cells. Am J Obstet Gynecol 2010;202:459 e451-458.
29. Barnea ER, et al. Preimplantation factor (PIF) promoting role in embryo implantation: increases endometrial integrin-alpha2beta3, amphiregulin and epiregulin while reducing betacellulin expression via MAPK in decidua. Reprod Biol Endocrinol 2012;10:50.
30. Duzyj CM, et al. PreImplantation Factor (PIF*) promotes embryotrophic and neuroprotective decidual genes: effect negated by epidermal growth factor. Journal of Neurodevelopmental Disorders 2014;6:36.
31. Barnea ER, et al. PreImplantation Factor (PIF) orchestrates systemic antiinflammatory response by immune cells: effect on peripheral blood mononuclear cells. Am J Obstet Gynecol 2012;207:313 e311-311.
32. Barnea ER, et al. Blocks mitogen-activated PBMCs proliferation, promotes TH2/TH1 bias, independent of Ca(2+). Immunobiology 2015;220:865-875.
33. Barnea ER, et al. PreImplantation factor (PIF*) regulates systemic immunity and targets protective regulatory and cytoskeleton proteins. Immunobiology 2016;221:778-793.
34. Chen YC, et al. PreImplantation factor prevents atherosclerosis via its immunomodulatory effects without affecting serum lipids. Thromb Haemost 2016;115(5):1010-24
35. Azar Y, et al. Preimplantation factor reduces graft-versus-host disease by regulating immune response and lowering oxidative stress (murine model). Biol Blood Marrow Transplant 2013;19:519-528.
36. Shainer R, et al. PreImplantation factor (PIF) therapy provides comprehensive protection against radiation induced pathologies. Oncotarget. 2016 Sep 13;7(37):58975-58994
37. Weiss L, et al. Preimplantation factor (PIF) analog prevents type I diabetes mellitus (TIDM) development by preserving pancreatic function in NOD mice. Endocrine 2011;40:41-54.
38. Weiss L, et al. Preimplantation factor (PIF*) reverses neuroinflammation while promoting neural repair in EAE model. J Neurol Sci 2012;312:146-157.
39. Shainer R, et al. Immune Regulation and Oxidative Stress Reduction by Preimplantation Factor following Syngeneic or Allogeneic Bone Marrow Transplantation. Conference Papers in Medicine 2013;2013:1-8.
40. Mueller M, et al. PreImplantation factor promotes neuroprotection by targeting microRNA let-7. Proc Natl Acad Sci U S A 2014;111:13882-13887.
41. Mueller M, et al. PreImplantation Factor bolsters neuroprotection via modulating Protein Kinase A and Protein Kinase C signaling. Cell death and differentiation 2015;22:2078-2086.
42. Moindjie H, et al. Preimplantation factor (PIF) promotes human trophoblast invasion. Biol Reprod 2014;91:118.
43. Barnea ER, et al. PreImplantation Factor (PIF*) Endogenously Prevents Preeclampsia: Promotes Trophoblast Invasion and Reduces Oxidative Stress. J Reprod Immunology 2015;doi: 10.1016/j.jri.2015.06.002
44. Duzyj CM, et al. Preimplantation factor promotes first trimester trophoblast invasion. Am J Obstet Gynecol 2010;203:402 e401-404.
45. Tillburgs, T. Human HLA-G+ extravillous trophoblasts: Immune-activating cells that interact with decidual leukocytes. Proc Natl Acad Sci U S A. 2015 Jun 9;112(23):7219-24.
46. Moindjie H, et al. PreImplantation Factor is an anti-apoptotic effector in human trophoblasts involving p53 signaling pathway. Cell Death & Disease 2016;7(12)e2504
47. Liu Z, et al. The Roles of p38 MAPK and ERK1/2 in Coplanar Polychlorinated Biphenyls-Induced Apoptosis of Human Extravillous Cytotrophoblast-Derived Transformed Cells. Cell Physiol Biochem. 2015;36(6):2418-32.
48. Shaikly VR, et al. Analysis of HLA-G in maternal plasma, follicular fluid, and preimplantation embryos reveal an asymmetric pattern of expression. J Immunol 2008;180:4330-4337.
49. Menier C, et al. Characterization of monoclonal antibodies recognizing HLA-G or HLA-E: new tools to analyze the expression of nonclassical HLA class I molecules. Hum Immunol 2003;64:315-326.
50. Palmisano GL, et al. HLA-E surface expression is independent of the availability of HLA class I signal sequence-derived peptides in human tumor cell lines. Hum Immunol 2005;66:1-12.
51. Zhao L, et al. Reassessment of HLA-G isoform specificity of MEM-G/9 and 4H84 monoclonal antibodies. Tissue Antigens 2012;80:231-238.
52. Cooper JC, et al. An impaired breeding phenotype in mice with a genetic deletion of beta-2 microglobulin and diminished MHC class I expression: role in reproductive fitness. Biol Reprod 2007;77:274-279.
53. Cherry RJ, et al. Detection of dimers of dimers of human leukocyte antigen (HLA)-DR on the surface of living cells by single-particle fluorescence imaging. The Journal of cell biology 1998;140:71-79.
54. Sasse J, Gallagher SR. Staining proteins in gels. Curr Protoc Immunol 2004; 8:Unit 8 9.
55. Sasse J, Gallagher SR. Staining proteins in gels. Curr Protoc Mol Biol 2003;10:Unit 10 16.
56. Lipton MS, Pasa-Tolic L. Mass spectrometry of proteins and peptides: methods and protocols. Preface. Methods Mol Biol 2009;492:v.
57. Padliya ND, Wood TD. Improved peptide mass fingerprinting matches via optimized sample preparation in MALDI mass spectrometry. Anal Chim Acta 2008;627:162-168.
58. Murthi P, et al. Placental CLIC3 is increased in fetal growth restriction and pre-eclampsia affected human pregnancies. Placenta 2012;33:741-744.
59. Hiby SE, et al. Maternal activating KIRs protect against human reproductive failure mediated by fetal HLA-C2. J Clin Invest 2010;120:4102-4110.
60. Lee N, et al. HLA-E surface expression depends on binding of TAP-dependent peptides derived from certain HLA class I signal sequences. J Immunol 1998;160:4951-4960.
61. Lee N, et al. HLA-E is a major ligand for the natural killer inhibitory receptor CD94/NKG2A. Proc Natl Acad Sci U S A 1998;95:5199-5204.
62. Hiby SE, et al. Combinations of maternal KIR and fetal HLA-C genes influence the risk of preeclampsia and reproductive success. J Exp Med 2004;200:957-965.
63. Fan M, et al. Rac1/β-Catenin Signalling Pathway Contributes to Trophoblast Cell Invasion by Targeting Snail and MMP9. Cell Physiol Biochem. 2016;38(4):1319-32.
64. Li H, et al. Differential Proteomic Analysis of Syncytiotrophoblast Extracellular Vesicles from Early-Onset Severe Preeclampsia, using 8-Plex iTRAQ Labeling Coupled with 2D Nano LC-MS/MS. Cell Physiol Biochem. 2015;36(3):1116-30.
65. Huang QT, et al. Activation of PAR-1/NADPH oxidase/ROS signaling pathways is crucial for the thrombin-induced sFlt-1 production in extravillous trophoblasts: possible involvement in the pathogenesis of preeclampsia. Cell Physiol Biochem.2015;35(4):1654-62.
66. Bhattacharyya S, et al. Antibodies to hCG inhibit progesterone production from human syncytiotrophoblast cells. Placenta 1992;13:135-139.
67. Somerset DA, et al. Normal human pregnancy is associated with an elevation in the immune suppressive CD25+ CD4+ regulatory T-cell subset. Immunology 2004;112:38-43.
68. Zipfel PF, Skerka C. Complement regulators and inhibitory proteins. Nat Rev Immunol 2009;9:729-740.
69. Granot I, et al. Endometrial inflammation and effect on implantation improvement and pregnancy outcome. Reproduction 2012;144:661-668.
70. Challis JR, et al. Inflammation and pregnancy. Reprod Sci 2009;16:206-215.
71. Chaouat G, et al. The emerging role of IL-10 in pregnancy. Am J Reprod Immunol 1996;35:325-329.
72. Moreau P, et al. IL-10 selectively induces HLA-G expression in human trophoblasts and monocytes. Int Immunol 1999;11:803-811.
73. Rizzo R, et al. The HLA-G genotype is associated with IL-10 levels in activated PBMCs. Immunogenetics 2005;57:172-181.
74. Di Simone, N, et al. (in press): Synthetic Prelmplantation Factor (PIF) prevents fetal loss by modulating LPS induced inflammatory response. In press PLoS One 2017.

### Example 2

### Bovine Adrenal Cell Transplantation

### Abstract

The main treatment algorithm for adrenal insufficiency is hormonal replacement. Inadequate hormone substitution often leads to highly undesirable side effects. Adrenal cell transplantation could represent an option, but requires life-long immune suppressive therapy. Using the Prelmplantation Factor (PIF) could solve the problem. This peptide, endogenously secreted by viable embryos, leads to maternal tolerance without immunosuppression and was shown effective for allogeneic cell transplantation. We report here that PIF exerts a dual regulatory effect targeting mostly hyper-activated cells. The PIF regulatory effect was obtained in primary culture of bovine adrenocortical cells (BAC) used for bioartifical adrenal gland development. PIF's effect on BAC depends on initial functional status of BAC and the presence of a specific stimulator - adrenocorticotropic hormone (ACTH). PIF reduces ACTH-stimulated cortisol secretion in high responsive cells (HRC) while not affecting normally responsive cells (NRC), importantly, without affecting basal cortisol secretion in both groups. Reverse transcription real time PCR analysis revealed that PIF regulates cortisol secretion by modulating Steroidogenic Factor 1 (SF1) activator of steroidogenesis and Cytochrome P450 17A1 (CYP17A1) - a steroidogenic enzyme. PIF increased basal expression of SF1 and CYP17A1 in both groups. Following ACTH stimulation, CYP17A1 was decreased in both groups while SF1 expression increased only in the NRC group. Downregulation of CYP17A1 by PIF was proportional to the initial ACTH induced increase in this gene expression. Encapsulation of BACs in alginate preserved basal and ACTH stimulated cortisol PIF 24 day post-therapy In conclusion, PIF regulates stress-induced adrenal steroidogenesis and anti-inflammatory cytokine (IL10). This carries PIF's clinical implication of reducing host's rejection immune response following xenotransplantation.

### Introduction

Adrenal insufficiency describes the inability of the adrenal gland to release sufficient hormones through the limbic hypothalamic pituitary adrenal (LHPA) axis. Congenital Adrenal Hyperplasia (CAH) due to deficiency of 21-hydroxylase is a rather common genetic adrenal disorder in humans. It is associated with clinical symptoms of virilization, neuroendocrine perturbations and metabolic disease [1]. Current treatment algorithm with glucocorticoid substitution can reverse these symptoms only partially and is associated with side effects, involving diabetes, hypertension, osteoporosis. Therefore, restoring normal adrenal function by adrenal cell transplantation would be eminently suited to treat this common and sometimes serious disease. Transplanted adrenocortical cells would respond to physiological demand and reconstitute endocrine feed-back including the ultra- and circadian rhythm of hormone secretion. However, this strategy is extremely limited due to the requirement of life-long use of immunosuppressive drugs [2, 3], which can result in serious side effects such as infection and malignancy [4]. Importantly, such side effects may lower compliance causing rejection of the organ [5]. Intense efforts are ongoing to overcome those deleterious limitations by using organ encapsulation of bovine adrenal cells (BAC) for example, as recently reported [6]. Further improvements in immune regulation without suppression are required in order to progress to a common use of such transplant. One promising therapeutic agent which might improve the outcome of the transplantation without systemic immune suppression could be Prelmplantation Factor (PIF) [7].

PIF is an evolutionary conserved peptide secreted by viable human and in general mammalian embryos, from the two-cell stage onwards [8-10]. After implantation, PIF levels in maternal circulation correlate with favorable pregnancy outcome [11]. PIF promotes implantation and trophoblast invasion involving the anti-apoptotic p53 pathway and regulates activated, while not affecting, basal systemic immunity consequently leading to tolerance without resorting to deleterious immune suppression [12-15]. PIF has been shown to have a protective effect negating adverse environment [10, 16] and to target the embryo to reduce oxidative stress and protein misfolding, critical for survival [17, 18]. PIF targets the innate immunity through antigen presenting cells (APC's) and regulates the adaptive arm of immunity, reducing MLR, proliferation and leading to Th2/Th1 cytokine bias [18-21]. Specifically, PIF targets the cortisone binding site, Kv1.3b channels [22], reducing K+ flux while not affecting early Ca ++ mobilization, a hallmark of immune suppressive drugs. Short-term PIF administration following semi/allotransplant reduces graft vs. host disease (GVHD) and systemic inflammation long-term [23, 24]. Evidence for lack of immune suppression was shown since PIF while controlling GVHD, maintained the beneficial graft vs. leukemia effect [7, 18, 25, 26]. Moreover, PIF presents a very high safety profile. For its Phase I clinical trial, PIF received FAST-TRACK designation by the FDA and successfully completed the university-sponsored clinical trial for autoimmune disease. Consequently, we postulate that PIF's ability to eliminate apoptotic cells, reduce oxidative stress and prevent protein misfolding in damaged cells could represent a new, safe and valuable route to treat adrenal insufficiency and in general PIF's pro-tolerance profile would be beneficial for treatments involving bioartificial organs [12, 17].

Considering the significant potential of PIF in transplantation tolerance and maintenance, we assess here its effect on bovine adrenocortical cells (BAC) - the source for creation of a bioartificial adrenal gland [6]. Bioartificial adrenal, following transplantation, aims to treat adrenal insufficiency. PIF would be a promising therapeutic agent to potentially improve cellular function and reduce the host's immune reaction to the transplant.

The aim of this study was to examine the effect of PIF on BAC. For this cortisol secretion and steroidogenic enzyme activity preventing cell exhaustion while promoting an immune tolerant milieu were measured and analyzed.

### Material and methods

PIF, MVRIKPGSANKPSDD, was provided by Bio-Synthesis, Inc. (Lewisville, TX). Peptide identity was verified by matrix-assisted laser desorption/ionization time-of-flight (MALDI-TOF) mass spectrometry and amino-acid analysis, and the peptide was purified to >95% by HPLC, as documented by mass spectrometry.

### Experimental layout

To study the influence of PIF on the functionality of cell culture of BAC, we performed three series of experiments. In the first set we analyzed the influence of PIF on cortisol production. Based on the observed difference by PIF effects on the cells, isolated from different adrenal glands, subsequent analysis was implemented to define three groups of cells, determined by quantile analysis of stimulation index. Highly Responsive Cells (HRC) were defined by a stimulation index computed by quantile analysis >Q=75; range 16.3 - 20.6, n=12), Normally Responsive Cells, (NRC) computed by quantile analysis Q=30-70 (median ±20); range 5.9 - 10.5, n=12). BAC with weak response to ACTH stimulation (stimulation index computed by quantile analysis Q<25; stimulation index <5) showed short functional life span (data not shown), therefore have no practical value when applied for creation of bioartificial adrenal and did not participate in the subsequent research. The presence of the reciprocal relationship between cell proliferation, viability and apoptosis on one side and release of cortisol and expression of genes SF1 and CYP17A1 on the other side was investigated by correlation analysis.

### Cell preparation and culture

Adrenocortical cells were isolated from bovine adrenal glands shortly after the slaughtering of 1-3 year old cattle as previously described [6]. Briefly, adrenal glands were transported to the laboratory in cold (+4°C) Euro Collins Solution supplemented with 1% (vol/vol) penicillin-streptomycin solution (Thermo Fisher Scientific). The glands were then liberated from fat and connective tissue and rinsed several times with PBS through the central vein to remove remaining blood. Afterwards, a longitudinal incision was made to cut the adrenals in half, the medulla was removed and the cortex was scraped off the capsule and cut in small pieces. Adrenal cortex was digested for 50 min in Dulbecco's modified Eagle's /Ham's F12 (DMEM/F12) medium (Thermo Fisher Scientific), containing 2 mg/ml collagenase and 0.1 mg/ml DNase (both from Sigma-Aldrich) at 37°C while shaking. Collected cells were washed with culture medium, pelleted by centrifugation (8 min, at 300g) and filtered through a 100-pm cell strainer (Becton Dickinson). Then, primary adrenocortical cells were placed in cell culture flasks (Thermo Fisher Scientific) and cultivated at 37°C in a humidified atmosphere (95% air, 5% CO₂) in DMEM/F12 medium with 10% (vol/vol) FBS, 10% (vol/vol) horse serum (both from Thermo Fisher Scientific), 0.1 ng/ml recombinant FGF-2 (PromoCell GmbH) and 1% (vol/vol) penicillin-streptomycin solution for 24 hours.

### Experiments with PIF

PIF was diluted in cell culture medium to a concentration of 0.1 µg/ml. BAC were cultivated with PIF containing medium for 3 days starting the day after cell isolation. During this period, the cells received freshly prepared standard or PIF containing medium every day.

### Steroid release and measurement

For cortisol release experiments, the day after isolation the cells from different adrenals were seeded in a 24 well plate 5×10⁴ cells per well in six replicates for each condition. The cells were cultured with (experimental group) or without (control group) presence of PIF in the cultivation medium. After 48 hours of cultivation in 24 well plates, three wells of the control group of cells from each adrenal gland were stimulated with medium, containing 3 ng/ml ACTH₁₋₂₄ (Synacthen, Sigma-tau Arzneimittel GmbH) and other three with standard medium (basal). The experimental group received PIF containing medium with or without ACTH.

Basal and ACTH stimulated cortisol secretion was measured in cell culture supernatants after 24 hours of cultivation by cortisol ELISA (IBL). Stimulation index was determined by division of ACTH stimulated cortisol versus basal cortisol.

### Reverse-transcription and semi quantitative real-time PCR

Total RNA from bovine adrenocortical cells was isolated using the RNeasy Micro kit (Qiagen) according to the manufacturer's protocol. For reverse transcription, up to 1 µg of total RNA was converted to first-strand cDNA using M-MLV reverse transcriptase, reaction buffer, RNase inhibitor, dNTP mix and oligo(dT) 15/random hexamer primer according to the manufacturer's instructions (Promega).

Semi quantitative real-time PCR was performed using SYBR green (Qiagen) and a Roche Light Cycler 1.5 (Roche). Primers were designed by Primer-BLAST - NCBI software to span at least one intron to prevent unspecific amplification of DNA remnants. To normalize data, the *RPS9* gene was used as an internal control gene. Evaluation of different housekeeping genes in our laboratory (*GAPDH, β-actin*, *TBP*) revealed that *RPS9* is the most stable gene in our system. Typical genes used as internal controls (*GAPDH* and *β-actin*) increased their expression in cultured cells in response on traumatization compared to freshly isolated cells. These data correspond with previously published results [35]. PIF and ACTH effects were defined by comparison of gene expressions for all groups with the mean value of the same gene in control group.

Primers used are listed below:

| Gene | Primer sequence | Annealing Temperature, °C | Product size (base pairs) |
|---|---|---|---|
| RPS9 | F:CGGAACAAACGTGAGGTCT | 60 | 126 |
| | R: CGCAACAGGGCATTACCTTC | | |
| SF1 | F: GCTACGCCGCTGGACTTC | 60 | 388 |
| | R: CACGTGTTGCTGGAGGTTTG | | |
| CYP17A1 | F: GGGGACATCTTCGGGGCTGG | 60 | 497 |
| | R: CTCTGCAGCAGCCGGGACAT | | |
| IL-10 | F: CAGTGAGCTCCAAGAGAGGG | 65 | 242 |
| | R: CTCTAGGGGAGAGGCACAGT | | |

### Assessment of Proliferation, Apoptosis and Viability

The day after the isolation, BAC from different adrenals were seeded in 96 well plate 1×10⁴ cells per well in triplicates for each group. Control cells were incubated with standard cell culture medium, whereas the medium for experimental cells contained PIF. The cells were cultivated this way for 3 days before assessment of cell proliferation, apoptosis and viability.

Proliferation was measured using Cell Proliferation ELISA, BrdU (Roche) following the manufacturer's protocol. Apoptosis was assayed by determination of caspase 3/7 activity using Caspase-Glo 3/7 Assay (Promega) according to the manufacturer's instructions. Viability of BAC was defined by Cell Proliferation Kit II (XTT) (Roche) following the manufacturer instructions.

### BACs Encapsulation

BACS were cultivated with PIF containing medium for 3 days starting the day after cell isolation. During this period the cells received freshly prepared standard or PIF containing medium every day. Experimental group of cells received PIF contained medium the day after cell isolation and were cultivated so for 3 days. Control group of cells was cultivated in standard medium. On day 7 after cell isolation, BACs were encapsulated in alginate in a shape of slabs. For stimulated cortisol ACTH (Synacthen) in concentration 3 ng/mL was used. Basal and ACTH stimulated cortisol was collected on day 10, 17 and 24 after cell encapsulation in alginate. On day 24 after cell encapsulation the experiment was terminated and slabs were collected for RNA isolation and future RT-PCR analysis. Cortisol in supernatant was measured by EIA (IBL).
Cell Encapsulation. On the day following cell isolation, cells were removed from the culture flask (0.25% trypsin EDTA, Gibco, Invitrogen), pelleted by centrifugation, gently mixed with 3.5% (wt/vol) sterile high guluronic acid (HG) alginate [UP-MVG (ultrapure medium viscosity sodium alginate where minimum 60% of the monomer units are guluronate), (Novamatrix], and dissolved in Custodiol-HTK solution (H.S.Pharma). The alginate- cell mixture was then placed either on a glass (for slabs) or spread in the cell compartment of the chamber device (4). Then alginate was cross-linked by applying flat Sintered glass (Pyrexsaturated with 70 mM strontium chloride plus 20 mM Hepes. The thickness of the alginate/cell slab was 550 µm.

The method was previously reported [6]. On day of cells isolation were removed form culture flask using 025% trypsin EDTA, Gibco, Invitrogen). Cells were pelleted and BACs were gently mixed with 3.5% (wt/vol) sterile high guluronic acid (HG) alginate, dissolved in Custodiol-HTK solution (H.S. Pharma). The ultrapure alginate had 60% monomer units. Novamatrix The alginate-cell mixture was then placed either on a glass (for slabs). Then alginate was cross-linked by applying flat Sintered glass (Pyrex), saturated with 70 mM strontium chloride plus 20 mM Hepes. The thickness of alginate/ cell slab was 550 µm. BACs from different adrenal glands were divided on Highly Responsive Cells (HRC) and Normally Responsive Cells (NRC) by the strength of response on ACTH stimulation.

### Statistical analyses

Quantitative data is represented as mean ± s.e. Statistical significance was determined by a two-tailed Student's t-test, one-way analysis of variance (ANOVA) with the post hoc Bonferroni's multiple comparison test, Spearman's rank correlation coefficient or quantile analysis where appropriate. A value of p ≤ 0.05 was considered as significant in all tests. Graph Pad Prism 5.0 (GraphPad Software, Inc., La Jolla, USA) was used for statistical analysis.

### Results

### PIF influence on cortisol production of BAC

PIF significantly decreased ACTH stimulated cortisol release by 84% in highly responsive cells (HRC) (Fig.12A). When applied on the BAC with normally responsive cells (NRC) level of stimulation index, PIF effect was not observed (p>0.1). At the same time, PIF had no effect (p>0.1) on basal cortisol secretion in both groups (Fig. 12B).

### Characterization of groups with normal and heightened response to ACTH stimulation

Basal and ACTH stimulated cortisol production of NRC and HRC cells is presented in Fig. 13A (basal cortisol 47±2 ng/ml for NRC and 63±4 ng/ml for HRC respectively, p<0.01; and ACTH stimulated - 313±98 ng/ml for NRC and 1052±117 ng/ml for HRC, p<0.05). We found that the difference in the intensity of the response to ACTH stimulation between the two groups was mediated by the level of mRNA gene expression of SF1 and steroidogenic enzyme CYP17A1. In HRC CYP17A1 and SF1 genes are upregulated while in NRC the same genes are downregulated (CYP17A1 expression was 0.16±0.01 for NRC and 0.83±0.07 for HRC, p<0.01; and SF1 - 0.29±0.1 for NRC and 1.4±0.23 for HRC respectively, p<0.001; Fig.13B and 13C). ACTH stimulation increased cortisol release and upregulated expression of SF1 and CYP17A1 in both groups of cells (expression of SF1 increased 9.6 fold in NRC and by 4.9 fold in HRC groups, p<0.001; and expression of CYP17A1 was upregulated by 4482 fold in NRC and by 3016 fold in the HRC group, respectively, p<0.001; Fig.13B and 13C). The significant difference between NRC and HRC groups with cortisol production and expression of SF1 and CYP17A1 remained unchanged following ACTH stimulation (p<0.05 for SF1 and p<0.01 for CYP17A1; Fig.13B and 13C).

Differing from HRC, the NRC group shows a higher rate of cell proliferation (absorbance 0.33±0.02 OD for NRC and 0.24±0.01 OD for HRC, p<0.05; Fig.14A). Cell viability and apoptosis were also higher in the NRC group as compared to HRC (absorbance 1.46±0.03 OD for NRC and 1.18±0.04 OD for HRC, p<0.05 for cell viability and luminescence 218900±2149 RLU for NRC and 204370±4227 RLU for HRC, p<0.05; Fig.14B and 14C). NRC have a strong trend to downregulate the expression of IL-10 (gene expression of 1.38±0.51 for NRC and 0.18±0.08 for HRC, p=0.08; Fig. 14D).

Furthermore, we found a negative correlation between cell proliferation and the level of cortisol production (r=-0.87, n=12, p < 0.001); cell proliferation and the expression of SF1 (r=-0.92, n=12, p < 0.05) and proliferation in CYP17A1 (r=-0.81, n=12, p < 0.05). ACTH stimulation also induced significant reduction in proliferation of BAC (69 ± 7.9% of control, n=6, p<0.05).

### PIF regulatory effect on the expression of CYP17A1 and SF1

We further investigated the role of SF1 and CYP17A1 as a possible target for the action of PIF. Data showed that PIF increased basal expression of CYP17A1 in both NRC and HRC groups. Interestingly, in the NRC group following PIF exposure there was a major increase in the expression of CYP17A1 when compared to HRC group (13 fold vs. 4.5 fold respectively).

Similarly, basal level of SF1 following PIF exposure in both groups of cells was significantly elevated. An increase in SF1 expression after impact of PIF in the NRC group reached the initial level of expression found in the HRC group of BAC (1.08±0.1 for NRC and 1.37±0.23 for HRC respectively; Fig.15B). After ACTH stimulation PIF reduced CYP17A1 expression 1.7 fold in NRC group of cells, whereas in HRC group of BAC PIF downregulated the same gene expression by 5 fold (p<0.05 for NRC and p<0.01 for HRC respectively; Fig. 15C).

During ACTH stimulation, PIF significantly downregulated SF1 expression in the HRC group (2.8 fold, p<0.05) without affecting expression of SF1 in the NRC group (p>01). Interestingly, reduced expression of SF1 by PIF in HRC cells became identical to the initial SF1 expression in NRC group of BAC (2.4±1.01 for HRC and 2.82±0.85 for NRC group; Fig.15D).

### PIF effect on IL-10

PIF's effects on the expression of IL-10 in BAC were significant but opposite in the two tested groups. In the NRC group, which initially demonstrated higher expression of IL-10, PIF blocked its expression (p<0.05). In contrast, in the HRC group, where initially IL-10 levels were lower, the expression of IL-10 was significantly upregulated by PIF (21 fold, p<0.001) (Fig.15E).

### Long-term effects of PIF on encapsulated BACs (enBACs) cortisol secretion

Ultimate goal for BACs is to use for transplantation where the alginate encapsulation was already shown to be effective in creating an immune privileged environment, *in vivo* [6]. Whether PIF has a long term effect on BACs after stopping exposure in culture was examined. This element is essential for low term survival of these cells in an isolated environment. The short term preconditioning of allogeneic MSC prior to transplant supported such a premise [18]. For three days in culture BACs were exposed to PIF, the same dose used for the short term cultures. Following BACs encapsulation in alginate after 24 days or 28 days after withdrawing of PIF from the cell culture media the effect on cortisol secretion was determined. Fig. 16 examined the global cortisol production during the 10-24 days of the observation period. Data shows that PIF significantly increased basal cortisol secretion by enBACs in NRC while in HRC minimal effect was noted. Following ACTH stimulation PIF mildly increased cortisol secretion in NRC while significantly reducing the steroid secretion in HRC group. We further examined the same parameters basal and ACTH stimulated enBACs in both NRC and HRC cultures in different time points (Fig. 17). Divergent effects were noted in the two time periods. As for basal cortisol secretion by day 10 PIF pre-exposure increased the basal secretion of cortisol. As well increased that in HRC. On the other hand, while importantly in NRC, PIF induced increase was maintained that in HRC was not. As for ACTH stimulated enBACs the effect on NRC as well HRC was mild on day 10, however at 17-24 days after encapsulation the inhibitory effect on HRC became highly significant (Fig.18).

### Long-term effects of PIF on encapsulated BAC steroidogenesis

To determine whether PIF induced delayed effect on cortisol secretion in enBACs at day 24 correlate with steroidogenesis was determined. In line with the secreted steroid data PIF reduced amount of total RNA, gene expression of SF1 and CYP17A1, significantly (Fig. 19). No effects of PIF were observed when PIF was applied directly on enBACs at that time point. Data reveals short term PIF sustained effect in preserving encapsulated BACs function long term.

### Discussion

This study demonstrates that PIF, originally an embryo-secreted peptide, made synthetically exerts potent regulatory effects on cortisol production and release in primary culture of BAC. We demonstrate that the action of PIF depends on the initial functional status of the cells, basal or activated, determined by the degree of their response to ACTH stimulation. The regulatory effect of PIF primarily consists of its selective ability to reduce cortisol release by cells that have a heightened response to ACTH stimulation while not affecting cells with "normal" response to ACTH. These results reveal the selective activity of PIF on BAC, confirming previously published results when tested on systemic mononuclear cells. The effect of PIF on activated cells was more pronounced compared to unstimulated cells [19, 20]. We also show that PIF enable long term preservation of BAC once encapsulated preserving the ability to regulate cortisol secretion. Therefore, we document that this dual regulatory mechanism also applies to non-immune BAC. We were able to show that PIF affects the cortisol synthesis pathway of BAC indirectly by targeting SF1 and CYP17A1 genes expression. The CYP17A1 gene is a cytochrome P450 17A1 enzyme, involved in synthesis of steroids from cholesterol. ACTH stimulation activates CYP17 transcription by promoting the binding of SF1 [27]. SF1 plays an essential role in homeostatic proliferation of the adult adrenal gland. It acts as an obligatory activator of most steroidogenic enzymes in the adrenal cortex and participates in both proliferation and differentiation (steroidogenesis) of the adult gland [28]. HRC's have much higher cortisol production levels and expression of CYP17A1 and SF1 genes when compared to NRC, respectively. Cell activation by ACTH leads to significant upregulation of these genes.

Data indicates that the regulatory effect of PIF on SF1 and CYP17A1 depends on their initial level of expression. Added to the adrenocortical cells, PIF markedly increased SF1 and CYP17A1 in cells where basal expression was low, and, in contrast, had only a mild effect on genes in cells where the basal expression was already relatively high. ACTH significantly upregulates SF1 and CYP17A1 expression while PIF significantly downregulates those in NRC and even more effectively in HRC. Notably, the observed effects of PIF directly depend on the rate of response to ACTH stimulation.

The mechanism of PIF's action is to block the abrupt activation of BAC by the specific physiologic activator. Consequently, selective suppression of cells associated with high response by the cytochrome p450 enzyme complex takes place. PIF targets hyper functioning cells that could become exhausted and damaged. This reveals the unique protective mechanism of PIF's action which may enable long-term cell survival. PIF's inhibitory effect begins when activation of cytochrome p450 reaches a certain level. Involvement of PIF in additional local protective pathways may also involve adrenodoxin reductase, a p450 mitochondrial enzyme, which has the highest expression in the adrenal cortex [29]. PIF may act by regulating this enzyme's oxidoreductase activity thus reducing oxidative stress and protein misfolding [17, 20, 21].

The protective mechanism of PIF's action is further shown by the observed reduction in BAC hyper functionality, which was coupled with an increase in IL-10, a key anti-inflammatory cytokine. IL-10 is known to reduce alloimmune response in transplantation [30]. IL-10 expression may be connected to local defense mechanisms mitigating stimulated immune cells activity as well as protecting overactive BAC. Moreover, IL-10 also promotes proliferation and cell differentiation [31, 32]. PIF was already shown to promote IL-10 both *in vitro* as well *in vivo,* to increase cell viability and to reduce apoptosis [33, 34]. However, in our study, the effect of PIF on BAC proliferation and apoptosis was not observed, possibly due to the short observation period or the low number of immune cells present in culture.

PIF has a dual regulatory effect on BAC: it activates downregulated key steroidogenic enzymes while reducing those that are overactive. PIF thereby potentiates underperforming BAC by promoting cortisol secretion while limiting the same corticosteroid release in overactive cells. PIF exerts a local immune regulatory effect through IL-10 expression.

The short term observation with PIF led us to examine whether those beneficial effects on cortisol secretion can persist long term. Such is critical since those cells are aimed to be transferred to those that have low adrenal function. As we have recently showed the superiority of cells that are encapsulated in alginate (enBACs) following transplant their function compared to free cells [6]. The data showed acceptance of the graft by immune competent rats. Following transplantation maintained oxygen and nutrients exchange, angiogenesis around the transplant and lack of fibrosis facilitated the graft functionality up to 4 weeks. However, as data showed further improvement in the cells functionality had to be achieved before these cells provide a long term solution this patient population. Therefore this model enabled to test whether short term PIF exposure is capable of long term effect of cells that are no further exposed to the peptide. The finding with using enBACs herein fully support the value of PIF in this setting. The production of cortisol persisted up to 4 weeks of observation with maintained differences between basal and ACTH stimulated cells. This activity was also confirmed by the reduced steroidogenic enzymes thereby adverse exhaustion of these cells which reduces their functionality was averted. Such confirmed the dual regulatory action that are seen in short term cultures. This supports the view that PIF based preconditioned enBACs transplantation should be contemplated. In addition, since PIF was shown to protect against type diabetes by preserving insulin expression application of such encapsulation for islets cells could contemplated as well. This is also supported since PIF protected against vascular inflammation thereby may favor angiogenesis [22].

In summary, PIF is a promising agent for bioartificial transplant applications. PIF features address most currently known limitations of xenotransplant use and stand to improve the outcome of xenotransplantation of a bioartificial adrenal gland. By regulating BAC function, PIF may enable development of a safe and functional bioartificial adrenal gland.
1. Bornstein, S.R., Predisposing factors for adrenal insufficiency. N Engl J Med, 2009. 360(22): p. 2328-39.
2. Andrews, P.A., et al., Summary of the British Transplantation Society/Renal Association UK Guidelines for Living Donor Kidney Transplantation. Transplantation, 2012. 93(7): p. 666-673.
3. Martin, P., et al., Evaluation for Liver Transplantation in Adults: 2013 Practice Guideline by the American Association for the Study of Liver Diseases and the American Society of Transplantation. Hepatology, 2014. 59(3): p. 1144-1165.
4. Wagner, M., et al., Mycophenolic acid versus azathioprine as primary immunosuppression for kidney transplant recipients. Cochrane Database Syst Rev, 2015(12): p. CD007746.
5. Bamgbola, O., Metabolic consequences of modern immunosuppressive agents in solid organ transplantation. Ther Adv Endocrinol Metab, 2016. 7(3): p. 110-27.
6. Balyura, M., et al., Transplantation of bovine adrenocortical cells encapsulated in alginate. Proc Natl Acad Sci U S A, 2015. 112(8): p. 2527-32.
7. Shainer, R., et al., Immune Regulation and Oxidative Stress Reduction by Preimplantation Factor following Syngeneic or Allogeneic Bone Marrow Transplantation. Conference Papers in Medicine, 2013. 2013(Article ID 718031): p. 1-8.
8. Barnea, E.R., Insight into early pregnancy events: the emerging role of the embryo. Am J Reprod Immunol, 2004. 51(5): p. 319-22.
9. Barnea, E.R., Applying Embryo-Derived Immune Tolerance to the Treatment of Immune Disorders. Annals of the New York Academy of Sciences, 2007. 1110: p. 602-618.
10. Stamatkin, C.W., et al., PreImplantation Factor (PIF) correlates with early mammalian embryo developmentbovine and murine models. Reprod Biol Endocrinol, 2011. 9: p. 63.
11. Ramu, S., et al., PreImplantation factor (PIF) detection in maternal circulation in early pregnancy correlates with live birth (bovine model). Reprod Biol Endocrinol, 2013. 11: p. 105.
12. Paidas, M.J., et al., A genomic and proteomic investigation of the impact of preimplantation factor on human decidual cells. Am J Obstet Gynecol, 2010. 202(5): p. 459 e1-8.
13. Barnea, E.R., D. Kirk, and M.J. Paidas, Preimplantation factor (PIF) promoting role in embryo implantation: increases endometrial integrin-alpha2beta3, amphiregulin and epiregulin while reducing betacellulin expression via MAPK in decidua. Reprod Biol Endocrinol, 2012. 10: p. 50.
14. Duzyj, C.M., et al., PreImplantation Factor (PIF*) promotes embryotrophic and neuroprotective decidual genes: effect negated by epidermal growth factor. Journal of Neurodevelopmental Disorders, 2014. 6(1): p. 36.
15. Barnea, E.R., et al., PreImplantation Factor (PIF*) Endogenously Prevents Preeclampsia: Promotes Trophoblast Invasion and Reduces Oxidative Stress. J. Reprod. Immunology, 2015.
16. Stamatkin, C.W., et al., Preimplantation factor negates embryo toxicity and promotes embryo development in culture. Reprod Biomed Online, 2011. 23(4): p. 517-24.
17. Barnea, E.R., et al., Insight into PreImplantation Factor (PIF*) mechanism for embryo protection and development: target oxidative stress and protein misfolding (PDI and HSP) through essential RIPK binding site. PLoS One, 2014. 9(7): p. e100263.
18. Goodale, L.F., et al., PreImplantation Factor (PIF) protects cultured embryos against oxidative stress: relevance for recurrent pregnancy loss (RPL) therapy. Oncotarget, 2017: p. 1-14.
19. Barnea, E.R., et al., PreImplantation Factor (PIF) orchestrates systemic antiinflammatory response by immune cells: effect on peripheral blood mononuclear cells. Am J Obstet Gynecol, 2012. 207(4): p. 313 e1-11.
20. Barnea, E.R., et al., PIF direct immune regulation: Blocks mitogen-activated PBMCs proliferation, promotes TH2/TH1 bias, independent of Ca(2+). Immunobiology, 2015. 220(7): p. 865-75.
21. Barnea, E.R., et al., PreImplantation factor (PIF*) regulates systemic immunity and targets protective regulatory and cytoskeleton proteins. Immunobiology, 2016. 221(7): p. 778-93.
22. Chen, Y.C., et al., PreImplantation factor prevents atherosclerosis via its immunomodulatory effects without affecting serum lipids. Thromb Haemost, 2016. 115(5): p. 1010-24.
23. Almogi-Hazan, O., et al., The Role of Nitric Oxide Toxicity and Oxidative Stress in Graft vs Host Disease, in Oxidative Stress: Causes, Role in Diseases and Biological Effects. 2014, Nova Science Publishers, Inc.
24. Azar, Y., et al., Preimplantation factor reduces graft-versus-host disease by regulating immune response and lowering oxidative stress (murine model). Biol Blood Marrow Transplant, 2013. 19(4): p. 519-28.
25. Shainer, R., et al., PreImplantation factor (PIF) therapy provides comprehensive protection against radiation induced pathologies. Oncotarget, 2016.
26. Wang, B., et al., ATXN1L, CIC, and ETS Transcription Factors Modulate Sensitivity to MAPK Pathway Inhibition. Cell Rep, 2017. 18(6): p. 1543-1557.
27. Li, D., et al., Cyclic AMP-stimulated interaction between steroidogenic factor 1 and diacylglycerol kinase theta facilitates induction of CYP17. Mol Cell Biol, 2007. 27(19): p. 6669-85.
28. Walczak, E.M. and G.D. Hammer, Regulation of the adrenocortical stem cell niche: implications for disease. Nat Rev Endocrinol, 2015. 11(1): p. 14-28.
29. Hanukoglu, I., et al., Isolation of a cDNA for adrenodoxin reductase (ferredoxin-NADP+ reductase). Implications for mitochondrial cytochrome P-450 systems. Eur J Biochem, 1987. 169(3): p. 449-55.
30. Shinozaki, K., et al., Allograft transduction of IL-10 prolongs survival following orthotopic liver transplantation. Gene Ther, 1999. 6(5): p. 816-22.
31. Dinant, S., et al., IL-10 attenuates hepatic I/R injury and promotes hepatocyte proliferation. J Surg Res, 2007. 141(2): p. 176-82.
32. Heine, G., et al., Autocrine IL-10 promotes human B-cell differentiation into IgM- or IgG-secreting plasmablasts. Eur J Immunol, 2014. 44(6): p. 1615-21.
33. Mueller, M., et al., PreImplantation Factor bolsters neuroprotection via modulating Protein Kinase A and Protein Kinase C signaling. Cell Death Differ, 2015. 22(12): p. 2078-86.
34. Mueller, M., et al., PreImplantation factor promotes neuroprotection by targeting microRNA let-7. Proc Natl Acad Sci U S A, 2014. 111(38): p. 13882-7.
35. Lee, J.H., et al., Mechanical injury of cartilage explants causes specific time-dependent changes in chondrocyte gene expression. Arthritis Rheum, 2005. 52(8): p. 2386-95.

### Example 3

### Allogeneic ovarian transplantation using immunomodulator Preimplantation Factor (PIF) as monotherapy restored ovarian function in olive baboon

### Abstract

*Objective:* Allogeneic ovarian transplantation may be an alternative in the future to oocyte donation in women with premature ovarian failure. The objectives of this study were to a) evaluate allotransplantation feasibility for restoration of ovarian function and b) assess efficacy of synthetic PIF monotherapy as sole immune-acceptance regimen.

*Design:* Experimental animal study using non-human primates (Papio anubis). *Setting:* Academic research center. *Animals:* Two female olive baboons. *Intervention(s):* Allogeneic orthotopic ovarian tissue transplantation. PIF was administered as a monotherapy to prevent immune rejection, achieve transplant maintenance and function. Subjects underwent bilateral oophorectomy followed by transplantation of prepared ovarian cortex. Postoperatively, subjects were monitored for clinical and biochemical signs of graft rejection and return of function. Weekly blood samples were obtained to monitor graft acceptance and endocrine function restoration. *Main Outcome Measure(s):* Transplant acceptance and return of ovarian function.

*Result(s)*: Postoperatively, there were no clinical signs of rejection. Laboratory parameters (Alanine aminotransferase (ALT), Aspartate Aminotransferase (AST), Blood Urea Nitrogen (BUN), creatinine) did not indicate organ rejection at any stage of the experiment. Initially, significant loss of follicles was noticed after grafting and serum FSH and E2 levels were consistent with ovarian failure. Seven months after transplantation, one animal exhibited restored ovarian function (perineal swelling and return of menstruation). Also reflecting graft acceptance also the laparotomy wound completely healed with restored hair growth.

*Conclusion(s):* Organ rejection after allogeneic ovarian transplantation was prevented using PIF as monotherapy for the first-time and no side-effects were recorded. The
study shows the clinical feasibility of ovarian allotransplantation to obtain ovarian function and fertility.

### Introduction

Advances in cancer therapy have markedly improved survival rates, which resulted in an increased number of young cancer survivors of reproductive age. Unfortunately, high dose cytotoxic chemotherapy and/or ionized radiation often impairs fertility and frequently leads to primary ovarian insufficiency (POI) (1). The POI not only affects fertility but also impairs physical and psychological well-being. As a possible treatment for gonadotoxic induced POI, cryopreservation of ovarian tissue followed by transplantation has been lately used (2). However, in women who already underwent cytotoxic treatment or who suffer from idiopathic or genetic-related POI, egg donation is the only current option for achieving pregnancy. This procedure, however, is prohibited in many countries and requires IVF, thus eliminating the chances of natural conception.

An alternative approach for addressing POI is by performing allogeneic ovarian transplantation (AOT). Although several studies investigated AOT, the use of high dose immunosuppressants remains as the main barrier (3-5). These drugs are associated with serious side effects leading to diabetes, hypertension, increased susceptibility to infections, malignancies and nephrotoxicity (6). For non-life saving transplant such as with the ovary the toxicity of current drugs is hard to justify. Although AOT with standard immunosuppressants has been attempted in few patients, no information on long term outcome including pregnancy is available (7, 8).

Pregnancy is a unique immune milieu, where semiallogeneic embryos or fully allogeneic embryos (in cases of ovum donation) can successfully survive and thrive, i.e. achieve both immune modulation/adaptation and transplant acceptance (9). Identification of a pregnancy specific responsible compound involved could revolutionize the field of transplantation by promoting tolerance, preserving anti-pathogen activity while avoiding the deleterious immune suppression.

The Prelmplantation Factor (PIF), a peptide secreted by viable embryos, is a promising therapeutic candidate in many areas (10-13). Established functions of this peptide include immune modulation without suppression and transplant acceptance without rejection. PIF is secreted from the two-cell stage onwards by mammalian embryos, and levels in early maternal circulation correlate with favorable pregnancy outcome (14, 15). Immune regulatory features of PIF were successfully transposed to non-pregnant immune disorders using synthetic PIF (same sequence as endogenous PIF) (11). PIF is effective in diverse clinically-relevant autoimmunity, ionic radiation, vascular inflammation, neuroprotection and transplant acceptance models (16-21).

Especially, short-term low dose PIF prevented deleterious graft vs. host disease (GVHD) development following semi or importantly allogeneic bone marrow transplantation (BMT) (22). While GVHD was reduced, the beneficial graft vs. leukemia effect was preserved, reflecting maintained immune function. In addition, the reduced skin, liver, and colon damage (23, 24) was coupled with decreased systemic inflammation (25). The high safety and tolerability profiles of PIF in humans have been established in the FDA-awarded FAST-TRACK, university-sponsored phase 1a/b first-in-human clinical trial for autoimmune disease (Clinicaltrials.gov, NCT02239562).

The current study aimed to evaluate PIF monotherapy used as a sole immunomodulator without any immunosuppressants to achieve allogeneic ovarian transplant acceptance in conjunction with restoration of ovarian function in a primate model.

### Materials and Methods

### Animals used for allotransplantation

The study was approved by the Institutional Animal Care and Use Committee (IACUC) of the Institute of Primate Research (IPR), Karen, Kenya. Two female (8 years old), regularly cycling healthy olive baboons (*Papio anubis*) were obtained for this study. Subjects were housed in individual cages, and kept on a diet of commercially available monkey chow (Unga Feeds Ltd, Nairobi, Kenya) supplemented with vegetables, fruits and water ad libitum (26).

### Ovariectomy

For surgery, anesthesia induction was performed with 200 mg of ketamine (Agraket^{®}, Agrar Holland BV, Soest, Holland) combined with 10 mg xylazine (Ilium Xylazil^{®}, Troy Lab Pty Limited, Smithfield, Australia) i.m. After local lidocaine (Xylocain) spray orally, the animals were intubated. Anesthesia was maintained using halothane to minimal alveolar concentration of >1.3 and an oxygen/nitrous oxide mixture as described before (27). Before skin incision, each animal received a single dose of gentamycin (Vetgenta, Dawa ltd, Nairobi, Kenya) 100mg i.v. Through a small, subumbilical midline incision the ovaries were identified and ovarian vessels were ligated with 4.0 glycolide and epsilon-caprolactone monofilament sutures (Monocryl^{®}, Ethicon, Inc, Somerville, NJ, USA).
Both ovaries were resected after stepwise ligation of the mesoovarian tissue and the ovarian ligament with 4.0 monofilament sutures.

### Preparation of ovarian cortex

The collected ovaries were bisected in the longitudinal axis to create two fairly flat halves. The ovarian medulla was dissected out using a fine scalpel and micro-surgical scissors (S&T Microsurgery, Neuhausen, Switzerland). The remaining medullary tissue was further scraped away to obtain a thin cortical tissue (1 mm thickness) (Fig. 20). Prepared cortical tissue was incubated in a medium containing 20ml PBS (Medicago, Uppsala, Sweden), 20mg PIF dissolved in 4ml PBS and 100 µl of Penicillin G/ Streptomycine (Eagle Long-PS, Eagle vet tech co Ltd, Chungnam, Korea) for 1 hour at 37°C with 5% CO2.

### Transplantation

For transplantation a peritoneal pocket was created beneath the fimbriae on the broad ligament of both sides. Each ovarian cortex (10×10×1-2mm) from the other animal was placed onto the surface of the peritoneal pocket and fixated with three to four 8.0 monofilament interrupted sutures (Monocryl^{®}, Ethicon, Inc, Somerville, NJ, USA). After confirming a good contact of grafts to the peritoneum and hemostasis, the peritoneal pocket was left open.

Postoperative recovery was uneventful and animals received analgesia according to the research site regulations with Diclofenac 1ml/25mg i.m. (Shandong Shenglu Pharmaceutical Co.,Ltd; Shandong,China).

### Achieving Immunomodulation using PIF

Synthetic PIF (purity documented by HPLC and mass spectrometry: >95%), proprietary, was produced by Biosynthesis (Lewisville, TX, USA) and was supplied by BioIncept LLC, Cherry Hill, NJ, USA.

Immunomodulation/transplant acceptance was initiated using PIF 10 mg (with 0.75 mg/kg = 10 mg dissolved in 1 ml PBS) injected twice a day s.c. starting one day before surgery (Fig. 21). Immunomodulation/transplant acceptance regimen after ovarian transplantation was carried out for 12 weeks injecting PIF 10 mg (dissolved in 1 ml of PBS) twice daily s.c. for three weeks with one week break in between each cycle (Fig. 21). Afterwards, upon observing no rejection or side effects, PIF administration was discontinued according to the protocol and subjects were followed without any therapy for an additional 6 months for a total of 9 months (Fig. 21).

### Surveillance for immune acceptance (monitoring signs of rejection) and return of ovarian function

Animals were followed up closely after surgery with a weekly physical examination including blood pressure, body temperature, pulse and respiratory rates. In addition, weight, urine output, skin and perineum were monitored. The blood sampling was done under short anesthesia with 200 mg of ketamine combined with 10 mg xylazin i.m. Blood tests for blood urea nitrogen (BUN), creatinine, aspartate aminotransferase (AST) and alanine aminotransferase (ALT) were performed weekly to assess indirect signs of rejection.

Follicle stimulating hormone (FSH) and estradiol (E2) assays were performed monthly to determine return of ovarian function. Serum FSH radioimmunoassay (RIA) was performed by the Endocrine Technologies Support Core (ETSC) at the Oregon National Primate Research Center (ONPRC; Beaverton, OR) using reagents from Dr. Albert Parlow at the National Hormone and Peptide Program (NHPP, Harbor-UCLA Medical Center, Los Angeles). This is a homologous cynomolgus macaque assay with recombinant cynomolgus FSH (AFP-6940A) for both iodination and standards, and has previously been used in the ETSC to measure FSH in multiple nonhuman primate species including baboons. The rabbit anti-cynomolgus FSH, AFP-782594, was used at a final dilution of 1:1,038,462. The standard curve ranged between 0.005 and 10 ng/tube and the detection limit of the assay was 0.005 - 0.02 ng/tube. The intraassay variation for this assay was 10.6%. Because all samples were analyzed in one assay, no inter-assay variation was calculated for these samples, but the overall inter-assay variation for this assay in the ETSC is less than 15%.

Estradiol (E2) levels were analyzed by immunoassay using a Roche cobas e411 automated clinical platform (Roche Diagnostics, Indianapolis, IN) at the ETSC. This assay was previously validated for use in nonhuman primates (28, 29). The assay sensitivity range for the E2 assay was 5 - 3000 pg/ml. Intra- and inter-assay CVs for the Roche assays are consistently less than 7%.

Return of ovarian function was confirmed when perineal swelling followed by menstruation (representing typical signs of cyclicity in this species) was observed (Fig. 22)(30).

### Histology to assess ovarian tissue status before and after allografting

Ovarian biopsies collected shortly before transplantation and after euthanasia were fixed in 10% formaldehyde and dehydrated progressively in increasing concentrations of ethyl alcohol (50%, 70%, 80%, 90% and absolute alcohol), after which they were immersed in toluene. Subsequently they were processed through toluene three times and then infiltrated in a paraffin wax mixture in an oven (Oven model-5831, National appliance Co., Portland, Oregon, USA). The tissues were then oriented in a perpendicular fashion on a piece of embedding ring and embedded in molten wax. For histological evaluation paraffin-embedded tissue was processed as 5 µm sections, deparaffinized and stained with hematoxylin-eosin (HE). The sections were stained with harris haematoxylin for 7 minutes then washed in running tap water before decolorizing in 0.5% acid alcohol and toning in ammonia water. Toned sections were counterstained with eosin Y for 5 minutes then washed in water. Stained sections were then dehydrated through several changes of increasing concentrations of ethanol, cleared in xylene and finally mounted using DPX.

Histology sections were analyzed in a Leica DM500 Microscope (Leica Microsystems Ltd, Heerburgg, Switzerland) and photo documentation was performed with a Leica ICC50 Camera (Leica Microsystems Ltd, Heerburgg, Switzerland).

### Results

### Clinical and laboratory parameters to assess transplant immune acceptance with PIF monotherapy as sole maintenance regimen

Postoperative recovery was uneventful in both animals. No clinical or laboratory signs of rejection were observed at any time throughout the 9 months of follow up. Scarless wound-healing was observed within the first 16 weeks and by week 31 regrowth of local hair was documented (Fig. 23). Biochemical parameters revealed no major deviations from normal levels in kidney and liver-function parameters that would indicate rejection (Fig. 24). Clinical parameters for both animals remained stable throughout the 9 months observation period (i.e. 3 months of treatment and 6 months of post-treatment observation) with no clinical signs of rejection.

### Endocrine assays to evaluate the return of ovarian function

Both animals showed a continuous decline of FSH levels after transplantation (Fig. 25). Animal number one showed a decline of 39.33% and animal number two a decline of 29.27% in FSH levels within the first 7 months postoperatively. Animal number one, with the more distinct FSH decline had a 29% lower mean FSH than animal number two. Estradiol levels remained undetectable for the first 7 months after transplantation.

A rise in estradiol levels (28.73 pg/ml) in animal number one was documented 229 days after ovarian transplantation following perineal inflation and an episode of menstruation (Fig. 22A). Thereafter no further signs of cyclicity were detected and at 324 days after transplantation the animals were euthanized for autopsy.

### Gross morphology and histology of ovarian grafts

Pre-transplant histology showed intact ovarian tissue with multiple follicles at different stages in both animals. Macroscopically antral follicles and corpus luteum cysts were observed. When ovarian grafts were inspected in situ, there was no sign of rejection macroscopically (Fig. 26). However, post-transplantation histology revealed a significant depletion of follicles. Microscopic evaluation demonstrated the presence of a number of early stage follicles, without evidence of inflammatory activity (Fig. 27).

### Discussion

Herein we report a successful organ allotransplantation without encountered signs of rejection followed by restored function. We show that PIF, an immune modulatory synthetic peptide monotherapy, achieved long term transplant acceptance after stopping therapy, which led to restored ovarian function in a primate model. Importantly, PIF treatment throughout the treatment period and for several months afterwards was not associated with side effects or any apparent signs of rejection, supporting its use for ovarian allotransplantation and beyond.

Over the last decade growing attention has been paid to the field of gynecological organ transplantation. Ovarian auto-transplantation to restore fertility after cancer treatment, is slowly moving from an experimental procedure towards a routine clinical strategy, with more than 100 children born worldwide to date. However, due to variability in the graft survival further improvements are required to enable sustained long term ovarian function (2, 31). Recently, first successful cases of uterine allotransplantation resulted in several live-births (32, 33). Due to the non-lifesaving character of the procedure, the use of immunosuppression in uterine allotransplantation remains controversial (32). Ovarian allotransplantation combined with classical immunosuppressive agents would encounter similar medical and ethical challenges. The ovary is not an immunologically privileged organ unlike some authors suggested in the past. Indeed, most research showed aggressive immunorejection in animal models of ovarian allogeneic transplantation, when immunosuppressive drugs were not utilized (34-36). Even with high dose cyclosporine, allotransplants failed to survive and were rejected (34). In mice that had undergone allogeneic ovarian transplantation with no immunosuppression, bioluminescence methodology demonstrated a rapid loss of ovarian function and aggressive organ rejection (37). Furthermore, low CD4+/CD8+ ratio of peripheral T-cells with high CD4+/CD8+ cells
infiltration into the ovarian allograft confirmed aggressive immune rejection after allotransplantation (38).

Ovarian allotransplantation could serve as a potential cure for women suffering from POI. Etiology of POI includes: gonadotoxic cancer-treatment, benign surgery (e.g. endometriosis), genetic disorders (e.g. Turner Syndrome) and idiopathic POI (39). Besides infertility, patients with POI are at increased risk for cardiovascular disease and osteoporosis, leading to reduced life expectancy, if not treated properly (40-42). Various POI manifestations like infertility and vasomotor symptoms impair the quality of life and psychological wellbeing (43). Oocyte donation is a reasonable option to achieve pregnancy in patients with POI and several well tolerated medications are available to manage the side effects of hormone deprivation (44-46). Established immunosuppressants, such as glucocorticoids, the calcineurin inhibitors cyclosporine and tacrolimus as well as the antiproliferative agents azathioprine and mycophenolate mofetil are associated serious side-effects like nephrotoxicity, hypertension, diabetes, malignancies and infections (6). As the ovary is not a vital organ and when considering known risks of rejection and the toxicity of immunosuppressants, clinical application of AOT can be a very controversial issue.

Nevertheless, AOT is a potentially useful and powerful tool not only to restore fertility but more importantly to restore endocrine function in women suffering from POI, provided that the organ rejection can be prevented by using simple to administer and non-toxic agents or methods. Ideal transplant regimens would be safe (devoid of deleterious side effects), assure acceptance for the long term (obviate need for transplant removal), obviate the need for organ matching and facilitate/enable restoration of organ functionality (menstrual cyclicity, and/or even pregnancy).

The search for safe and effective immune regulatory compound(s) that would lead to allograft acceptance without immune suppression and restore function is a long-term quest. In this study, PIF immunomodulator was used and shown to prevent organ rejection and lead to organ function. Endogenous PIF is an evolutionarily conserved mammalian peptide secreted by viable embryos and present in maternal circulation of viable pregnancies (10, 11, 14, 15, 47, 48). Synthetic PIF replicates endogenous PIF's function and is effective in several non-pregnant preclinical models, acting in integrated manner locally (target organ) and on the systemic immunity. Despite short circulating halflife, PIF exerts long-term pharmacodynamic effects after being cleared from circulation (16). Such a delayed beneficial effect of PIF monotherapy following semi/allogeneic bone marrow transplant and after lethal total body irradiation was already reported (22-24). PIF targets CD14+ cells, namely antigen presenting cells (APC) shifting them from effector to regulatory phenotype. Through upregulation of B7H1 expression T-cells response is redirected to repair instead of inflammation. PIF reduces NK cells cytotoxicity by decreasing CD69 expression - a possible contribution to the observed organ acceptance (49). Notably, CD69, an inducer of T-cell activation is increased in acute organ rejection in renal and heart transplant patients (50-52). Collectively, borrowing from embryo/maternal interaction, PIF selective action on innate and adaptive immunity "at need" enables this critical balance between tolerance/rejection and maintained immune response to danger signals.

In this study PIF monotherapy was administered as the sole transplant maintenance regimen (no steroids, or any other drugs administered) and used at three different settings. First the donor/ recipient were injected with PIF prior to transplant with the goal of achieving recipients' immune homeostasis, reducing surgery induced inflammatory response. Second, ovaries were preconditioned with PIF ex vivo in between ovarian harvesting and transplantation in short-term culture to reduce inflammation and possibly to preserve functionality prior to transplant, as reported for allogeneic stem cells (22). Third, following the successful transplant, PIF was administered (in a physiological dose range) for three weeks on and one week off for three consecutive months to regulate immune response, decrease vascular inflammation and oxidative stress to support transplant acceptance without the need of an immune suppressor (22-24). It is important to differentiate PIF's action from anti-inflammatory agent, since inflammation is necessary for proper healing. This is supported since PIF administration healed the laparotomy scar completely and restored hair growth. Whether the graft's local acceptance was aided is unknown at present.

We designed the study with a baboon model due to its similarity to humans in anatomy and menstruation pattern. Furthermore, its cyclic hormonal activities can be very easily assessed by perineal inflation (26, 53-56). Studies on uterus and kidney transplantation indicate that baboons have a similar rejection pattern as in humans (26, 57), enabling the current study on AOT. Monitoring rejection following AOT is difficult, since organ biopsy cannot be performed due to the small size and location of the grafts. Herein, rejection was assessed by changes of clinical signs (skin, perineum, vital signs, behavior, and weight) as well as in laboratory tests (serum markers for liver and kidney function). Since the goal of this study was restoration of ovarian function and fertility, we did not test systemic immunological markers. Instead, we focused on monitoring endocrine function with serum FSH and E2 levels and return of menstruation as a proof of successful immunologic acceptance.

In addition to transplant acceptance without side effects, we demonstrated return of ovarian function after AOT in one animal, evidenced by increased E2, perineal inflation, and menstruation, albeit only for a short period. The histology of recovered ovarian grafts (from both animals) at the time of autopsy revealed significant loss of follicles. Mechanisms involved in follicular loss after transplantation are complex (but most important factor can be ischemia (58)), which could further be augmented by the silent immune rejection. However, the gross morphology indicated that there were corpora lutea thus ovarian function at least temporarily was active. This is also shown by the presence of primordial follicles based on histological evaluation which persisted even for nine months post-surgery.

In our view, live donors will be the main source of donated ovarian grafts for future human AOT due to paired character of ovaries and the relatively low-risk laparoscopic surgery to perform unilateral oophorectomy. The use of posthumous organ donors for AOT could also be a viable option, but it may raise ethical concerns (59). To improve the efficacy and prevent misuse of this technology, it may be necessary to establish ovarian donor selection criteria such as age and ovarian reserve.

In summary, we demonstrated the first successful ovarian allotransplantation using PIF monotherapy in a non-human primate model, which resulted in transplant acceptance and restoration of ovarian function without the use of any immunosuppression or any other treatment. The study opens the door to using PIF as acceptance and maintenance regimen in organ transplantation. Further studies are required to assess the efficacy of AOT using PIF treatment in women.
1. Donnez, J., et al. Restoration of ovarian activity and pregnancy after transplantation of cryopreserved ovarian tissue: a review of 60 cases of reimplantation. Fertil Steril, 2013. 99(6): p. 1503-13.
2. Scott, J.R., et al. Pregnancy after tubo-ovarian transplantation. Obstet Gynecol, 1987. 70(2): p. 229-34.
3. Carmona, F., et al. Ovarian function, tubal viability and pregnancy after tubo-ovarian transplantation in the rabbit. Hum Reprod, 1993. 8(6): p. 929-31.
4. Kim, S.S., et al. The future of human ovarian cryopreservation and transplantation: fertility and beyond. Fertil Steril, 2001. 75(6): p. 1049-56.
5. Denton, M.D., et al. Sayegh, Immunosuppressive strategies in transplantation. Lancet, 1999. 353(9158): p. 1083-91.
6. Mhatre, P., et al. Ovarian transplant: a new frontier. Transplant Proc, 2005. 37(2): p. 1396-8.
7. Mhatre, P. and J. Mhatre. Orthotopic ovarian transplant--review and three surgical techniques. Pediatr Transplant, 2006. 10(7): p. 782-7.
8. Silber, S.J., et al. Ovarian transplantation between monozygotic twins discordant for premature ovarian failure. N Engl J Med, 2005. 353(1): p. 58-63.
9. Donnez, J., et al. Allograft of ovarian cortex between two genetically non-identical sisters: case report. Hum Reprod, 2007. 22(10): p. 2653-9.
10. Silber, S.J. and R.G. Gosden. Ovarian transplantation in a series of monozygotic twins discordant for ovarian failure. N Engl J Med, 2007. 356(13): p. 1382-4.
11. Azar, Y., et al. Preimplantation factor reduces graft-versus-host disease by regulating immune response and lowering oxidative stress (murine model). Biol Blood Marrow Transplant, 2013. 19(4): p. 519-28.
12. Shainer, R., et al. Immune Regulation and Oxidative Stress Reduction by Preimplantation Factor following Syngeneic or Allogeneic Bone Marrow Transplantation. Conference Papers in Medicine, 2013. 2013(Article ID 718031): p. 1-8.
13. Barnea, E.R., et al. PreImplantation Factor (PIF) orchestrates systemic antiinflammatory response by immune cells: effect on peripheral blood mononuclear cells. Am J Obstet Gynecol, 2012. 207(4): p. 313 e1-11.
14. Barnea, E.R., et al. PIF direct immune regulation: Blocks mitogen-activated PBMCs proliferation, promotes T2/T1 bias, independent of Ca. Immunobiology, 2015.
15. Barnea, E.R., et al. PreImplantation factor (PIF*) regulates systemic immunity and targets protective regulatory and cytoskeleton proteins. Immunobiology, 2016.
16. Mueller, M., et al. PreImplantation factor promotes neuroprotection by targeting microRNA let-7. Proc Natl Acad Sci U S A, 2014. 111(38): p. 13882-7.
17. Mueller, M., et al. PreImplantation Factor Bolsters Neuroprotection via Modulating Q10 Protein Kinase A and Protein Kinase C Signaling. Cell Death Differ, 2015. DOI: 10.1038/cdd.2015.55.
18. Barnea, E.R., et al. Immune regulatory and neuroprotective properties of preimplantation factor: From newborn to adult. Pharmacol Ther, 2015. 156: p. 10-25.
19. Enskog, A., et al. Uterus transplantation in the baboon: methodology and long-term function after auto-transplantation. Hum Reprod, 2010. 25(8): p. 1980-7.
20. Bauer, C. The baboon (Papio sp.) as a model for female reproduction studies. Contraception, 2015. 92(2): p. 120-3.
21. Donnez, J., et al. Livebirth after orthotopic transplantation of cryopreserved ovarian tissue. Lancet, 2004. 364(9443): p. 1405-10.
22. Donnez, J. and M.M. Dolmans. Ovarian cortex transplantation: 60 reported live births brings the success and worldwide expansion of the technique towards routine clinical practice. J Assist Reprod Genet, 2015. 32(8): p. 1167-70.
23. Brannstrom, M., et al. Livebirth after uterus transplantation. Lancet, 2015. 385(9968): p. 607-16.
24. Johannesson, L., et al. Uterus transplantation trial: 1-year outcome. Fertil Steril, 2015. 103(1): p. 199-204.
25. Yin, H., et al. Transplantation of intact rat gonads using vascular anastomosis: effects of cryopreservation, ischaemia and genotype. Hum Reprod, 2003. 18(6): p. 1165-72.
26. Gosden, R.G. Survival of ovarian allografts in an experimental animal model. Pediatr Transplant, 2007. 11(6): p. 628-33.
27. Meraz, M.M., et al. Restoration of endocrine function and fertility with orthotopic tubal-ovarian allotransplant as the anatomical-functional unit in rabbits. J Invest Surg, 2008. 21(6): p. 348-59.
28. Lin, Y.H., et al. Evaluating the effects of immunosuppression by in-vivo bioluminescence imaging after allotransplantation of ovarian grafts. Reprod Biomed Online, 2011. 22(2): p. 220-7.
29. Chen, C.H., et al. Tracking the rejection and survival of mouse ovarian iso- and allografts in vivo with bioluminescent imaging. Reproduction, 2010. 140(1): p. 105-12.
30. Robertson, J.A. Ethical issues in ovarian transplantation and donation. Fertil Steril, 2000. 73(3): p. 443-6.
31. Almogi-Hazan, O., et al. The Role of Nitric Oxide Toxicity and Oxidative Stress in Graft vs Host Disease, in Oxidative Stress: Causes, Role in Diseases and Biological Effects. 2014, Nova Science Publishers, Inc.
32. Weiss, L., et al. Preimplantation factor (PIF) analog prevents type I diabetes mellitus (TIDM) development by preserving pancreatic function in NOD mice. Endocrine, 2011. 40(1): p. 41-54.
33. Weiss, L., et al. Preimplantation factor (PIF*) reverses neuroinflammation while promoting neural repair in EAE model. J Neurol Sci, 2012. 312(1-2): p. 146-57.
34. Chen, Y.C., et al. PreImplantation factor prevents atherosclerosis via its immunomodulatory effects without affecting serum lipids. Thromb Haemost, 2016. 115(5).
35. Barnea, E.R., et al. Preimplantation factor (PIF) promoting role in embryo implantation: increases endometrial integrin-alpha2beta3, amphiregulin and epiregulin while reducing betacellulin expression via MAPK in decidua. Reprod Biol Endocrinol, 2012. 10: p. 50.
36. Roussev, R.G., et al. Preimplantation factor inhibits circulating natural killer cell cytotoxicity and reduces CD69 expression: implications for recurrent pregnancy loss therapy. Reprod Biomed Online, 2013. 26(1): p. 79-87.
37. Lopez-Cabrera, M., et al. Molecular cloning, expression, and chromosomal localization of the human earliest lymphocyte activation antigen AIM/CD69, a new member of the C-type animal lectin superfamily of signaltransmitting receptors. J Exp Med, 1993. 178(2): p. 537-47.
38. Schowengerdt, K.O., et al. Increased expression of the lymphocyte early activation marker CD69 in peripheral blood correlates with histologic evidence of cardiac allograft rejection. Transplantation, 2000. 69(10): p. 2102-7.
39. Posselt, A.M., et al. CD69 expression on peripheral CD8 T cells correlates with acute rejection in renal transplant recipients. Transplantation, 2003. 76(1): p. 190-5.
40. Maclaran, K. and N. Panay. Premature ovarian failure. J Fam Plann Reprod Health Care, 2011. 37(1): p. 35-42.
41. van der Schouw, Y.T., et al. Age at menopause as a risk factor for cardiovascular mortality. Lancet, 1996. 347(9003): p. 714-8.
42. Rocca, W.A., et al. Survival patterns after oophorectomy in premenopausal women: a population-based cohort study. Lancet Oncol, 2006. 7(10): p. 821-8.
43. Popat, V.B., et al. Bone mineral density in estrogen-deficient young women. J Clin Endocrinol Metab, 2009. 94(7): p. 2277-83.
44. Schmidt, P.J., et al. Depression in women with spontaneous 46, XX primary ovarian insufficiency. J Clin Endocrinol Metab, 2011. 96(2): p. E278-87.
45. Quigley, C.A., et al. Effects of low-dose estrogen replacement during childhood on pubertal development and gonadotropin concentrations in patients with Turner syndrome: results of a randomized, double-blind, placebocontrolled clinical trial. J Clin Endocrinol Metab, 2014. 99(9): p. E1754-64.
46. Poirot, C., et al. Induction of puberty by autograft of cryopreserved ovarian tissue. Lancet, 2012. 379(9815): p. 588.
47. Ernst, E., et al. Case report: stimulation of puberty in a girl with chemo- and radiation therapy induced ovarian failure by transplantation of a small part of her frozen/thawed ovarian tissue. Eur J Cancer, 2013. 49(4): p. 911-4.
48. Kim, S.S. Assessment of long term endocrine function after transplantation of frozen-thawed human ovarian tissue to the heterotopic site: 10 year longitudinal follow-up study. J Assist Reprod Genet, 2012. 29(6): p. 489-93.
49. Jensen, A.K., et al. Outcomes of transplantations of cryopreserved ovarian tissue to 41 women in Denmark. Hum Reprod, 2015. 30(12): p. 2838-45.
50. Committee, I.P., et al. Recommendations for fertility preservation in patients with lymphoma, leukemia, and breast cancer. J Assist Reprod Genet, 2012. 29(6): p. 465-8.
51. Imbert, R., et al. Safety and usefulness of cryopreservation of ovarian tissue to preserve fertility: a 12-year retrospective analysis. Hum Reprod, 2014. 29(9): p. 1931-40.
52. Khan, Z., et al. Unilateral oophorectomy results in compensatory follicular recruitment in the remaining ovary at time of ovarian stimulation for in vitro fertilization. Fertil Steril, 2014. 101(3): p. 722-7.
53. Bjelland, E.K., et al. Is unilateral oophorectomy associated with age at menopause? A population study (the HUNT2 Survey). Hum Reprod, 2014. 29(4): p. 835-41.
54. Stevens, V.C. Some reproductive studies in the baboon. Hum Reprod Update, 1997. 3(6): p. 533-40.
55. D'Hooghe, T., et al. The Baboon as a Nonhuman Primate Model for the Study of Human Reproduction, in Gynecol Obstet Invest. 2003. p. 1-60.
56. Amorim, C.A., et al. Successful vitrification and autografting of baboon (Papio anubis) ovarian tissue. Hum Reprod, 2013. 28(8): p. 2146-56.
57. Johannesson, L., et al. Preclinical report on allogeneic uterus transplantation in non-human primates. Hum Reprod, 2013. 28(1): p. 189-98.
58. Nyachieo, A., et al. Ovarian tissue cryopreservation by vitrification in olive baboons (papio anubis): a pilot study. Gynecol Obstet Invest, 2013. 75(3): p. 157-62.
59. Todo, S., et al. Renal transplantation in baboons under FK 506. Surgery, 1989. 106(2): p. 444-50; discussion 450-1.

### Example 4

### PIF promotes insulin producing cells survival and proliferation

The availability of pancreatic islets for transplantation is in short supply. Currently it is difficult to achieve a long term survival by transplanting human or porcine islet cells. Another potential source are insulinoma producing cells (INS-1) which mimic primary islet cells in several aspects and therefore are good surrogates to understand whether PIF can regulate their function. In this study we examined the effect of PIF on these cells in culture. There is a delicate balance once cells are placed in culture. Namely, the longer the cultivation period the more cell survival is affected. By using PIF in BAC cells this aspect was already shown to improve (Example 3). Herein the ratio of cells viability, apoptosis and proliferation were analyzed using regression analysis. The data shows a strong correlation between cell viability and length of culture (Fig. 28A). Further, dependence of cells viability correlates with the degree of apoptosis vs. cells proliferation (Fig. 28B). Finally, apoptosis and proliferation were also significantly correlated (Fig. 28C). When PIF's effect within the parameters of viability, apoptosis and proliferation were examined (Fig. 29A,B,C), respectively, the following picture emerged: At 48 hours of culture PIF increased cells viability but as expected the degree of apoptosis increased as well. The beneficial effect was accomplished through the elimination of non viable cells through autophagy, enabling surviving cells to better function at 48 hours. When the culture was prolonged to 72 hours, ten fold lower PIF concentrations were needed to achieve close to 50% increase in proliferation. At this dose however, at 48 hours, minimal effect was noted on the viability/apoptosis ratio. In contrast, at the high dose of PIF at 72 hours, there was no effect on proliferation, reflecting cellular exhaustion. This implies that a fine balance between dose and islet cell line functionality can be driven. These are clearly translatable to improve islet function in preparation for transplantation.

### Methods

Ins-1 cells of passage 24 were used in the experiments. For viability, apoptosis and proliferation assay cells were seeded in 96 well plates (1×10⁴ cells per well, sixplicate). PIF was used in three concentrations: 0.01 µg/ml; 0.1 µg/ml and 1 µg/ml. Medium, containing substrates, was changed every day. Viability was assessed using XTT Cell Proliferation Assay (Roche). Proliferation was measured using BrdU Cell Proliferation Assay (Roche). Apoptosis was assayed by determination of caspase 3/7 activity using Caspase-Glo 3/7 Assay (Promega). Received results were analyzed by methods of regression analysis for evaluation of the processes, occurring in the tested models.

For evaluation of the effects of PIF on cells Wilcoxon signed-rank test for related samples and Spearman's rank correlation and Student's t-test were used.
Aspects and features of the present disclosure are set out in the following numbered clauses which contain the subject matter of the claims of the parent application as filed.
1. A method of modulating or maintaining endocrine function in a subject comprising administering to the subject a therapeutically effective amount of a Preimplantation Factor (PIF) peptide, compositions thereof, mimetics thereof, pharmaceutically acceptable salts thereof, or combinations thereof of any of the aforementioned.
2. The method of clause 1, wherein the endocrine function is endocrine function from ovarian tissue.
3. The method of clause 1, wherein the therapeutically effective amount of a PIF peptide is the only therapeutic compound administered to the subject.
4. The method of clause 1, wherein the therapeutically effective amount is from about 0.1 mg/kg to about 5.0 mg/kg of subject.
5. The method of clause 1, wherein the method is free of administration of any immunomodulators.
6. The method of clause 1, wherein the method further comprises administration of a therapeutically effective dose of one or a plurality of immunomodulators, analgesics, anti-inflammatory compounds, alpha-adrenergic agonists, antiarrhythmic compounds, anesthetic compounds, or combinations thereof.
7. The method of clause 1, wherein the method further comprises administration of a therapeutically effective dose of one or a plurality of immunomodulators chosen from: azficel-T, etanercept, glatiramer acetate, lenalidomide, mifamurtide, pimecrolimus, thymalfasin, tinocordin, 6-mercaptopurine, anakinra, C1 esterase inhibitor recombinant, C1 esterase inhibitor human, dimethyl fumarate, ecallantide, fingolimod, glatiramer, icatibant, immunoglobulins, interferon alfa-n3, interferon alfacon-1, interferon beta-1a, interferon beta-Ib, mercaptopurine, peginterferon beta-1a, rilonacept, siltuximab, and tocilizumab.
8. The method of clause 1, wherein the administering comprises one or more of the following modes of administration: intravenous, subcutaneous, intraperitoneal, intranasal, topical, intradermal, intramucosal, sublingual, oral, intravaginal, intramuscular, intracavernous, intraocular, intrarectal, into a sinus, gastrointestinal, intraductal, intrathecal, subdural, extradural, intraventricular, intrapulmonary, into an abscess, intraarticular, into a bursa, subpericardial, into an axilla, intrauterine, into the pleural space, transmucosal, and transdermal.
9. The method of clause 1, wherein the method is free of exposing the subject to x-rays or depletion of T-cell counts.
10. The method of clause 1, wherein endocrine function is maintained for more than about 30, 40, 50, 60, 70, 80, 90 or 100 days.
11. The method of clause 1, wherein the step of administering comprises administration of a therapeutically effective amount of a PIF peptide or salt thereof.
12. The method of clause 1, wherein the step of administering a therapeutically effective amount of a PIF peptide or salt thereof is preceded by transplanting or engrafting endocrine tissue from a tissue donor into the subject, wherein the subject is deficient in a normally functioning endocrine tissue or in need of a transplant of endocrine tissue.
13. The method of clause 12, wherein the endocrine tissue is ovarian tissue.
14. The method of clause 12, wherein the endocrine tissue is xenotransplanted endocrine tissue.
15. The method of clause 14, wherein the xenotransplanted endocrine tissue is from culture cells from a species that is not from a species of the subject.
16. A method of enhancing endocrine function in a subject comprising administering to the subject a therapeutically effective amount of a PIF peptide, compositions thereof, mimetics thereof, pharmaceutically acceptable salts thereof, or combinations thereof.
17. The method of clause 16, wherein the endocrine function is ovarian function.
18. The method of clause 16, wherein the therapeutically effective amount of a PIF peptide is the only therapeutic compound administered to the subject.
19. The method of clause 16, wherein the therapeutically effective amount is from about 0.1 mg/kg to about 5.0 mg/kg of subject.
20. The method of clause 16, wherein the method is free of administration of any immunomodulators.
21. The method of clause 16, wherein the method further comprises administration of a therapeutically effective dose of one or a plurality of immunomodulators, analgesics, anti-inflammatory compounds, alpha-adrenergic agonists, antiarrhythmic compounds, anesthetic compounds, or combinations thereof.
22. The method of clause 16, wherein the method further comprises administration of one or a plurality of immunomodulators chosen from: azficel-T, etanercept, glatiramer acetate, lenalidomide, mifamurtide, pimecrolimus, thymalfasin, tinocordin, 6-mercaptopurine, anakinra, C1 esterase inhibitor recombinant, C1 esterase inhibitor human, dimethyl fumarate, ecallantide, fingolimod, glatiramer, icatibant, immunoglobulins, interferon alfa-n3, interferon alfacon-1, interferon beta-1a, interferon beta-Ib, mercaptopurine, peginterferon beta-1a, rilonacept, siltuximab, and tocilizumab.
23. The method of clause 16, wherein the administering comprises one or more of the following modes of administration: intravenous, subcutaneous, intraperitoneal, intranasal, topical, intradermal, intramucosal, sublingual, oral, intravaginal, intramuscular, intracavernous, intraocular, intrarectal, into a sinus, gastrointestinal, intraductal, intrathecal, subdural, extradural, intraventricular, intrapulmonary, into an abscess, intraarticular, into a bursa, subpericardial, into an axilla, intrauterine, into the pleural space, transmucosal, and transdermal.
24. The method of clause 16, wherein the method is free of exposing the subject to x-rays or depletion of T-cell counts.
25. The method of clause 16, wherein endocrine function is enhanced for more than about 30, 40, 50, 60, 70, 80, 90 or 100 days.
26. A method of restoring endocrine function in a subject comprising administering to the subject a therapeutically effective amount of a PIF peptide, compositions thereof, mimetics thereof, pharmaceutically acceptable salts thereof, or combinations thereof.
27. The method of clause 26, wherein the endocrine function is ovarian function.
28. The method of clause 26, wherein the therapeutically effective amount of a PIF peptide is the only therapeutic compound administered to the subject.
29. The method of clause 26, wherein the therapeutically effective amount is from about 0.1 mg/kg to about 5.0 mg/kg of subject.
30. The method of clause 26, wherein the method is free of administration of any immunomodulators.
31. The method of clause 26, wherein the method further comprises administration of one or a plurality of immunomodulators, analgesics, anti-inflammatory compounds, alpha-adrenergic agonists, antiarrhythmic compounds, anesthetic compounds, or combinations thereof.
32. The method of clause 26, wherein the method further comprises administration of one or a plurality of immunomodulators chosen from: azficel-T, etanercept, glatiramer acetate, lenalidomide, mifamurtide, pimecrolimus, thymalfasin, tinocordin, 6-mercaptopurine, anakinra, C1 esterase inhibitor recombinant, C1 esterase inhibitor human, dimethyl fumarate, ecallantide, fingolimod, glatiramer, icatibant, immunoglobulins, interferon alfa-n3, interferon alfacon-1, interferon beta-1a, interferon beta-Ib, mercaptopurine, peginterferon beta-1a, rilonacept, siltuximab, and tocilizumab.
33. The method of clause 26, wherein the administering comprises one or more of the following modes of administration: intravenous, subcutaneous, intraperitoneal, intranasal, topical, intradermal, intramucosal, sublingual, oral, intravaginal, intramuscular, intracavernous, intraocular, intrarectal, into a sinus, gastrointestinal, intraductal, intrathecal, subdural, extradural, intraventricular, intrapulmonary, into an abscess, intraarticular, into a bursa, subpericardial, into an axilla, intrauterine, into the pleural space, transmucosal, and transdermal.
34. The method of clause 26, wherein the method is free of exposing the subject to x-rays or depletion of T-cell counts.
35. The method of clause 26, wherein endocrine function is restored for more than about 30, 40, 50, 60, 70, 80, 90 or 100 days.
36. A method of xenotransplantation to a subject, the method comprising: culturing a cell from a non-human animal in a medium comprising a PIF peptide, and transplanting the cell into the subject.
37. The method of clause 36, wherein the cell is an endocrine cell.
38. The method of clause 36, wherein the cell is an ovarian cell.
39. The method of clause 37, wherein the cell is not a pancreatic islet cell.
40. The method of clause 37, wherein the cell is not an adrenal cell.
41. The method of clause 36, further comprising restoring endocrine function in the subject after allowing the transplanted cell to "grow" in the subject for a time period sufficient to restore endocrine function to normal level.
42. A method of increasing the likelihood of success of a xenotransplantation in a subject comprising:
   i. administering to a nonhuman animal a therapeutically effective amount of a PIF peptide, compositions thereof, mimetics thereof, pharmaceutically acceptable salts thereof, or combinations thereof; and
   ii. transplanting a cell, tissue, or organ from the nonhuman animal into the subject.
43. The method of clause 42, wherein the cell, tissue, or organ from the non-human animal comprises an endocrine cell.
44. The method of clause 42, wherein the cell, tissue, or organ from the nonhuman animal comprises an ovarian cell.
45. The method of clause 42, further comprising a step of administering a therapeutically effective amount of a PIF peptide, compositions thereof, mimetics thereof, pharmaceutically acceptable salts thereof, or combinations thereof, once, twice, or three times in a 30 day period.
46. A method of restoring endocrine tissue function after transplantation of the endocrine tissue in a subject comprising administering to the subject a therapeutically effective amount of a PIF peptide, compositions thereof, mimetics thereof, pharmaceutically acceptable salts thereof, or combinations thereof.
47. The method of clause 46, wherein the endocrine tissue comprises an ovarian cell.
48. The method of clause 46, wherein the endocrine tissue is free of pancreatic islet cells.
49. The method of clause 46, wherein the endocrine tissue is free of adrenal cells.
50. The method of clause 46, wherein the endocrine tissue is an organ or part of an organ or a graft of an organ.
51. The method of clause 46, wherein the endocrine tissue comprises one or a plurality of cells optionally grown *in vitro.*
52. The method of clause 46, wherein the therapeutically effective amount of a PIF peptide is the only therapeutic compound administered to the subject.
53. The method of clause 46, further comprising a step of contacting donor tissue with a therapeutically effective amount of a PIF peptide simultaneously or prior to transplantation of the tissue.
54. A method of modulating expression of CYP17A1 or IL-10 in one or a plurality of adrenocortical cells comprising contacting the adrenocortical cell with an effective amount of a PIF peptide, compositions thereof, mimetics thereof, pharmaceutically acceptable salts thereof, or combinations thereof.
55. The method of clause 54, wherein the one or plurality of adrenocortical cells is in a culture of cells *in vitro.*
56. The method of clause 54, wherein the one or plurality of adrenocortical cells are in a culture of cells ex *vivo.*
57. The method of clause 54, wherein the one or plurality of adrenocortical cells are in a subject in need of a transplant of adrenocortical cells or in a subject having received one or a plurality of donor adrenocortical cells.
58. The method of clause 54, wherein the one or plurality of adrenocortical cells are contacted with the PIF peptide, compositions thereof, mimetics thereof, pharmaceutically acceptable salts thereof, or combinations thereof for a time period sufficient to induce expression of CYP17A1 or IL-10 in the cell prior to or simultaneously with transplant into a transplant recipient.
59. A method of inducing transplant tolerance of a semi-allogeneic and/or xeno-embryo in a subject by increasing expression of HLA-Class I molecules in the subject or on the embryo to an amount sufficient to increase the likelihood of transplant acceptance of the embryo as compared to the levels of HLA Class I molecules on an embryo or in a subject not treated with a PIF peptide, compositions thereof, mimetics thereof, pharmaceutically acceptable salts thereof, or combinations thereof; the method comprising contacting the embryo and/or the subject with a therapeutically effective amount of a PIF peptide, compositions thereof, mimetics thereof, pharmaceutically acceptable salts thereof, or combinations thereof.
60. The method of clause 59, wherein the method comprises contacting the embryo with a therapeutically effective amount of a PIF peptide, compositions thereof, mimetics thereof, pharmaceutically acceptable salts thereof, or combinations thereof.
61. The method of clause 59, wherein the method comprises administering a therapeutically effective amount of a PIF peptide, compositions thereof, mimetics thereof, pharmaceutically acceptable salts thereof, or combinations thereof to the subject.
62. The method of clause 59, wherein the method comprises administering a therapeutically effective amount of a PIF peptide, compositions thereof, mimetics thereof, pharmaceutically acceptable salts thereof, or combinations thereof to the subject prior to or simultaneously with transplantation of the embryo.
63. The method of clause 59, wherein the method comprises contacting the embryo with a therapeutically effective amount of a PIF peptide, compositions thereof, mimetics thereof, pharmaceutically acceptable salts thereof, or combinations thereof prior to or simultaneously with transplantation into the subject.
64. The method of clause 59, wherein the HLA-Class I molecules comprise HLA-G.
65. The method of clause 59, wherein the HLA-Class I molecules comprise HLA-C.
66. The method of clause 59, wherein the HLA-Class I molecules comprise HLA-E.
67. The method of clause 59, wherein the HLA-Class I molecules comprise HLA-F.
68. The method of clause 59, wherein the method is free of a step of administering to the subject and/or free of contacting the embryo with an active agent other than PIF peptide, compositions thereof, mimetics thereof, pharmaceutically acceptable salts thereof, or a combinations thereof.
69. A method of inducing transplant tolerance of one or a plurality of donor semi-allogeneic cell and/or cells derived from a species other than the transplant recipient in a recipient subject by increasing expression of HLA-Class I molecules in the subject or on the donor cells to an amount sufficient to increase the likelihood of transplant acceptance of the cells as compared to the levels of HLA Class I molecules on donor cells or in the subject untreated with a PIF peptide, compositions thereof, mimetics thereof, pharmaceutically acceptable salts thereof, or combinations thereof; the method comprising contacting the one or plurality of donor cells and/or the subject with a therapeutically effective amount of a PIF peptide, compositions thereof, mimetics thereof, pharmaceutically acceptable salts thereof, or combinations thereof.
70. The method of clause 69, wherein the method comprises contacting the one or plurality of donor cells with a therapeutically effective amount of a PIF peptide, compositions thereof, mimetics thereof, pharmaceutically acceptable salts thereof, or combinations thereof.
71. The method of clause 69, wherein the method comprises administering a therapeutically effective amount of a PIF peptide, compositions thereof, mimetics thereof, pharmaceutically acceptable salts thereof, or combinations thereof to the subject.
72. The method of clause 69, wherein the method comprises administering a therapeutically effective amount of a PIF peptide, compositions thereof, mimetics thereof, pharmaceutically acceptable salts thereof, or combinations thereof to the subject prior to or simultaneously with transplantation of the one or plurality of donor cells.
73. The method of clause 69, wherein the method comprises contacting the one or plurality of donor cells with a therapeutically effective amount of a PIF peptide, compositions thereof, mimetics thereof, pharmaceutically acceptable salts thereof, or combinations thereof prior to or simultaneously with transplantation of the donor cell or donor cells into the subject.
74. The method of clause 69, wherein the HLA-Class I molecules comprise HLA-G.
75. The method of clause 69, wherein the HLA-Class I molecules comprise HLA-C.
76. The method of clause 69, wherein the HLA-Class I molecules comprise HLA-E.
77. The method of clause 69, wherein the HLA-Class I molecules comprise HLA-F.
78. The method of clause 69, wherein the method is free of a step of administering to the subject and/or free of contacting the one or plurality of donor cells with an active agent other than PIF peptide, compositions thereof, mimetics thereof, pharmaceutically acceptable salts thereof, or a combinations thereof.
79. A method of inducing expression of an HLA-Class I molecule in a subject and/or in a donor tissue, the method comprising contacting the donor tissue and/or administering to the subject a therapeutically effective amount of a PIF peptide, compositions thereof, mimetics thereof, pharmaceutically acceptable salts thereof, or combinations thereof.
80. The method of clause 79, wherein the HLA-Class I molecules comprise HLA-G.
81. The method of clause 79, wherein the HLA-Class I molecules comprise HLA-C.
82. The method of clause 79, wherein the HLA-Class I molecules comprise HLA-E.
83. The method of clause 79, wherein the HLA-Class I molecules comprise HLA-F.
84. The method of clause 79, wherein the method is free of a step of administering to the subject an active agent other than PIF peptide, compositions thereof, mimetics thereof, pharmaceutically acceptable salts thereof, or a combinations thereof.
85. The method of clause 79 further comprising administering to the subject one or a plurality of steroids in a therapeutically effective amount.
86. The method of clause 85, wherein the steroid is dexamethasone.
87. The method of clause 85, wherein the steroid is progesterone.
88. A method of restoring menstruation in a mammal in need of restoration comprising administering to the mammal a therapeutically effective amount of a PIF peptide, compositions thereof, mimetics thereof, pharmaceutically acceptable salts thereof, or combinations thereof.
89. The method of clause 88, wherein the mammal in need of restoration has an endocrine tissue dysfunction that renders the mammal unable to menstruate in the absence of a therapy.
90. The method of clause 88, wherein the mammal in need of restoration has an endocrine tissue dysfunction that renders the mammal unable to menstruate in the absence of a therapy.
91. The method of clause 88, wherein the mammal in need of restoration has an endocrine tissue dysfunction that renders the mammal unable to menstruate normally in the absence of a therapy.
92. The method of clause 88 further comprising a step of transplanting endocrine tissue to the mammal.
93. The method of clause 92, wherein the endocrine tissue is ovarian tissue.
94. The method of clause 88, wherein the endocrine tissue is free of pancreatic islet cells or adrenal cells.
95. The method of clause 88, wherein the method is free of a step of administering to the subject an active agent other than PIF peptide, compositions thereof, mimetics thereof, pharmaceutically acceptable salts thereof, or a combinations thereof.
96. The method of clause 92 further comprising a step of isolating donor endocrine tissue prior to transplanting the endocrine tissue in the mammal.
97. The method of 96 further comprising a step of culturing donor endocrine tissue prior to transplanting the endocrine tissue in the mammal.
98. The method of 92 further comprising a step of contacting the donor endocrine tissue with an effective amount of PIF peptide, compositions thereof, mimetics thereof, pharmaceutically acceptable salts thereof, or a combinations thereof prior to transplanting the donor endocrine tissue in the mammal.
99. A pharmaceutical composition comprising:
   (i) a therapeutically effective amount of a PIF peptide, compositions thereof, mimetics thereof, pharmaceutically acceptable salts thereof, or a combinations thereof;
   (ii) a therapeutically effective amount of a steroid; and
   (iii) a pharmaceutically acceptable carrier.
100. A method of promoting or enhancing wound healing in a subject comprising: administering to the subject a pharmaceutical composition comprising a therapeutically effective amount of PIF peptide, compositions thereof, mimetics thereof, pharmaceutically acceptable salts thereof, or a combination thereof.
101. The method of clause 100, wherein the step of administering results in at least one of accelerated wound closure, rapid re-epithelialization, improved angiogenesis, improved tissue remodeling relative to untreated controls and a decrease in local inflammation relative to untreated controls.
102. The method of clause 100, wherein the subject has undergone a surgical procedure.
103. The method of clause 100, wherein the step of administering comprises one or more of the following modes of administration: intravenous, subcutaneous, intraperitoneal, intranasal, topical, intradermal, intramucosal, sublingual, oral, intravaginal, intramuscular, intracavernous, intraocular, intrarectal, into a sinus, gastrointestinal, intraductal, intrathecal, subdural, extradural, intraventricular, intrapulmonary, into an abscess, intraarticular, into a bursa, subpericardial, into an axilla, intrauterine, into the pleural space, transmucosal, and transdermal administration of the pharmaceutical composition.
104. The method of clause 100, wherein the method is free of a step of administering to the subject an active agent other than PIF peptide, compositions thereof, mimetics thereof, pharmaceutically acceptable salts thereof, or a combinations thereof.
105. A method of potentiating the effects of a steroid as a treatment in a subject in need thereof, the method comprising: (i) administering to the subject a pharmaceutical composition comprising a therapeutically effective amount of PIF peptide, compositions thereof, mimetics thereof, pharmaceutically acceptable salts thereof, or a combination thereof; and (ii) administering a therapeitucally effective amount of steroid before, during or after step (i).
106. The method of clause 105, wherein the steroid is dexamethasone.

## Claims

1. A therapeutically effective amount of a Preimplantation Factor (PIF) peptide, compositions thereof, mimetics thereof, pharmaceutically acceptable salts thereof, or combinations thereof of any of the aforementioned, for use in a method of modulating or maintaining endocrine function in a subject.

2. A therapeutically effective amount of a PIF peptide, compositions thereof, mimetics thereof, pharmaceutically acceptable salts thereof, or combinations thereof, for use in a method of enhancing endocrine function in a subject.

3. A therapeutically effective amount of a PIF peptide, compositions thereof, mimetics thereof, pharmaceutically acceptable salts thereof, or combinations thereof, for use in a method of restoring endocrine function in a subject.

4. A PIF peptide for use in a method of xenotransplantation to a subject, the method comprising: culturing a cell from a non-human animal in a medium comprising a PIF peptide, and transplanting the cell into the subject.

5. A therapeutically effective amount of a PIF peptide, compositions thereof, mimetics thereof, pharmaceutically acceptable salts thereof, or combinations thereof, for use in a method of increasing the likelihood of success of a xenotransplantation in a subject, the method comprising:
i. administering to a nonhuman animal a therapeutically effective amount of a PIF peptide, compositions thereof, mimetics thereof, pharmaceutically acceptable salts thereof, or combinations thereof; and
ii. transplanting a cell, tissue, or organ from the nonhuman animal into the subject.

6. A therapeutically effective amount of a PIF peptide, compositions thereof, mimetics thereof, pharmaceutically acceptable salts thereof, or combinations thereof, for use in a method of restoring endocrine tissue function after transplantation of the endocrine tissue in a subject.

7. A effective amount of a PIF peptide, compositions thereof, mimetics thereof, pharmaceutically acceptable salts thereof, or combinations thereof, for use in a method of modulating expression of CYP17A1 or IL-10 in one or a plurality of adrenocortical cells comprising contacting the adrenocortical cell.

8. A a therapeutically effective amount of a PIF peptide, compositions thereof, mimetics thereof, pharmaceutically acceptable salts thereof, or combinations thereof, for use in a method of inducing transplant tolerance of a semi-allogeneic and/or xeno-embryo in a subject by increasing expression of HLA-Class I molecules in the subject or on the embryo to an amount sufficient to increase the likelihood of transplant acceptance of the embryo as compared to the levels of HLA Class I molecules on an embryo or in a subject not treated with a PIF peptide, compositions thereof, mimetics thereof, pharmaceutically acceptable salts thereof, or combinations thereof; the method comprising contacting the embryo and/or the subject with a therapeutically effective amount of a PIF peptide, compositions thereof, mimetics thereof, pharmaceutically acceptable salts thereof, or combinations thereof.

9. A PIF peptide, compositions thereof, mimetics thereof, pharmaceutically acceptable salts thereof, or combinations thereof, for use in a method of inducing transplant tolerance of one or a plurality of donor semi-allogeneic cell and/or cells derived from a species other than the transplant recipient in a recipient subject by increasing expression of HLA-Class I molecules in the subject or on the donor cells to an amount sufficient to increase the likelihood of transplant acceptance of the cells as compared to the levels of HLA Class I molecules on donor cells or in the subject untreated with a PIF peptide, compositions thereof, mimetics thereof, pharmaceutically acceptable salts thereof, or combinations thereof; the method comprising contacting the one or plurality of donor cells and/or the subject with a therapeutically effective amount of a PIF peptide, compositions thereof, mimetics thereof, pharmaceutically acceptable salts thereof, or combinations thereof.

10. A therapeutically effective amount of a PIF peptide, compositions thereof, mimetics thereof, pharmaceutically acceptable salts thereof, or combinations thereof, for use in a method of inducing expression of an HLA-Class I molecule in a subject and/or in a donor tissue, the method comprising contacting the donor tissue and/or administering to the subject the PIF peptide, compositions thereof, mimetics thereof, pharmaceutically acceptable salts thereof.

11. A therapeutically effective amount of a PIF peptide, compositions thereof, mimetics thereof, pharmaceutically acceptable salts thereof, or combinations thereof, for use in a method of restoring menstruation in a mammal in need of restoration.

12. A pharmaceutical composition comprising:
(i) a therapeutically effective amount of a PIF peptide, compositions thereof, mimetics thereof, pharmaceutically acceptable salts thereof, or a combinations thereof;
(ii) a therapeutically effective amount of a steroid; and
(iii) a pharmaceutically acceptable carrier.

13. A therapeutically effective amount of PIF peptide, compositions thereof, mimetics thereof, pharmaceutically acceptable salts thereof, or a combination thereof, for use in a method of promoting or enhancing wound healing in a subject.

14. A therapeutically effective amount of PIF peptide, compositions thereof, mimetics thereof, pharmaceutically acceptable salts thereof, or a combination thereof, for use in a method of potentiating the effects of a steroid as a treatment in a subject in need thereof, the method comprising: (i) administering to the subject a pharmaceutical composition comprising a therapeutically effective amount of PIF peptide, compositions thereof, mimetics thereof, pharmaceutically acceptable salts thereof, or a combination thereof; and (ii) administering a therapeitucally effective amount of steroid before, during or after step (i).

15. The method of claim 14, wherein the steroid is dexamethasone.
